# EUROPEAN PATENT SPECIFICATION

(11) **EP 4 568 957 B1**
(45) Date of publication and mention of the grant of the patent: **15.07.2026**
(21) Application number: 24782667.0
(22) Date of filing: 30.08.2024
(51) Int. Cl.: C07D 413/14, C07D 491/052, A61P 35/00, A61K 31/4355, A61K 31/4545

(54) **SUBSTITUTED PHENYL OXOOXAZOLYL PIPERIDINE DIONE COMPOUNDS**
SUBSTITUIERTE PHENYL-OXOOXAZOLYL-PIPERIDINDION-VERBINDUNGEN
COMPOSÉS DE PHÉNYL OXOOXAZOLYL PIPÉRIDINE DIONE SUBSTITUÉS

(30) Priority: 02.09.2023 IN 202311058935
(43) Date of publication of application: 18.06.2025
(73) Proprietor: Bristol-Myers Squibb Company, Princeton, NJ 08543 (US)
(72) Inventor: SHIRUDE, Pravin Sudhakar, Bangalore 560099 (IN); PENMETSA, Suresh Babu Vishwa Krishna, Bangalore 560099 (IN); SHIMPUKADE, Bharat Dinkar, Bangalore 560099 (IN); CHERNEY, Emily Charlotte, Princeton, New Jersey 08543 (US); KANTHETI, Durgarao, Bangalore 560099 (IN)
(74) Representative: Mathys & Squire
(86) International application number: PCT/US2024/044574
(87) International publication number: WO 2025/049840

(56) References cited:
- WO-A1-2019/060693
- WO-A1-2022/216644

## Description

The present invention generally relates to substituted phenyl oxooxazolyl piperidine dione compounds that decrease the levels of the Ikaros, Helios, Aiolos, and Eos proteins. Provided herein are substituted phenyl oxooxazolyl piperidine dione compounds, compositions comprising such compounds, and methods of their use. The invention further pertains to pharmaceutical compositions comprising at least one compound according to the invention that are useful for the treatment of proliferative disorders, such as cancer and viral infections.

### BACKGROUND

The Ikaros zinc finger family (IKZF) of transcription factors (TFs) play critical roles in lymphocyte development and function (Heizmann et al., 2018, Curr Opin Immunol. 51: 14-23). In mammals, the following five members of this family of TFs are expressed in immune cells: Ikaros (encoded by IKZF1), Helios (IKZF2), Aiolos (IKZF3), Eos (IKZF4), and Pegasus (IKZF5). The amino acid sequences of these proteins are highly homologous, with Ikaros and Aiolos, and Helios and Eos being the most homologous pairs, and Pegasus being the most distantly related IKZF member. These TFs serve both overlapping and unique functions in lymphocytes (Read et al., 2020, Immunological Reviews, 300:1). Reductions in the protein levels of IKZF TFs can boost antitumor T cell responses.

IKZF1 encodes Ikaros, which is broadly and abundantly expressed in human and mouse B, NK, and T lymphocyte populations, and moderately expressed in other immune cell types, including myeloid cells. In T cells, loss of Ikaros protein or expression of a dominant-negative protein relieves repression of loci related to the differentiation of the effector T cell state, resulting in increased expression of effector cytokines including IFN-γ, TNF-α, and GM-CSF (Lyon de Ana, et al., 2019, Journal of Immunology 202: 1112-1123; Heller et al., 2014, Journal of Immunology, 193: 3934-3946); Wang et al., 2020, Cell Transplantation, 29).

IKZF2 encodes Helios, which shows a more restricted expression profile limited to human and mouse regulatory T (Treg) cells, some CD8+ T cells and MAIT cells, and NK cells (Akimova et al., 2011, PLoS One, 6:e24226; Dias et al., 2017, Proceedings of the National Academy of Sciences USA, 114:E5434-E5443; Thornton and Shevach, 2019, Immunology, 158:161-170).

IKZF3 encodes Aiolos, which is broadly and abundantly expressed in human and mouse B lymphocytes, and broadly expressed at lower levels in T and NK cells. In T cells, Aiolos gene repression targets show a large amount of overlap with Ikaros target genes (Powell et al., 2019, Frontiers in Immunology, 10:1299). Compared to Ikaros, Aiolos may have a stronger effect on follicular helper T cell and T helper 17 type responses (Quintana et al., 2012, Nature Immunology, 13:770-777; Read et al., 2017 Journal of Immunology, 7:2377-2387), which have been implicated in tissue immune responses and, in some cases, antitumor immunity.

IKZF4 encodes Eos, which is abundantly expressed in Treg cells, and is also broadly expressed at low levels among B, NK, and T lymphocytes. In Treg cells, loss of Eos expression in FoxP3+ Treg cells drives improved antitumor responses in preclinical syngeneic tumor models (Gokhale et al., 2019, Journal of Autoimmunity, 105:102300). Additionally, Eos expression levels can increase after T cell activation in conventional CD4+ and CD8+ T cells, where it can limit effector T cell responses (Rieder et al., 2015, Journal of Immunology, 195:553-563).

Common functions shared among IKZF TFs relate to the suppression of gene expression in cells at specific loci. IKZF TFs can bind to genomic loci either as homodimers or heterodimers, such as Ikaros:Ikaros or Ikaros:Helios, respectively. These dimeric TFs both bind to DNA and interact with complexes that regulate histone acetylation and nucleosomes, which in turn results in gene expression modulation. Mechanistically, Ikaros, Helios, and Aiolos have each been shown to interact with the nucleosome-remodeling and deacetylase (NuRD) and Sin3 histone deacetylase (HDAC) complexes to repress gene expression (Zhang et al., 2011, Nature Immunology, 13:86-94*;* Georgopoulos et al., 2017, Genes and Development, 31: 439-450). Similarly, Ikaros, Helios, and Aiolos all can associate with centromeric heterochromatin and contribute to the expression of genes located at centromeric loci (Brown et al., 1997, Cell, 91:845-854; Thompson et al., 2007, Immunity, 26:335-344). Eos co-operates with Ikaros, but not Aiolos, to interact with the transcriptional repressor C-terminal binding protein 1 (CtBP1) in lymphocytes (Koipally et al., 2002, Journal of Biological Chemistry, 277:27697-27705); Pan et al., 2009, Science, 325:1142-1146). Together, the overlapping functions of IKZF TFs may partially compensate for the loss or degradation of one or several TFs. Therefore, in cells that express multiple IKZF members, broad therapeutic degradation of this family of TFs would be expected to drive a stronger phenotypic change compared to selective degradation of one or two IKZF TFs.

In T cells and Treg cells, the shared roles of IKZF TFs in regulating a gene locus important for antitumor immune responses is exemplified by regulation of the gene encoding interleukin-2 (IL-2). Ikaros can directly bind to the IL-2 locus in CD4+ T cells and recruit HDAC complexes; loss of Ikaros results in increased IL-2 production by CD4+ and CD8+ T cells (Bandyopadhyay et al., 2007, Blood, 109: 2671-2672; Thomas et al., 2007, Journal of Immunology, 179: 7305-7315; O'Brien et al., 2014, Journal of Immunology, 192:5118-5129). Helios directly binds to the IL-2 locus in Treg cells to recruit HDAC complexes and enforce IL-2 gene silencing (Blaine et al., 2013, Journal of Immunology, 190:1008-1016). Eos also represses IL-2 expression in Treg cells and may act via a mechanism that involves interactions with the TF FoxP3 (Pan et al., 2009, Science, 325: 1142-1146; Sharma et al., 2013, Immunity, 38:998-1012). The role of direct Aiolos binding to the IL-2 loss is less clear, but siRNA knockdown of Aiolos in human Treg cells has been reported to increase IL-2 production (Gandhi et al., 2010, Nature Immunology, 11:846-853). In summary, the IKZF TFs act to modulate IL-2 production by multiple lymphocyte subtypes, particularly Treg cells in which all four of these IKZF TFs are abundantly expressed and IL-2 production is normally negligible.

Treg cells, which are marked by expression of the transcription factor FoxP3, are a subset of immunosuppressive lymphocytes that use several mechanisms to maintain immune homeostasis (Sakaguchi et al., 2020, Annual Review of Immunology, 38:541-566; Whibley et al., 2019, Nature Immunology, 20:386-396). Patients who have deleterious mutations in the gene encoding FoxP3 lack functional Treg cells and exhibit immune dysregulation, polyendocrinopathy, enteropathy, X-linked (IPEX) syndrome, a multi-organ autoimmune disorder. In the tumor microenvironment (TME), the activities of Treg cells are co-opted to promote and maintain an immunosuppressive state (Plitas and Rudensky, 2020, Annual Review of Cancer Biology, 4:459-477). By secreting suppressive molecules, sequestering cytokines (e.g., IL-2), and directly impeding T cell and antigen presenting cell activation, Treg cells can promote TME-mediated resistance to immunotherapy by modulating multiple axes in the cancer-immunity cycle (Chen and Mellman, 2013, Immunity, 39:1-10). In preclinical models, ablation of Treg cells results in the regression of aggressive, established tumors (Bos et al., 2013, Journal of Experimental Medicine, 210:2435-2466).

Once activated by a specific antigen, Treg cells can suppress responder T cells in an antigen-nonspecific and bystander manner *in vitro* (Takahashi et al., 1998, Int Immunol. 10:1969-80; Thornton et al., 1998, J Exp. Med. 188:287-96). FoxP3+CD25+CD4+Treg cells are capable of suppressing a wide range of antitumor immune responses involving CD4+ helper T cells, CD8+ T cells, natural killer cells, and natural killer T cells (Tanaka et al., 2017, Cell Research 27:109-118). In preclinical models, the intratumoral depletion of CD25+CD4+Treg cells induced regression of established tumors with a change in the cytokine milieu at tumor sites (Yu et al., 2005, J Exp Med. 201: 779-91). In addition, transfer of Treg cell-depleted CD4+ T cells markedly augmented antitumor immune responses compared with the transfer of Treg cell-sufficient CD4+ T cells (Antony et al., 2005, J Immunol 174:2591-601). Tumor-infiltrating Treg cells activated by either tumor-derived self-antigens or tumor-associated antigens can similarly suppress specific antitumor immune responses.

Clinically, increased Treg cell frequencies in the TME are correlated with worse outcomes in multiple solid tumor indications (Shang et al., 2015, Scientific Reports, 5:15179). Moreover, correlations between PD-L1+ Treg cell frequencies and responses to anti-PD-1 therapy in non-small cell lung cancer (NSCLC) patients (Wu et al., 2018, Journal of Thoracic Oncology, 13:521-532) highlight the therapeutic potential of Treg cell targeting in the TME. Modulation of the activities of key factors to control Treg cell differentiation and/or functional suppressor state could represent a potential therapeutic strategy for the treatment of certain diseases, including cancer and viral infections.

Furthermore, removal of FoxP3+ Treg cells was also reported to enhance vaccine-induced antitumor T-cell responses (Nishikawa et al., 2010, Int. J. Cancer 127: 759-767), suggesting that decreasing Helios levels could be beneficial in boosting the efficacy of cancer vaccines. Besides anti-tumor immunotherapy, during viral infections, Treg cells can limit the immunopathology resulting from excessive inflammation, yet potentially inhibit effective antiviral T cell responses and promote virus persistence (Schmitz et al., 2013, PLOS Pathogens 9: e1003362). Chronic, but not acute, infection of mice with lymphocytic choriomeningitis virus results in a marked expansion of FoxP3+ Treg cells, implying a potential mechanism that certain infectious agents could evade host immune responses by activation and expansion of Treg cells (Punkosdy et al., 2011, PNAS 108: 3677-3682). Treatment benefits could be achieved by decreasing Helios levels in activated Treg cells in a context relevant to chronic viral infections.

Approaches to targeting tumor Treg cells include antibody-mediated depletion and/or functional modulation (Tanaka and Sakaguchi, 2019, European Journal of Immunology, 49:1140-1146), as well as small molecule-mediated "reprogramming" of the Treg cell immunosuppressive phenotype by altering gene expression in these cells (Kim et al., 2015, Science, 350:334-339; Sebastian et al., 2016, Journal of Immunology, 196:144-155). Mice with Treg cells engineered to lack Helios do not develop IPEX-like immunopathology characteristic of FoxP3-deficiency or complete Treg cell ablation, but instead have Treg cells that display a more T effector-like transcriptional program (Fu et al., 2012, Nature Immunology, 13: 972-980; Yates et al., 2018, Proceedings of the National Academy of Sciences USA, 115:2162-2167). Importantly, Helios controls activities of Treg cells that are critical in the TME as mice with Helios-deficient Treg cells show improved control of B16F10 and MC38 tumors (Nakagawa et al., 2016, Proceedings of the National Academy of Sciences USA, 113:6248-6253). Therefore, therapeutic modulation of Helios has the potential to reprogram tumor Treg cells towards a more effector-like phenotype to drive antitumor immunity. Notably, Eos also drives immunosuppressive Treg cell activity in the TME in preclinical tumor models, as mice lacking Eos expression in FoxP3 Treg cells more effectively control syngeneic tumors compared to controls (Gokhale et al., 2019, Journal of Autoimmunity,105:102300). Humans with germline loss-of-function *IKZF2* mutations similarly do not exhibit IPEX-like symptoms (including diabetes, dermatitis, hepatic inflammation, and systemic lymphadenopathy), but instead manifest immunophenotypes related to enhanced T cell activation and proinflammatory cytokine production (Hetemäki et al., 2021, Science Immunology, 6:eabe3454; Shahin et al., 2021, Science Immunology, 6:eabe3981). These data indicate that reduction of Helios and Eos protein levels in Treg cells would make them less suppressive of antitumor T cell responses in patients with solid tumors.

Small molecules that degrade Ikaros and Aiolos in Treg cells can also reduce the suppressive functions of these cells in vitro (Galustian et al., 2008, Cancer Immunology, Immunotherapy, 58:1033-1045). In engineered mouse models, the Ikaros and Aiolos degrader lenalidomide can modestly increase antitumor immune responses against highly immunogenic syngeneic tumors (Geng et al., 2022, Cell Chemical Biology, 29:1260-1272). Ikaros- and Aiolos-targeted degraders have also been tested clinically in patients with solid tumors, sometimes resulting in modest responses of stable disease. These studies included avadomide (CC-122) in advanced malignancies (Rasco et al., 2019, Clin Cancer Research, 25:90-98), lenalidomide (Semeraro et al., 2013, OncoImmunology, 2:11), and pomalidomide (Cooney et al., 2012, Cancer Chemotherapy and Pharmacology, 70, 755). Additionally, lenalidomide has been shown to augment T and NK cell functions in preclinical and clinical studies (Hideshima et al., Leukemia, 2021; D'Souza et al., Frontiers in Immunology, 2021).

In summary, the IKZF TFs, Ikaros, Helios, Aiolos, and Eos are abundantly expressed in Treg cells. Combined reduction of the individual protein levels of these four TFs in Treg cells would better reverse the immunosuppressive program, including repression of IL-2 transcription and other T cell effector genes, compared to approaches that selectively target single IKZF TFs or pairs of TFs, i.e., Ikaros and Aiolos or Helios and Eos. Beyond Treg cells, a pan-IKZF1-4 degrader would be expected to increase conventional CD4+ and CD8+ T cell effector functions and boost NK cell activity to drive robust antitumor responses in patients.

There remains a need for therapies that can decrease the levels of the four IKZF1-4 proteins Ikaros, Helios, Aiolos, and Eos.

The present invention fills the foregoing need by providing compounds that are useful to decrease the levels of the four IKZF1-4 proteins Ikaros, Helios, Aiolos, and Eos.

### SUMMARY

The present disclosure relates to substituted phenyl oxooxazolyl piperidine dione compounds of Formula (I), including stereoisomers, tautomers, and salts thereof, which are useful to decrease the levels of the four proteins Ikaros, Helios, Aiolos, and Eos.

The present invention provides a compound of Formula (I) or stereoisomers, tautomers, or salts thereof in accordance with the appended claims.

The present invention also provides a pharmaceutical composition comprising a compound of Formula (I), or stereoisomers, tautomers, or pharmaceutically acceptable salts thereof; and a pharmaceutically acceptable carrier.

The present invention also provides a compound, or stereoisomers, tautomers, or pharmaceutically acceptable salts thereof of the present invention, for use in the treatment of cancer. In embodiments, the cancer is selected from cancer of the colon, gastric, pancreatic cancer, breast cancer, prostate cancer, lung cancer, ovarian cancer, cervical cancer, renal cancer, cancer of the head and neck, lymphoma, leukemia, and melanoma.

The present invention also provides a compound, or a stereoisomer, a tautomer, or a pharmaceutically acceptable salt thereof of the present invention, for use in a method of treating cancer, wherein the method comprises decreasing Ikaros, Helios, Aiolos, and Eos protein levels in cells by contacting said cells with said compound, or a stereoisomer, a tautomer, or a pharmaceutically acceptable salt thereof, wherein
a) said Ikaros protein level is decreased by at least 30 %;
b) said Helios protein level is decreased by at least 50 %;
c) said Aiolos protein level is decreased by at least 30 %; and
d) said Eos protein level is decreased by at least 50 %.

The present disclosure also provides processes and intermediates for making the compounds of Formula (I), stereoisomers, tautomers, or salts thereof.

The present disclosure also provides the use of the compounds of Formula (I), or stereoisomers, tautomers, pharmaceutically acceptable salts, or prodrugs thereof, for the manufacture of a medicament to decrease Ikaros, Helios, Aiolos, and Eos protein levels, for the treatment of certain diseases, including cancer and viral infections.

The compounds of Formula (I) and compositions comprising the compounds of Formula (I) may be used in treating, preventing, or curing various proliferative disorders, such as cancer. Pharmaceutical compositions comprising these compounds are useful in treating, preventing, or slowing the progression of diseases or disorders in a variety of therapeutic areas, such as cancer.

The compounds of Formula (I) and compositions comprising the compounds of Formula (I) may be used in treating, preventing, or curing viral infections. Pharmaceutical compositions comprising these compounds are useful in treating, preventing, or slowing the progression of diseases or disorders, such as viral infections.

These and other features of the invention will be set forth in expanded form as the disclosure continues.

### DETAILED DESCRIPTION

Applicants have found substituted phenyl oxooxazolyl piperidine dione compounds that decrease the levels of the Ikaros, Helios, Aiolos, and Eos proteins. The substituted phenyl oxooxazolyl piperidine dione compounds are believed to facilitate the interactions of Ikaros, Helios, Aiolos, and Eos proteins with the corresponding E3 ubiquitin ligase complex (Cullin4-Cereblon, CUL4-CRBN), with concomitant degradation of the Ikaros, Helios, Aiolos, and Eos proteins. These compounds decrease the levels of Ikaros protein, Helios protein, Aiolos protein, and Eos protein. These compounds are useful for the treatment of certain diseases, including cancer and viral infections. The compounds are provided to be useful as pharmaceuticals with desirable stability, bioavailability, therapeutic index, and toxicity values that are important to their drugability.

The invention provides at least one compound of Formula (I): 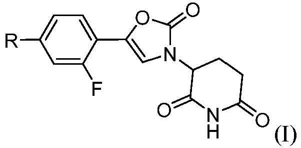 or stereoisomers, tautomers, or salts thereof, wherein:
R is:

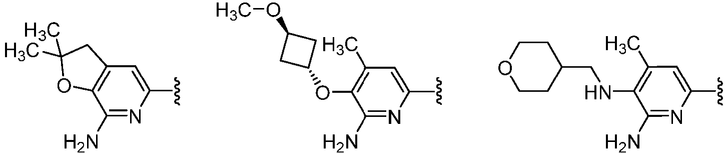

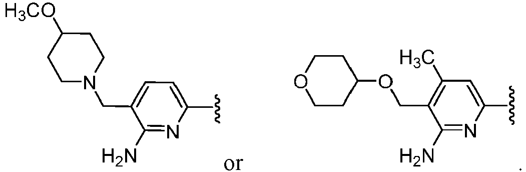

The present invention also provides at least one compound of Formula (I): 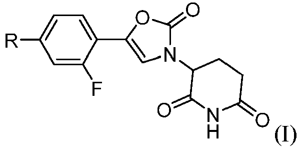 or stereoisomers, tautomers, or salts thereof, wherein:
R is: 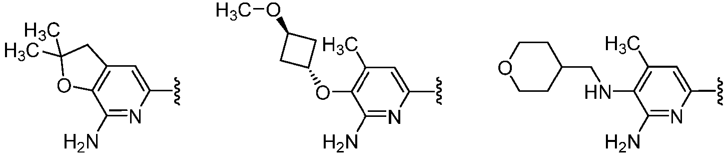 or

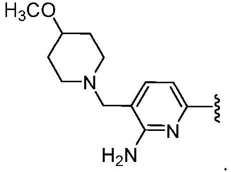

One embodiment provides a compound of Formula (I), or stereoisomers, tautomers, or pharmaceutically acceptable salts thereof.

One embodiment provides a compound of Formula (I), or stereoisomers or tautomers thereof.

One embodiment provides a salt of the compound of Formula (I), or stereoisomers or tautomers thereof.

One embodiment provides a pharmaceutically acceptable salt of the compound of Formula (I), or stereoisomers or tautomers thereof.

One embodiment provides a compound of Formula (I) or stereoisomers, tautomers, or salts thereof, wherein R is:

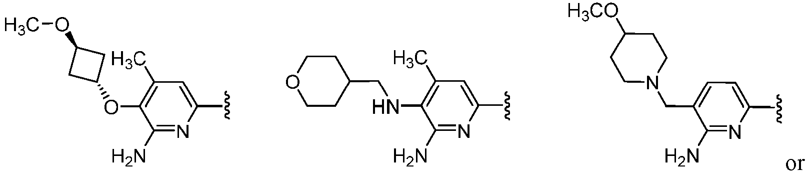

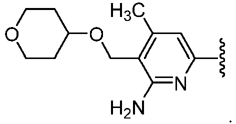

One embodiment provides a compound of Formula (I) or stereoisomers, tautomers, or salts thereof, wherein R is: 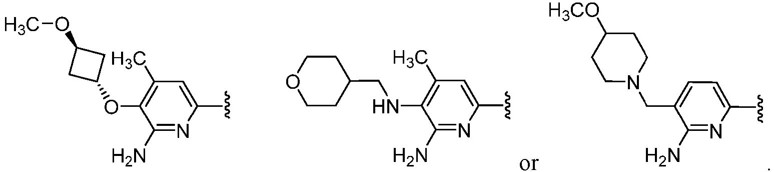 Included in this embodiment is a compound of Formula (I) or a pharmaceutically acceptable salt thereof. Also, included in this embodiment is a compound of Formula (I). Additionally, included in this embodiment is a pharmaceutically acceptable salt of a compound of Formula (I).

One embodiment provides a compound of Formula (I) having the structure: 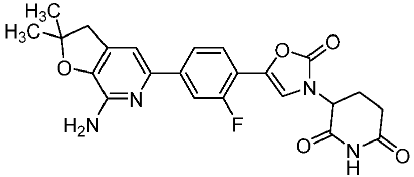 or stereoisomers, tautomers, or salts thereof.

One embodiment provides a compound of Formula (I) having the structure: 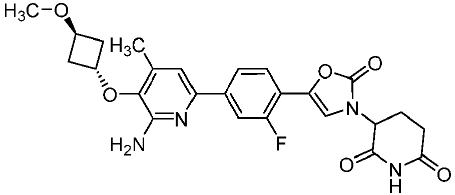 or stereoisomers, tautomers, or salts thereof.

One embodiment provides a compound of Formula (I) having the structure: 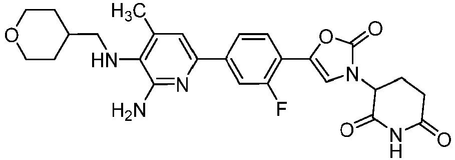 or stereoisomers, tautomers, or salts thereof.

One embodiment provides a compound of Formula (I) having the structure: 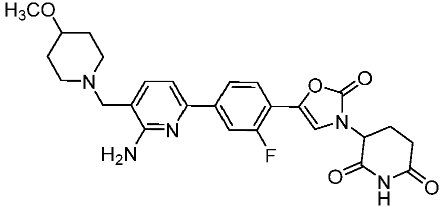 or stereoisomers, tautomers, or salts thereof.

One embodiment provides a compound of Formula (I) having the structure: 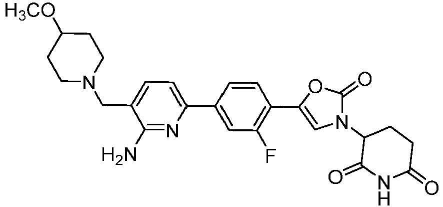 or stereoisomers, tautomers, or salts thereof.

One embodiment provides a compound of Formula (I), or stereoisomers, tautomers, or salts thereof, wherein said compound is:
3-(5-(4-(7-amino-2,2-dimethyl-2,3-dihydrofuro[2,3-c]pyridin-5-yl)-2-fluorophenyl)-2-oxooxazol-3(2*H*)-yl)piperidine-2,6-dione (1);
*trans* 3-(5-(4-(6-amino-5-(3-methoxycyclobutoxy)-4-methylpyridin-2-yl)-2-fluorophenyl)-2-oxooxazol-3(2H)-yl)piperidine-2,6-dione (2);
3-(5-(4-(6-amino-4-methyl-5-(((tetrahydro-2*H*-pyran-4-yl)methyl)amino)pyridin-2-yl)-2-fluorophenyl)-2-oxooxazol-3(2*H*)-yl)piperidine-2,6-dione (3);
3-(5-(4-(6-amino-5-((4-methoxypiperidin-1-yl)methyl)pyridin-2-yl)-2-fluorophenyl)-2-oxooxazol-3(2H)-yl)piperidine-2,6-dione (4); or
3-(5-(4-(6-amino-4-methyl-5-(((tetrahydro-2*H*-pyran-4-yl)oxy)methyl)pyridin-2-yl)-2-fluorophenyl)-2-oxooxazol-3(2*H*)-yl)piperidine-2,6-dione.

One embodiment provides a compound of Formula (I), or stereoisomers, tautomers, or salts thereof, wherein said compound is 3-(5-(4-(7-amino-2,2-dimethyl-2,3-dihydrofuro[2,3-c]pyridin-5-yl)-2-fluorophenyl)-2-oxooxazol-3(2*H*)-yl) piperidine-2,6-dione. Included in this embodiment are one or more pharmaceutically acceptable salts.

One embodiment provides a compound of Formula (I), or tautomers or salts thereof, wherein said compound is (*R*)-3-(5-(4-(7-amino-2,2-dimethyl-2,3-dihydrofuro[2,3-c]pyridin-5-yl)-2-fluorophenyl)-2-oxooxazol-3(2*H*)-yl)piperidine-2,6-dione. Included in this embodiment are one or more pharmaceutically acceptable salts.

One embodiment provides a compound of Formula (I), or tautomers or salts thereof, wherein said compound is (*S*)-3-(5-(4-(7-amino-2,2-dimethyl-2,3-dihydrofuro[2,3-c]pyridin-5-yl)-2-fluorophenyl)-2-oxooxazol-3(2*H*)-yl)piperidine-2,6-dione. Included in this embodiment are one or more pharmaceutically acceptable salts.

One embodiment provides a compound of Formula (I), or stereoisomers, tautomers, or salts thereof, wherein said compound is *trans* 3-(5-(4-(6-amino-5-(3-methoxycyclobutoxy)-4-methylpyridin-2-yl)-2-fluorophenyl)-2-oxooxazol-3(2H)-yl) piperidine-2,6-dione. Included in this embodiment are one or more pharmaceutically acceptable salts.

One embodiment provides a compound of Formula (I), or tautomers or salts thereof, wherein said compound is *trans* (*R*)*-* 3-(5-(4-(6-amino-5-(3-methoxycyclobutoxy)-4-methylpyridin-2-yl)-2-fluorophenyl)-2-oxooxazol-3(2H)-yl) piperidine-2,6-dione. Included in this embodiment are one or more pharmaceutically acceptable salts.

One embodiment provides a compound of Formula (I), or tautomers or salts thereof, wherein said compound is *trans* (*S*)-3-(5-(4-(6-amino-5-(3-methoxycyclobutoxy)-4-methylpyridin-2-yl)-2-fluorophenyl)-2-oxooxazol-3(2H)-yl) piperidine-2,6-dione. Included in this embodiment are one or more pharmaceutically acceptable salts.

One embodiment provides a compound of Formula (I), or stereoisomers, tautomers, or salts thereof, wherein said compound is 3-(5-(4-(6-amino-4-methyl-5-(((tetrahydro-2*H*-pyran-4-yl)methyl)amino)pyridin-2-yl)-2-fluorophenyl)-2-oxooxazol-3(2*H*)-yl)piperidine-2,6-dione. Included in this embodiment are one or more pharmaceutically acceptable salts.

One embodiment provides a compound of Formula (I), or tautomers or salts thereof, wherein said compound is (*R*)-3-(5-(4-(6-amino-4-methyl-5-(((tetrahydro-2*H-*pyran-4-yl)methyl)amino)pyridin-2-yl)-2-fluorophenyl)-2-oxooxazol-3(2*H*)-yl) piperidine-2,6-dione. Included in this embodiment are one or more pharmaceutically acceptable salts.

One embodiment provides a compound of Formula (I), or tautomers or salts thereof, wherein said compound is (*S*)-3-(5-(4-(6-amino-4-methyl-5-(((tetrahydro-2*H-*pyran-4-yl)methyl)amino)pyridin-2-yl)-2-fluorophenyl)-2-oxooxazol-3(2*H*)-yl)piperidine-2,6-dione. Included in this embodiment are one or more pharmaceutically acceptable salts.

One embodiment provides a compound of Formula (I), or stereoisomers, tautomers, or salts thereof, wherein said compound is 3-(5-(4-(6-amino-5-((4-methoxypiperidin-1-yl)methyl)pyridin-2-yl)-2-fluorophenyl)-2-oxooxazol-3(2H)-yl)piperidine-2,6-dione. Included in this embodiment are one or more pharmaceutically acceptable salts.

One embodiment provides a compound of Formula (I), or tautomers or salts thereof, wherein said compound is (*R*)-3-(5-(4-(6-amino-5-((4-methoxypiperidin-1-yl)methyl)pyridin-2-yl)-2-fluorophenyl)-2-oxooxazol-3(2H)-yl)piperidine-2,6-dione. Included in this embodiment are one or more pharmaceutically acceptable salts.

One embodiment provides a compound of Formula (I), or tautomers or salts thereof, wherein said compound is (*S*)-3-(5-(4-(6-amino-5-((4-methoxypiperidin-1-yl)methyl)pyridin-2-yl)-2-fluorophenyl)-2-oxooxazol-3(2H)-yl)piperidine-2,6-dione. Included in this embodiment are one or more pharmaceutically acceptable salts.

One embodiment provides a compound of Formula (I), or stereoisomers, tautomers, or salts thereof, wherein said compound is 3-(5-(4-(6-amino-4-methyl-5-(((tetrahydro-2*H*-pyran-4-yl)oxy)methyl)pyn* din-2-yl)-2-fluorophenyl)-2-oxooxazol-3(2*H*)-yl)piperidine-2,6-dione. Included in this embodiment are one or more pharmaceutically acceptable salts.

One embodiment provides a compound of Formula (I), or tautomers or salts thereof, wherein said compound is (*R*)- 3-(5-(4-(6-amino-4-methyl-5-(((tetrahydro-2*H-*pyran-4-yl)oxy)methyl)pyridin-2-yl)-2-fluorophenyl)-2-oxooxazol-3(2*H*)-yl)piperidine-2,6-dione. Included in this embodiment are one or more pharmaceutically acceptable salts.

One embodiment provides a compound of Formula (I), or tautomers or salts thereof, wherein said compound is (*S*)- 3-(5-(4-(6-amino-4-methyl-5-(((tetrahydro-2*H-*pyran-4-yl)oxy)methyl)pyridin-2-yl)-2-fluorophenyl)-2-oxooxazol-3(2*H*)-yl)piperidine-2,6-dione. Included in this embodiment are one or more pharmaceutically acceptable salts.

One embodiment provides a compound having the structure: 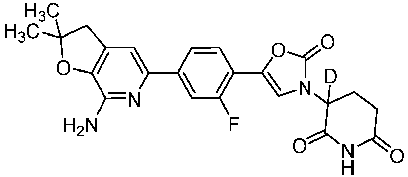 or stereoisomers, tautomers, or pharmaceutically acceptable salts thereof.

One embodiment provides a compound having the structure: 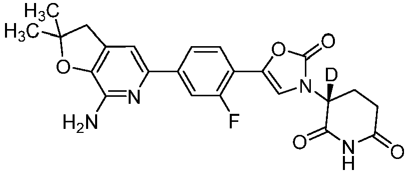 or tautomers or pharmaceutically acceptable salts thereof.

One embodiment provides a compound having the structure: 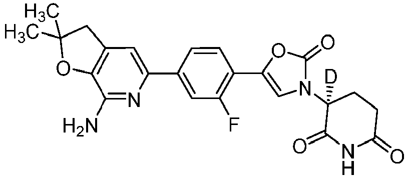 or tautomers or pharmaceutically acceptable salts thereof.

One embodiment provides a compound of Formula (I) having the structure: 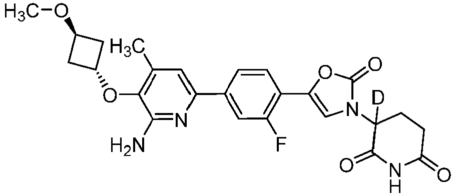 or stereoisomers, tautomers, or pharmaceutically acceptable salts thereof.

One embodiment provides a compound of Formula (I) having the structure: 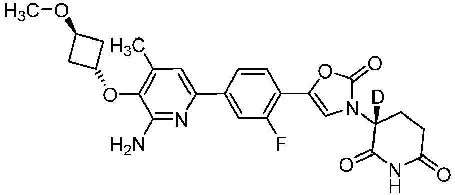 or tautomers or pharmaceutically acceptable salts thereof.

One embodiment provides a compound of Formula (I) having the structure: 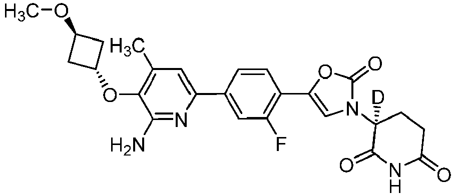 or tautomers or pharmaceutically acceptable salts thereof.

One embodiment provides a compound of Formula (I) having the structure: 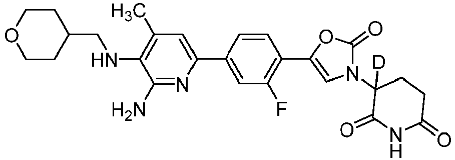 or stereoisomers, tautomers, or pharmaceutically acceptable salts thereof.

One embodiment provides a compound of Formula (I) having the structure: 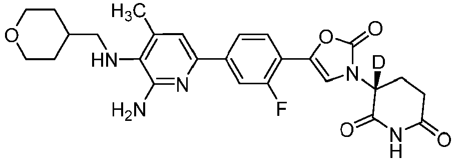 or tautomers, or pharmaceutically acceptable salts thereof.

One embodiment provides a compound of Formula (I) having the structure: 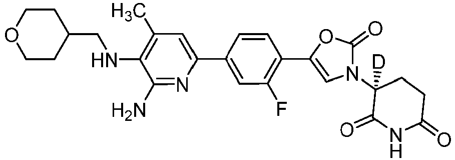 or tautomers or pharmaceutically acceptable salts thereof.

One embodiment provides a compound having the structure: 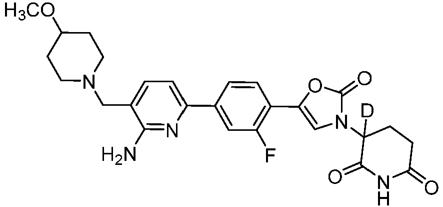 or stereoisomers, tautomers, or pharmaceutically acceptable salts thereof.

One embodiment provides a compound having the structure: 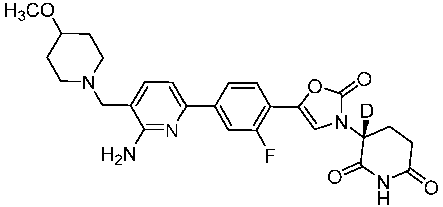 or tautomers or pharmaceutically acceptable salts thereof.

One embodiment provides a compound having the structure: 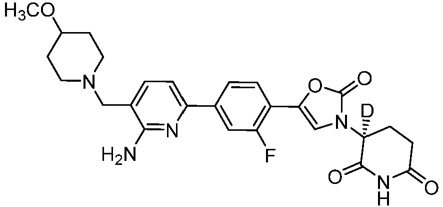 or tautomers or pharmaceutically acceptable salts thereof.

One embodiment provides a compound having the structure: 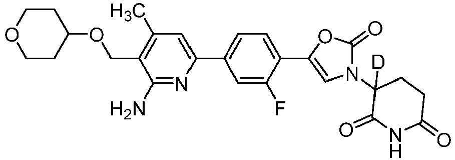 or stereoisomers, tautomers, or pharmaceutically acceptable salts thereof.

One embodiment provides a compound having the structure: 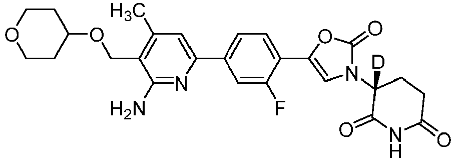 or tautomers or pharmaceutically acceptable salts thereof.

One embodiment provides a compound having the structure: 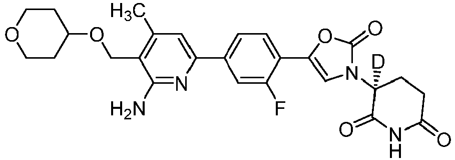 or tautomers or pharmaceutically acceptable salts thereof.

One embodiment provides a salt of a compound of having the structure: 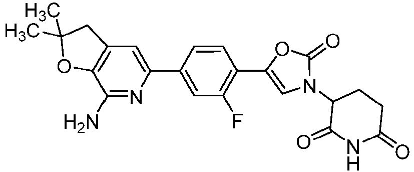 or stereoisomers or tautomers thereof. Included in this embodiment are one or more pharmaceutically acceptable salts of the compound.

One embodiment provides a salt of a compound having the structure: 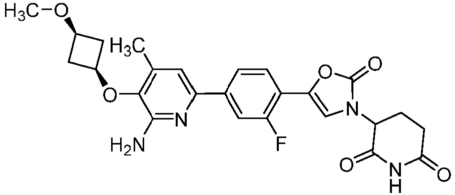 or stereoisomers or tautomers thereof. Included in this embodiment are one or more pharmaceutically acceptable salts of the compound.

One embodiment provides a salt of a compound having the structure: 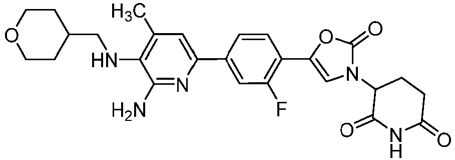 or stereoisomers or tautomers thereof. Included in this embodiment are one or more pharmaceutically acceptable salts of the compound.

One embodiment provides a salt of a compound having the structure: 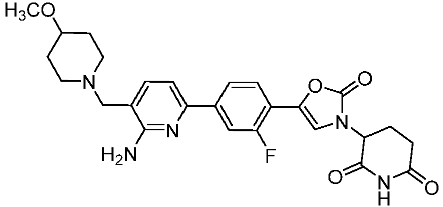 or stereoisomers or tautomers thereof. Included in this embodiment are one or more pharmaceutically acceptable salts of the compound.

One embodiment provides a salt of a compound having the structure: 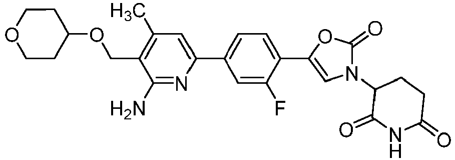 or stereoisomers or tautomers thereof. Included in this embodiment are one or more pharmaceutically acceptable salts of the compound.

The compounds of Formula (I) or stereoisomers, tautomers, or salts thereof, are useful to decrease the levels of the four IKZF1-4 proteins Ikaros, Helios, Aiolos, and Eos.

As used herein, "to decrease the level" of one of the IKZF1-4 proteins refers to reducing the level of the protein by the degradation and/or inactivation and/or inhibition and/or reducing the expression levels of the protein, or a combination thereof, compared to the initial protein level prior to contact or treatment with the compound of Formula (I) or stereoisomers, tautomers, or salts thereof.

Various methods can be employed to measure the decreases in the protein levels of the IKZF1-4 proteins, including the following assays described hereinbelow: (i) IKZF1: Human CD8⁺ T Cell Reprogramming Assay; (ii) IKZF2: Jurkat Cellular Degradation Assay; (iii) IKZF3: Human CD8⁺ T Cell Reprogramming Assay; and (iv) IKZF4: Human Regulatory T Cell Reprogramming Assay.

This invention encompasses all combinations of the aspects and/or embodiments of the invention noted herein. It is understood that any and all embodiments of the present invention may be taken in conjunction with any other embodiment or embodiments to describe additional embodiments. It is also to be understood that each individual element of the embodiments is meant to be combined with any and all other elements from any embodiment to describe an additional embodiment.

The features and advantages of the invention may be more readily understood by those of ordinary skill in the art upon reading the following detailed description. It is to be appreciated that certain features of the invention that are, for clarity reasons, described above and below in the context of separate embodiments, may also be combined to form a single embodiment. Conversely, various features of the invention that are, for brevity reasons, described in the context of a single embodiment, may also be combined so as to form sub-combinations thereof. Embodiments identified herein as exemplary or preferred are intended to be illustrative and not limiting.

Unless specifically stated otherwise herein, references made in the singular may also include the plural. For example, "a" and "an" may refer to either one, or one or more.

As used herein, the phrase "compounds and/or salts thereof" refers to at least one compound, at least one salt of the compounds, or a combination thereof. For example, compounds of Formula (I) and/or salts thereof includes a compound of Formula (I); two compounds of Formula (I); a salt of a compound of Formula (I); a compound of Formula (I) and one or more salts of the compound of Formula (I); and two or more salts of a compound of Formula (I).

Unless otherwise indicated, any atom with unsatisfied valences is assumed to have hydrogen atoms sufficient to satisfy the valences.

The definitions set forth herein take precedence over definitions set forth in any patent, patent application, and/or patent application publication referenced herein.

Listed below are definitions of various terms used to describe the present disclosure. These definitions apply to the terms as they are used throughout the specification (unless they are otherwise limited in specific instances) either individually or as part of a larger group.

Throughout the specification, groups and substituents thereof may be chosen by one skilled in the field to provide stable moieties and compounds.

In accordance with a convention used in the art, 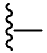 is used in structural formulas herein to depict the bond that is the point of attachment of the moiety or substituent to the core or backbone structure.

The term "amino" refers to the group -NH₂.

The term "oxo" refers to the group =O.

The compounds of the present invention include all isotopes of atoms occurring in the present compounds. Isotopes include those atoms having the same atomic number but different mass numbers. By way of general example and without limitation, isotopes of hydrogen include deuterium (D) and tritium (T). Isotopes of carbon include ¹³C and ¹⁴C. Isotopically-labeled compounds of the invention can generally be prepared by conventional techniques known to those skilled in the art or by processes analogous to those described herein, using an appropriate isotopically-labeled reagent in place of the non-labeled reagent otherwise employed.

As used herein, the term "tautomer" refers to each of two or more isomers of a compound that exist together in equilibrium, and are readily interchanged by migration of an atom or group within the molecule. For example, one skilled in the art would readily understand that a 1,2,3-triazole exists in two tautomeric forms as defined above: 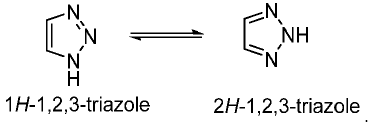 Thus, this disclosure is intended to cover all possible tautomers even when a structure depicts only one of them. For example, the compounds of Formula (I) can exist in tautomer forms: 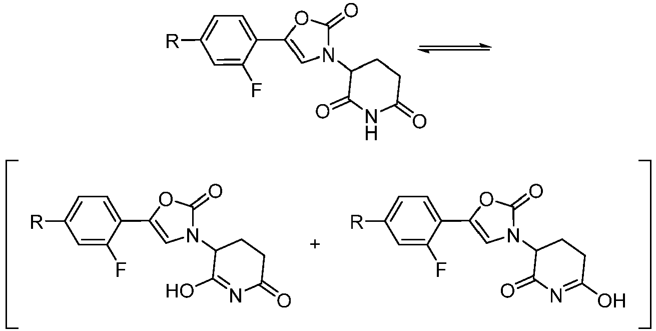 Other examples of tautomer forms include:

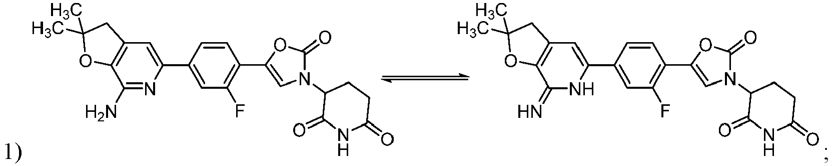

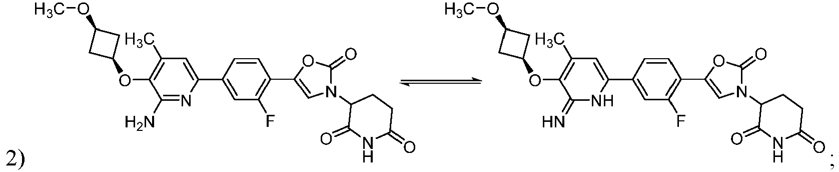

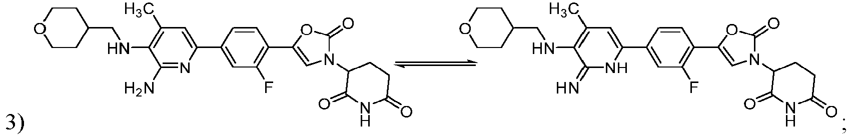

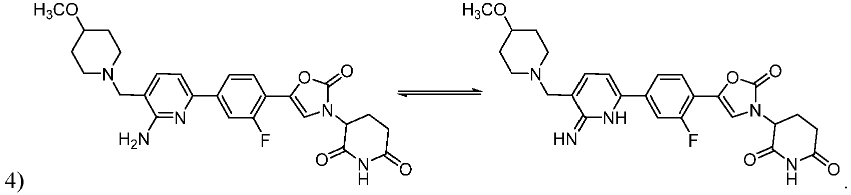

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

The compounds of Formula (I) can form salts which are also within the scope of this invention. Unless otherwise indicated, reference to an inventive compound is understood to include reference to one or more salts thereof. The term "salt(s)" denotes acidic salt(s) formed with inorganic and/or organic acids. **In** addition, the term "salt(s) may include zwitterions (inner salts), e.g., when a compound of Formula (I) contains both a basic moiety, such as an amine or a pyridine or imidazole ring, and an acidic moiety, such as a carboxylic acid. Pharmaceutically acceptable (i.e., non-toxic, physiologically acceptable) salts are preferred, such as, for example, acceptable metal and amine salts in which the cation does not contribute significantly to the toxicity or biological activity of the salt. However, other salts may be useful, *e.g.,* in isolation or purification steps which may be employed during preparation, and thus, are contemplated within the scope of the invention. Salts of the compounds of the formula (I) may be formed, for example, by reacting a compound of the Formula (I) with an amount of acid or base, such as an equivalent amount, in a medium such as one in which the salt precipitates or in an aqueous medium followed by lyophilization.

Exemplary acid addition salts include acetates (such as those formed with acetic acid or trihaloacetic acid, for example, trifluoroacetic acid), adipates, alginates, ascorbates, aspartates, benzoates, benzenesulfonates, bisulfates, borates, butyrates, citrates, camphorates, camphorsulfonates, cyclopentanepropionates, digluconates, dodecylsulfates, ethanesulfonates, fumarates, glucoheptanoates, glycerophosphates, hemisulfates, heptanoates, hexanoates, hydrochlorides (formed with hydrochloric acid), hydrobromides (formed with hydrogen bromide), hydroiodides, maleates (formed with maleic acid), 2-hydroxyethanesulfonates, lactates, methanesulfonates (formed with methanesulfonic acid), 2-naphthalenesulfonates, nicotinates, nitrates, oxalates, pectinates, persulfates, 3-phenylpropionates, phosphates, picrates, pivalates, propionates, salicylates, succinates, sulfates (such as those formed with sulfuric acid), sulfonates (such as those mentioned herein), tartrates, thiocyanates, toluenesulfonates such as tosylates, undecanoates, and the like.

The compounds of Formula (I) can be provided as amorphous solids or crystalline solids. Lyophilization can be employed to provide the compounds of Formula (I) as a solid.

It should further be understood that solvates (e.g., hydrates) of the compounds of Formula (I) are also within the scope of the present invention. The term "solvate" means a physical association of a compound of Formula (I) with one or more solvent molecules, whether organic or inorganic. This physical association includes hydrogen bonding. In certain instances, the solvate will be capable of isolation, for example when one or more solvent molecules are incorporated in the crystal lattice of the crystalline solid. "Solvate" encompasses both solution-phase and isolable solvates. Exemplary solvates include hydrates, ethanolates, methanolates, isopropanolates, acetonitrile solvates, and ethyl acetate solvates. Methods of solvation are known in the art.

Various forms of prodrugs are well known in the art and are described in Rautio, J. et al., Nature Review Drug Discovery, 17, 559-587 (2018).

In addition, compounds of Formula (I), subsequent to their preparation, can be isolated and purified to obtain a composition containing an amount by weight equal to or greater than 99% of a compound of Formula (I) ("substantially pure"), which is then used or formulated as described herein. Such "substantially pure" compounds of Formula (I) are also contemplated herein as part of the present invention.

"Stable compound" and "stable structure" are meant to indicate a compound that is sufficiently robust to survive isolation to a useful degree of purity from a reaction mixture, and formulation into an efficacious therapeutic agent. The present invention is intended to embody stable compounds.

The terms "IKZF1 degrader" and "Ikaros degrader" refer to an agent capable of reducing the level of the IKZF1 protein by degradation and/or inactivation and/or inhibition and/or reducing the expression levels of the IKZF1 protein, or a combination thereof.

The terms "IKZF2 degrader" and "Helios degrader" refer to an agent capable of reducing the level of the IKZF2 protein by degradation and/or inactivation and/or inhibition and/or reducing the expression levels of the IKZF2 protein, or a combination thereof.

The terms "IKZF3 degrader" and "Aiolos degrader" refer to an agent capable of reducing the level of the IKZF3 protein by degradation and/or inactivation and/or inhibition and/or reducing the expression levels of the IKZF3 protein, or a combination thereof.

The terms "IKZF4 degrader" and "Eos degrader" refer to an agent capable of reducing the level of the IKZF4 protein by degradation and/or inactivation and/or inhibition and/or reducing the expression levels of the IKZF4 protein, or a combination thereof.

The term "IKZF1-4 proteins" refers to the Ikaros (IKZF1), Helios (IKZF2), Aiolos (IKZF3), and Eos (IKZF4) proteins.

The term "pan IKZF1-4 degrader" refers to an agent capable of decreasing the protein levels of the four IKZF1-4 proteins Ikaros, Helios, Aiolos, and Eos.

As used herein, "Ikaros" protein is encoded by the IKZF1 gene. Ikaros is also known as IKAROS family zinc finger 1, ZNFN1A1, zinc finger protein, subfamily 1A, 1, Ikaros family zinc finger protein 1, IK1, lymphoid transcription factor LyF-1, Hs. 54452, PPP1R92, protein phosphatase 1, regulatory subunit 92, PRO0758, CVID13, and CLL associated antigen KW-6. "Ikaros" protein includes isoforms encoded by the following human isoforms listed below
Isoform 1 (UniPort Q13422-1) 









 (SEQ ID NO: 1)
Isoform 2 (UniProt Q13422-2) 
MDADEGQDMSQVSGKESPPVSDTPDEGDEPMIPEDLSTTSGGQQSSKSDRVVG
ERPFQCNQCGASFTQKGNLLRHIKLHSGEKPFKCHLCNYACRRRDALTGHLRT
HSVGKPHKCGYCGRSYKQRSSLEEHKERCHNYLESMGLPGTLYPVIKEETNHSE
MAEDLCKIGSERSLVLDRLASNVAKRKSSMPQKFLGDKGLSDTPYDSSASYEKE
NEMMKSHVMDQAINNAINYLGAESLRPLVQTPPGGSEVVPVISPMYQLHKPLAE
GTPRSNHSAQDSAVENLLLLSKAKLVPSEREASPSNSCQDSTDTESNNEEQRSGLI
YLTNHIAPHARNGLSLKEEHRAYDLLRAASENSQDALRVVSTSGEQMKVYKCEH
 
CRVLFLDHVMYTIHMGCHGFRDPFECNMCGYHSQDRYEFSSHITRGEHRFHMS
(SEQ ID NO: 2)
Isoform 3 (UniProt Q13422-3) 
MDADEGQDMSQVSGKESPPVSDTPDEGDEPMPIPEDLSTTSGGQQSSKSDRVVA
SNVKVETQSDEENGRACEMNGEECAEDLRMLDASGEKMNGSHRDQGSSALSGV
GGIRLPNGKLKCDICGIICIGPNVLMVHKRSHTGERPFQCNQCGASFTQKGNLL
RHIKLHSGEKPFKCHLCNYACRRRDALTGHLRTHSGDKGLSDTPYDSSASYEKE
NEMMKSHVMDQAINNAINYLGAESLRPLVQTPPGGSEVVPVISPMYQLHKPLAE
GTPRSNHSAQDSAVENLLLLSKAKLVPSEREASPSNSCQDSTDTESNNEEQRSGLI
YLTNHIAPHARNGLSLKEEHRAYDLLRAASENSQDALRVVSTSGEQMKVYKCEH
CRVLFLDHVMYTIHMGCHGFRDPFECNMCGYHSQDRYEFSSHITRGEHRFHMS
(SEQ ID NO: 3)
Isoform 4 (UniProt Q13422-4) 
MDADEGQDMASNVKVETQSDEENGRACEMNGEECAEDLRMLDASGEKMNGS
HRDQGSSALSGVGGIRLPNGKLKCDICGIICIGPNVLMVHKRSHTGERPFQCNQC
GASFTQKGNLLRHIKLHSGEKPFKCHLCNYACRRRDALTGHLRTHSGDKGLSD
TPYDSSASYEKENEMMKSHVMDQAINNAINYLGAESLRPLVQTPPGGSEVVPVIS
PMYQLHKPLAEGTPRSNHSAQDSAVENLLLLSKAKLVPSEREASPSNSCQDSTDT
ESNNEEQRSGLIYLTNHIAPHARNGLSLKEEHRAYDLLRAASENSQDALRVVSTS
GEQMKVYKCEHCRVLFLDHVMYTIHMGCHGFRDPFECNMCGYHSQDRYEFSSH
ITRGEHRFHMS (SEQ ID NO: 4)
Isoform 7 (UniProt Q13422-7) 
MDADEGQDMSQVSGKESPPVSDTPDEGDEPMPIPEDLSTTSGGQQSSKSDRVVA
SNVKVETQSDEENGRACEMNGEECAEDLRMLDASGEKMNGSHRDQGSSALSGV
GGIRLPNGKLKCDICGIICIGPNVLMVHKRSHTGERPFQCNQCGASFTQKGNLL
RHIKLHSGEKPFKCHLCNYACRRRDALTGHLRTHSVIKEETNHSEMAEDLCKIG
SERSLVLDRLASNVAKRKSSMPQKFLGDKGLSDTPYDSSASYEKENEMMKSHV
MDQAINNAINYLGAESLRPLVQTPPGGSEVVPVISPMYQLHKPLAEGTPRSNHSA
QDSAVENLLLLSKAKLVPSEREASPSNSCQDSTDTESNNEEQRSGLIYLTNHIAPH
ARNGLSLKEEHRAYDLLRAASENSQDALRVVSTSGEQMKVYKCEHCRVLFLDH
VMYTIHMGCHGFRDPFECNMCGYHSQDRYEFSSHITRGEHRFHMS (SEQ ID NO:
5)
Isoform 8 (UniProt Q13422-8)

MDADEGQDMSQVSGKESPPVSDTPDEGDEPMPIPEDLSTTSGGQQSSKSDRVVA
SNVKVETQSDEENGRACEMNGEECAEDLRMLDASGEKMNGSHRDQGSSALSGV
GGIRLPNGKLKCDICGIICIGPNVLMVHKRSHTGERPFQCNQCGASFTQKGNLL
RHIKLHSGEKPFKCHLCNYACRRRDALTGHLRTHSVIKEETNHSEMAEDLCKIG
SEISRAGQTSK (SEQ ID NO: 6)

The "Ikaros" protein isoforms 1, 2, 3, 4, 7, and 8 listed above includes the degron **FQCNQCGASFTQKGNLLRHIKLH** (SEQ ID NO: 22), which is the same as the degron for the "Aiolos" protein. Ikaros protein also includes isoforms encoded by amino acid sequences Q13422-5 and Q13422- 6.

As used herein, "Helios" protein refers a protein that is a member of the Ikaros family of zinc finger proteins. In humans, Helios is encoded by the IKZF2 gene. Helios is also known as IKAROS family zinc finger 2, ANF1A2, ZNF1A2, ZNFN1A2, zinc finger protein, subfamily 1 A, 2, and Ikaros family zinc finger protein 2. As used herein Helios protein includes various isoform, which includes the isoforms listed below.
Isoform 1 (UniProt Q9UKS7-1) 
METEAIDGYITCDNELSPEREHSNMAIDLTSSTPNGQHASPSHMTSTNSVKLEMQ
SDEECDRKPLSREDEIRGHDEGSSLEEPLIESSEVADNRKVQELQGEGGIRLPNGK
LKCDVCGMVCIGPNVLMVHKRSHTGERPFHCNQCGASFTQKGNLLRHIKLHS
GEKPFKCPFCSYACRRRDALTGHLRTHSVGKPHKCNYCGRSYKQRSSLEEHKER
CHNYLQNVSMEAAGQVMSHHVPPMEDCKEQEPIMDNNISLVPFERPAVIEKLTG
NMGKRKSSTPQKFVGEKLMRFSYPDIHFDMNLTYEKEAELMQSHMMDQAINNA
ITYLGAEALHPLMQHPPSTIAEVAPVISSAYSQVYHPNRIERPISRETADSHENNM
DGPISLIRPKSRPQEREASPSNSCLDSTDSESSHDDHQSYQGHPALNPKRKQSPAY
MKEDVKALDTTKAPKGSLKDIYKVFNGEGEQIRAFKCEHCRVLFLDH\/MYT
IHMGCHGY RDPLECNICGYRSQDRYEFSSHIVRGEHTFH (SEQ ID NO: 7)
Isoform 2 (UniProt Q9UKS7-2) 
METEAIDGYITCDNELSPEREHSNMAIDLTSSTPNGQHASPSHMTSTNSVKLEMQ
SDEECDRKPLSREDEIRGHDEGSSLEEPLIESSEVADNRKVQELQGEGGIRLPNGE
RPFHCNQCGASFTQKGNLLRHIKLHSGEKPFKCPFCSYACRRRDALTGHLRTH
SVGKPHKCNYCGRSYKQRSSLEEHKERCHNYLQNVSMEAAGQVMSHHVPPME
DCKEQEPIMDNNISLVPFERPAVIEKLTGNMGKRKSSTPQKFVGEKLMRFSYPDIH
 
FDMNLTYEKEAELMQSHMMDQAINNAITYLGAEALHPLMQHPPSTIAEVAPVISS
AYSQVYHPNRIERPISRETADSHENNMDGPISLIRPKSRPQEREASPSNSCLDSTDS
ESSHDDHQSYQGHPALNPKRKQSPAYMKEDVKALDTTKAPKGSLKDIYKVFNG
EGEQIRAFKCEHCRVLFLDHT/MYTIHMGCHGYRDPLECNICGYRSQDRYE FS
SHIVRG EHTFH (SEQ ID NO: 8)
Isoform 4 (UniProt Q9UKS7-4) 
METEAIDGYITCDNELSPEREHSNMAIDLTSSTPNGQHASPSHMTSTNSVKLEMQ
SDEECDRKPLSREDEIRGHDEGSSLEEPLIESSEVADNRKVQELQGEGGIRLPNGE
RPFHCNQCGASFTQKGNLLRHIKLHSGEKPFKCPFCSYACRRRDALTGHLRTH
SVGKPHKCNYCGRSYKQRSSLEEHKERCHNYLQNVSMEAAGQVMSHHGEKLM
RFSYPDIHFDMNLTYEKEAELMQSHMMDQAINNAITYLGAEALHPLMQHPPSTI
AEVAPVISSAYSQVYHPNRIERPISRETADSHENNMDGPISLIRPKSRPQEREASPS
NSCLDSTDSESSHDDHQSYQGHPALNPKRKQSPAYMKEDVKALDTTKAPKGSLK
DIYKVFNGEGEQRAFKCEHCRVLFLDHVMYTIHMGCHGYRDPLECNICGYRSQD
RYEF SSHIVRGEHTFH (SEQ ID NO: 9)
Isoform 6 (UniProt Q9UKS7-6) 
METEAIDGYITCDNELSPEREHSNMAIDLTSSTPNGQHASPSHMTSTNSVKLEMQ
SDEECDRKPLSREDEIRGHDEGSSLEEPLIESSEVADNRKVQELQGEGGIRLPNGK
LKCDVCGMVCIGPNVLMVHKRSHTGERPFHCNQCGASFTQKGNLLRHIKLHS
GEKPFKCPFCSYACRRRDALTGHLRTHSVGKPHKCNYCGRSYKQRSSLEEHKER
CHNYLQNVSMEAAGQVMSHHDS (SEQ ID NO: 10)
Isoform 7 (UniProt Q9UKS7-7)

METEAIDGYITCDNELSPEREHSNMAIDLTSSTPNGQHASPSHMTSTNSVKLEMQ
SDEECDRKPLSREDEIRGHDEGSSLEEPLIESSEVADNRKVQELQGEGGIRLPNGE
RPFHCNQCGASFTQKGNLLRHIKLHSGEKPFKCPFCSYACRRRDALTGHLRTH
SVPPMEDCKEQEPIMDNNISLVPFERPAVIEKLTGNMGKRKSSTPQKFVGEKLMR
FSYPDIHFDMNLTYEKEAELMQSHMMDQAINNAITYLGAEALHPLMQHPPSTIAE
VAPVISSAYSQVYHPNRIERPISRETADSHENNMDGPISLIRPKSRPQEREASPSNS
CLDSTDSESSHDDHQSYQGHPALNPKRKQSPAYMKEDVKALDTTKAPKGSLKDI
YKVFNGEGEQIRAFKCEHCRVLFLDHVMYTIHMGCHGYRDPLECNICGYRSQDR
Y EFSSHIVRGEHTFH (SEQ ID NO: 11)

The "Helios" isoforms 1, 2, 4, 6, and 7 listed above includes the degron **FHCNQCGASFTQKGNLLRHIKLH** (SEQ ID NO: 23). A degron is a portion of a protein that plays a role in regulating protein degradation rates. Helios protein also includes isoforms encoded by amino acid sequences Q9UKS7-3, Q9UKS7-5 and Q9UKS7-8.

As used herein, "Aiolos" protein is encoded by the IKZF3 gene. Aiolos protein is also known as IKAROS family zinc finger 3, ZNFN1A3, zinc finger protein, subfamily 1 A, 3, Ikaros family zinc finger protein 3, and AIO. Aiolos protein includes the following human isoforms listed below:
Isoform 1 (UniProt Q9UKT9-1) 
MEDIQTNAELKSTQEQSVPAESAAVLNDYSLTKSHEMENVDSGEGPANEDEDIG
DDSMKVKDEYSERDENVLKSEPMGNAEEPEIPYSYSREYNEYENIKLERHVVSFD
SSRPTSGKMNCDVCGLSCISFNVLMVHKRSHTGERPFQCNQCGASFTQKGNLL
RHIKLHTGEKPFKCHLCNYACQRRDALTGHLRTHSVEKPYKCEFCGRSYKQRSS
LEEHKERCRTFLQSTDPGDTASAEARHIKAEMGSERALVLDRLASNVAKRKSSM
PQKFIGEKRHCFDVNYNSSYMYEKESELIQTRMMDQAINNAISYLGAEALRPLVQ
TPPAPTSEMVPVISSMYPIALTRAEMSNGAPQELEKKSIHLPEKSVPSERGLSPNNS
GHDSTDTDSNHEERQNHIYQQNHMVLSRARNGMPLLKEVPRSYELLKPPPICPRD
SVKVINKEGEVMDVYRCDHCRVLFLDYVMFTIHMGCHGFRDPFECNMCGYRSH
DRYEFSSHIARGEHRALLK (SEQ ID NO: 12)
Isoform 3 (UniProt Q9UKT9-3) 
MEDIQTNAELKSTQEQSVPAESAAVLNDYSLTKSHEMENVDSGEGPANEDEDIG
DDSMKVKDEYSERDENVLKSEPMGNAEEPEIPYSYSREYNEYENIKLERHVVSFD
SSRPTSGKMNCDVCGLSCISFNVLMVHKRSHTGERPFQCNQCGASFTQKGNLL
RHIKLHTGEKPFKCHLCNYACQRRDALTGHLRTHSASAEARHIKAEMGSERALV
LDRLASNVAKRKSSMPQKFIGEKRHCFDVNYNSSYMYEKESELIQTRMMDQAIN
NAISYLGAEALRPLVQTPPAPTSEMVPVISSMYPIALTRAEMSNGAPQELEKKSIH
LPEKSVPSERGLSPNNSGHDSTDTDSNHEERQNHIYQQNHMVLSRARNGMPLLK
EVPRSYELLKPPPICPRDSVKVINKEGEVMDVYRCDHCRVLFLDYVMFTIHMGC
HGFRDPFECNMCGYRSHDRYEFSSHIARGEHRALLK (SEQ ID NO: 13)
Isoform 4 (UniProt Q9UKT9-4) 
MEDIQTNAELKSTQEQSVPAESAAVLNDYSLTKSHEMENVDSGEGPANEDEDIG
DDSMKVKDEYSERDENVLKSEPMGNAEEPEIPYSYSREYNEYENIKLERHVVSFD
 
SSRPTSGKMNCDVCGLSCISFNVLMVHKRSHTGERPFQCNQCGASFTQKGNLL
RHIKLHTGEKPFKCHLCNYACQRRDALTGHLRTHSVEKPYKCEFCGRSYKQRSS
LEEHKERCRTFLQSTDPGDTGEKRHCFDVNYNSSYMYEKESELIQTRMMDQAIN
NAISYLGAEALRPLVQTPPAPTSEMVPVISSMYPIALTRAEMSNGAPQELEKKSIH
LPEKSVPSERGLSPNNSGHDSTDTDSNHEERQNHIYQQNHMVLSRARNGMPLLK
EVPRSYELLKPPPICPRDSVKVINKEGEVMDVYRCDHCRVLFLDYVMFTIHMGC
HGFRDPFECNMCGYRSHDRYEFSSHIARGEHRALLK (SEQ ID NO: 14)
Isoform 6 (UniProt Q9UKT9-6) 
MEDIQTNAELKSTQEQSVPAESAAVLNDYSLTKSHEMENVDSGEGPANEDEDIG
DDSMKVKDEYSERDENVLKSEPMGNAEEPEIPYSYSREYNEYENIKLERHVVSFD
SSRPTSGKMNCDVCGLSCISFNVLMVHKRSHTGERPFQCNQCGASFTQKGNLL
RHIKLHTGEKPFKCHLCNYACQRRDALTGHLRTHSGEKRHCFDVNYNSSYMYE
KESELIQTRMMDQAINNAISYLGAEALRPLVQTPPAPTSEMVPVISSMYPIALTRA
EMSNGAPQELEKKSIHLPEKSVPSERGLSPNNSGHDSTDTDSNHEERQNHIYQQN
HMVLSRARNGMPLLKEVPRSYELLKPPPICPRDSVKVINKEGEVMDVYRCDHCR
VLFLDYVMFTIHMGCHGFRDPFECNMCGYRSHDRYEFSSHIARGEHRALLK
(SEQ ID NO: 15)
Isoform 7 (UniProt Q9UKT9-7) 
MEDIQTNAELKSTQEQSVPADDSMKVKDEYSERDENVLKSEPMGNAEEPEIPYS
YSREYNEYENIKLERHVVSFDSSRPTSGKMNCDVCGLSCISFNVLMVHKRSHTGE
RPFQCNQCGASFTQKGNLLRHIKLHTGEKPFKCHLCNYACQRRDALTGHLRT
HSVEKPYKCEFCGRSYKQRSSLEEHKERCRTFLQSTDPGDTASAEARHIKAEMGS
ERALVLDRLASNVAKRKSSMPQKFIGEKRHCFDVNYNSSYMYEKESELIQTRMM
DQAINNAISYLGAEALRPLVQTPPAPTSEMVPVISSMYPIALTRAEMSNGAPQELE
KKSIHLPEKSVPSERGLSPNNSGHDSTDTDSNHEERQNHIYQQNHMVLSRARNG
MPLLKEVPRSYELLKPPPICPRDSVKVINKEGEVMDVYRCDHCRVLFLDYVMFTI
HMGCHGFRDPFECNMCGYRSHDRYEFSSHIARGEHRALLK (SEQ ID NO: 16)
Isoform 8 (UniProt Q9UKT9-8) 
MEDIQTNAELKSTQEQSVPADDSMKVKDEYSERDENVLKSEPMGNAEEPEIPYS
YSREYNEYENIKLERHVVSFDSSRPTSGKMNCDVCGLSCISFNVLMVHKRSHTGE
RPFQCNQCGASFTQKGNLLRHIKLHTGEKPFKCHLCNYACQRRDALTGHLRT
HSASAEARHIKAEMGSERALVLDRLASNVAKRKSSMPQKFIGEKRHCFDVNYNS
 
SYMYEKESELIQTRMMDQAINNAISYLGAEALRPLVQTPPAPTSEMVPVISSMYPI
ALTRAEMSNGAPQELEKKSIHLPEKSVPSERGLSPNNSGHDSTDTDSNHEERQNH
IYQQNHMVLSRARNGMPLLKEVPRSYELLKPPPICPRDSVKVINKEGEVMDVYR
CDHCRVLFLDYVMFTIHMGCHGFRDPFECNMCGYRSHDRYEFSSHIARGEHRAL
LK (SEQ ID NO: 17)
Isoform 9 (UniProt Q9UKT9-9) 
MEDIQTNAELKSTQEQSVPAESAAVLNDYSLTKSHEMENVDSGEGPANEDEDIG
GERPFQCNQCGASFTQKGNLLRHIKLHTGEKPFKCHLCNYACQRRDALTGHL
RTHSVEKPYKCEFCGRSYKQRSSLEEHKERCRTFLQSTDPGDTASAEARHIKAEM
GSERALVLDRLASNVAKRKSSMPQKFIGEKRHCFDVNYNSSYMYEKESELIQTR
MMDQAINNAISYLGAEALRPLVQTPPAPTSEMVPVISSMYPIALTRAEMSNGAPQ
ELEKKSIHLPEKSVPSERGLSPNNSGHDSTDTDSNHEERQNHIYQQNHMVLSRAR
NGMPLLKEVPRSYELLKPPPICPRDSVKVINKEGEVMDVYRCDHCRVLFLDYVM
FTIHMGCHGFRDPFECNMCGYRSHDRYEFSSHIARGEHRALLK (SEQ ID NO: 18)
Isoform 14 (UniProt Q9UKT9-14)

MEDIQTNAELKSTQEQSVPAESAAVLNDYSLTKSHEMENVDSGEGPANEDEDIG
DDSMKVKDEYSERDENVLKSEPMGNAEEPEIPYSYSREYNEYENIKLERHVVSFD
SSRPTSGKMNCDVCGLSCISFNVLMVHKRSHTGERPFQCNQCGASFTQKGNLL
RHIKLHTGEKPFKCHLCNYACQRRDALTGHLRTHSVEKPYKCEFCGRSYKQRSS
LEEHKERCRTFLQSTDPGDTGTGWGWVELSHLGIRLQDLNVPWCRLH (SEQ ID
NO: 19)

The "Aiolos" protein isoforms 1, 3, 4, 6, 7, 8, 9, and 14 listed above includes the degron **FQCNQCGASFTQKGNLLRHIKLH** (SEQ ID NO: 24), which is the same as the degron for the "Ikaros" protein. Aiolos protein also includes isoforms encoded by amino acid sequences Q9UKT9-2, Q9UKT9-5, Q9UKT9-10, Q9UKT9-11, Q9UKT9-12, and Q9UKT9-13, Q9UKT9-15, and Q9UKT9-16.

As used herein, "Eos" protein is encoded by the IKZF4 gene, and is also known as IKAROS family zinc finger 4, ZNFN1A4, zinc finger protein, subfamily 1A, 4, Ikaros family zinc finger protein 4, and KIAA1782. "Eos" protein includes isoforms encoded by the following two human isoforms 1 (Q9H2S9-1) and 2 (Q9H2S9-2):
Isoform 1 (UniProt Q9H2S9-1) MHTPPALPRRFQGGGRVRTPGSHRQGKDNLERDPSGGCVPDFLPQAQDSNHFIM 
ESLFCESSGDSSLEKEFLGAPVGPSVSTPNSQHSSPSRSLSANSIKVEMYSDEESSR
LLGPDERLLEKDDSVIVEDSLSEPLGYCDGSGPEPHSPGGIRLPNGKLKCDVCGM
VCIGPNVLMVHKRSHTGERPFHCNQCGASFTQKGNLLRHIKLHSGEKPFKCPF
CNYACRRRDALTGHLRTHSVSSPTVGKPYKCNYCGRSYKQQSTLEEHKERCHN
YLQSLSTEAQALAGQPGDEIRDLEMVPDSMLHSSSERPTFIDRLANSLTKRKRSTP
QKFVGEKQMRFSLSDLPYDVNSGGYEKDVELVAHHSLEPGFGSSLAFVGAEHLR
PLRLPPTNCISELTPVISSVYTQMQPLPGRLELPGSREAGEGPEDLADGGPLLYRPR
GPLTDPGASPSNGCQDSTDTESNHEDRVAGWSLPQGPPPQPPPTIWGRHSPAYAK
EDPKPQEGLLRGTPGPSKEVLRWGESGEPVKAFKCEHCRILFLDHVMFTIHMGCH
GFRDPFECNICGYHSQDRYEFSSHIVRGEHKVG (SEQ ID NO: 20)
Isoform 2 (UniProt Q9H2S9-2)

MDSRYLQLQLYLPSCSLLQGSGDSSLEKEFLGAPVGPSVSTPNSQHSSPSRSLSAN
SIKVEMYSDEESSRLLGPDERLLEKDDSVIVEDSLSEPLGYCDGSGPEPHSPGGIRL
PNGKLKCDVCGMVCIGPNVLMVHKRSHTGERPFHCNQCGASFTQKGNLLRHI
KLHSGEKPFKCPFCNYACRRRDALTGHLRTHSVSSPTVGKPYKCNYCGRSYKQQ
STLEEHKERCHNYLQSLSTEAQALAGQPGDEIRDLEMVPDSMLHSSSERPTFIDRL
ANSLTKRKRSTPQKFVGEKQMRFSLSDLPYDVNSGGYEKDVELVAHHSLEPGFG
SSLAFVGAEHLRPLRLPPTNCISELTPVISSVYTQMQPLPGRLELPGSREAGEGPED
LADGGPLLYRPRGPLTDPGASPSNGCQDSTDTESNHEDRVAGWSLPQGPPPQPPP
TIWGRHSPAYAKEDPKPQEGLLRGTPGPSKEVLRWGESGEPVKAFKCEHCR
ILFLDHVMFTIHMGCHGFRDPFECNICGYHSQDRYEFSSHIVRGEHKVG (SEQ ID
NO: 21)

The "Eos" protein isoforms 1 and 2 listed above includes the degron **FHCNQCGASFTQKGNLLRHIKLH** (SEQ ID NO: 25), which is the same as the degron for the "Helios" protein.

As used herein, "Pegasus" protein is also known as IKAROS family zinc finger 5, ZNFN1A5, zinc finger protein, subfamily 1A, 5, and Ikaros family zinc finger protein 5. Pegasus is encoded by the IKZF5 gene.

As used herein, the term "contacting" refers to the bringing together of indicated moieties in an *in vitro* system or an *in vivo* system. For example, "contacting" IKZF1-4 proteins with a compound of Formula (I) includes the administration of a compound of the present invention to an individual or patient, such as a human, having Ikaros protein, Helios protein, Aiolos protein, and Eos protein as well as, for example, introducing a compound of Formula (I) into a sample containing a cellular or purified preparation containing Ikaros protein, Helios protein, Aiolos protein, and Eos protein.

The terms "treat," "treating," and "treatment," as used herein, refer to any type of intervention or process performed on, or administering an active agent to, the subject with the objective of reversing, alleviating, ameliorating, inhibiting, or slowing down or preventing the progression, development, severity or recurrence of a symptom, complication, condition or biochemical indicia associated with a disease. In contrast, "prophylaxis" or "prevention" refers to administration to a subject who does not have a disease to prevent the disease from occurring. "Treat," "treating," and "treatment" does not encompass prophylaxis or prevention.

"Therapeutically effective amount" is intended to include an amount of a compound of the present invention alone or an amount of the combination of compounds claimed or an amount of a compound of the present invention in combination with other active ingredients effective to decrease the levels of the IKZF1-4 proteins in the cells, or effective to treat or prevent viral infections and proliferative disorders, such as cancer.

As used herein, the term "cell" is meant to refer to a cell that is *in vitro, ex vivo* or *in vivo.* In some embodiments, an *ex vivo* cell can be part of a tissue sample excised from an organism such as a mammal. In some embodiments, an *in vitro* cell can be a cell in a cell culture. In some embodiments, an *in vivo* cell is a cell living in an organism such as a mammal.

The term "patient" includes human subjects.

The phrase "pharmaceutically acceptable carrier" as used herein means a pharmaceutically acceptable material, composition or vehicle, such as a liquid or solid filler, diluent, excipient, manufacturing aid (e.g., lubricant, talc magnesium, calcium or zinc stearate, or steric acid), or solvent encapsulating material, involved in carrying or transporting the subject compound from one organ, or portion of the body, to another organ, or portion of the body. Each carrier must be "acceptable" in the sense of being compatible with the other ingredients of the formulation, including, *i.e.,* adjuvant, excipient or vehicle, such as diluents, preserving agents, fillers, flow regulating agents, disintegrating agents, wetting agents, emulsifying agents, suspending agents, sweetening agents, flavoring agents, perfuming agents, antibacterial agents, antifungal agents, lubricating agents and dispensing agents, depending on the nature of the mode of administration and dosage forms; and not injurious to the patient.

The term "pharmaceutical composition" means a composition comprising a compound of the invention in combination with at least one additional pharmaceutically acceptable carrier.

### UTILITY

It is to be understood, even if not explicitly stated herein, that references to methods for the treatment of a disease, disorder, or condition should be read as relating to a substance or composition (e.g. a compound of the invention) for use in such a method.

The compounds of Formula (I) are useful for the treatment of cancer.

The compounds of Formula (I) are useful for the treatment of a viral infection.

In one embodiment, the treatment of cancer in a patient comprises administering to said patient a therapeutically effective amount of a compound according to Formula (I), or a stereoisomer, a tautomer, or a pharmaceutically acceptable salt thereof.

In one instance of the disclosure (which is not claimed as such), a method is provided for the treatment of a viral infection in a patient comprising administering to said patient a therapeutically effective amount of a compound according to Formula (I), or a stereoisomer, a tautomer, or a pharmaceutically acceptable salt thereof.

In one embodiment, the treatment of cancer in a patient comprises administering to said patient a therapeutically effective amount of a compound having the structure: 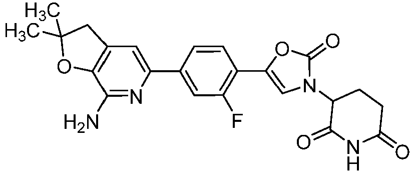 or a stereoisomer, a tautomer, or a pharmaceutically acceptable salt thereof.

In one embodiment, the treatment of cancer in a patient comprises administering to said patient a therapeutically effective amount of a compound having the structure: 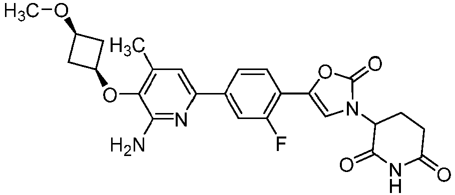 or a stereoisomer, a tautomer, or a pharmaceutically acceptable salt thereof.

In one embodiment, the treatment of cancer in a patient comprises administering to said patient a therapeutically effective amount of a compound having the structure: 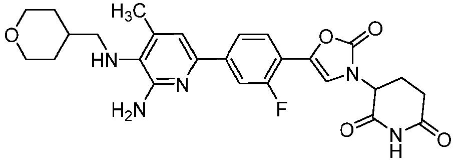 or a stereoisomer, a tautomer, or a pharmaceutically acceptable salt thereof.

In one embodiment, the treatment of cancer in a patient comprises administering to said patient a therapeutically effective amount of a compound having the structure: 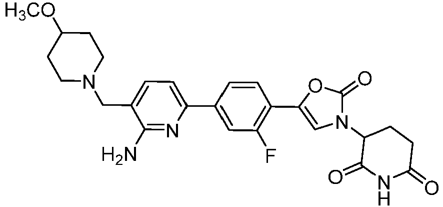 or a stereoisomer, a tautomer, or a pharmaceutically acceptable salt thereof.

One aspect provides an agent, which is a compound, or a stereoisomer, a tautomer, or a pharmaceutically acceptable salt thereof according to the invention, for use in a method of treating cancer by decreasing the levels of the four IKZF1-4 proteins Ikaros, Helios, Aiolos, and Eos, in cells by contacting said cells with said agent, wherein
a) said Ikaros protein level is decreased by at least 30 %;
b) said Helios protein level is decreased by at least 50 %;
c) said Aiolos protein level is decreased by at least 30 %; and
d) said Eos protein level is decreased by at least 50 %.

In one embodiment, the method comprises administering to said patient a therapeutically effective amount of the agent to decrease the Ikaros, Helios, Aiolos, and Eos protein levels (as defined herein), wherein: a) said Ikaros protein is the amino acid sequence encoded by SEQ ID NOs: 1, 2, 3, 4, 5, or 6; b) said Helios protein is the amino acid sequence encoded by SEQ ID NOs: 7,8, 9, 10, or 11; c) said Aiolos protein is the amino acid sequence encoded by SEQ ID NOs: 12, 13, 14, 15, 16, 17, 18, or 19; and d) said Eos protein is the amino acid sequence encoded by SEQ ID NOs: 20 or 21.

In Embodiment 1, the method comprises administering to said patient a therapeutically effective amount of the agent to decrease the Ikaros, Helios, Aiolos, and Eos protein levels, wherein: (i) said Ikaros (IKZF1) protein level is decreased by at least 30%; (ii) said Helios (IKZF2) protein level is decreased by at least 50%; (iii) said Aiolos (IKZF3) protein level is decreased by at least 30%; and (iv) said Eos (IKZF4) protein level is decreased by at least 50%.

In Embodiment 2, the method comprises administering to said patient a therapeutically effective amount of the agent to decrease the Ikaros, Helios, Aiolos, and Eos protein levels, wherein: (i) said Ikaros (IKZF1) protein level is decreased by at least 40%; (ii) said Helios (IKZF2) protein level is decreased by at least 50%; (iii) said Aiolos (IKZF3) protein level is decreased by at least 40%; and (iv) said Eos (IKZF4) protein level is decreased by at least 50%.

In Embodiment 3, the method comprises administering to said patient a therapeutically effective amount of the agent to decrease the Ikaros, Helios, Aiolos, and Eos protein levels, wherein: (i) said Ikaros (IKZF1) protein level is decreased by at least 50%; (ii) said Helios (IKZF2) protein level is decreased by at least 50%; (iii) said Aiolos (IKZF3) protein level is decreased by at least 50%; and (iv) said Eos (IKZF4) protein level is decreased by at least 50%.

In Embodiment 4, the method comprises administering to said patient a therapeutically effective amount of the agent to decrease the Ikaros, Helios, Aiolos, and Eos protein levels, wherein: (i) said Ikaros (IKZF1) protein level is decreased by at least 60%; (ii) said Helios (IKZF2) protein level is decreased by at least 50%; (iii) said Aiolos (IKZF3) protein level is decreased by at least 60%; and (iv) said Eos (IKZF4) protein level is decreased by at least 50%.

In Embodiment 5, the method comprises administering to said patient a therapeutically effective amount of the agent to decrease the Ikaros, Helios, Aiolos, and Eos protein levels, wherein: (i) said Ikaros (IKZF1) protein level is decreased by at least 30%; (ii) said Helios (IKZF2) protein level is decreased by at least 60%; (iii) said Aiolos (IKZF3) protein level is decreased by at least 30%; and (iv) said Eos (IKZF4) protein level is decreased by at least 60%.

In Embodiment 6, the method comprises administering to said patient a therapeutically effective amount of the agent to decrease the Ikaros, Helios, Aiolos, and Eos protein levels, wherein: (i) said Ikaros (IKZF1) protein level is decreased by at least 40%; (ii) said Helios (IKZF2) protein level is decreased by at least 60%; (iii) said Aiolos (IKZF3) protein level is decreased by at least 40%; and (iv) said Eos (IKZF4) protein level is decreased by at least 60%.

In Embodiment 7, the method comprises administering to said patient a therapeutically effective amount of the agent to decrease the Ikaros, Helios, Aiolos, and Eos protein levels, wherein: (i) said Ikaros (IKZF1) protein level is decreased by at least 50%; (ii) said Helios (IKZF2) protein level is decreased by at least 60%; (iii) said Aiolos (IKZF3) protein level is decreased by at least 50%; and (iv) said Eos (IKZF4) protein level is decreased by at least 60%.

In Embodiment 8, the method comprises administering to said patient a therapeutically effective amount of the agent to decrease the Ikaros, Helios, Aiolos, and Eos protein levels, wherein: (i) said Ikaros (IKZF1) protein level is decreased by at least 60%; (ii) said Helios (IKZF2) protein level is decreased by at least 60%; (iii) said Aiolos (IKZF3) protein level is decreased by at least 60%; and (iv) said Eos (IKZF4) protein level is decreased by at least 60%.

In Embodiment 9, the method comprises administering to said patient a therapeutically effective amount of the agent to decrease the Ikaros, Helios, Aiolos, and Eos protein levels, wherein: (i) said Ikaros (IKZF1) protein level is decreased by at least 30%; (ii) said Helios (IKZF2) protein level is decreased by at least 70%; (iii) said Aiolos (IKZF3) protein level is decreased by at least 30%; and (iv) said Eos (IKZF4) protein level is decreased by at least 65%.

In Embodiment 10, the method comprises administering to said patient a therapeutically effective amount of the agent to decrease the Ikaros, Helios, Aiolos, and Eos protein levels, wherein: (i) said Ikaros (IKZF1) protein level is decreased by at least 40%; (ii) said Helios (IKZF2) protein level is decreased by at least 70%; (iii) said Aiolos (IKZF3) protein level is decreased by at least 40%; and (iv) said Eos (IKZF4) protein level is decreased by at least 65%.

In Embodiment 11, the method comprises administering to said patient a therapeutically effective amount of the agent to decrease the Ikaros, Helios, Aiolos, and Eos protein levels, wherein: (i) said Ikaros (IKZF1) protein level is decreased by at least 50%; (ii) said Helios (IKZF2) protein level is decreased by at least 70%; (iii) said Aiolos (IKZF3) protein level is decreased by at least 50%; and (iv) said Eos (IKZF4) protein level is decreased by at least 65%.

In Embodiment 12, the method comprises administering to said patient a therapeutically effective amount of the agent to decrease the Ikaros, Helios, Aiolos, and Eos protein levels, wherein: (i) said Ikaros (IKZF1) protein level is decreased by at least 60%; (ii) said Helios (IKZF2) protein level is decreased by at least 70%; (iii) said Aiolos (IKZF3) protein level is decreased by at least 60%; and (iv) said Eos (IKZF4) protein level is decreased by at least 65%.

In Embodiment 13, the method comprises administering to said patient a therapeutically effective amount of the agent to decrease the Ikaros, Helios, Aiolos, and Eos protein levels, wherein: (i) said Ikaros (IKZF1) protein level is decreased by at least 30%; (ii) said Helios (IKZF2) protein level is decreased by at least 80%; (iii) said Aiolos (IKZF3) protein level is decreased by at least 30%; and (iv) said Eos (IKZF4) protein level is decreased by at least 60%.

In Embodiment 14, the method comprises administering to said patient a therapeutically effective amount of the agent to decrease the Ikaros, Helios, Aiolos, and Eos protein levels, wherein: (i) said Ikaros (IKZF1) protein level is decreased by at least 40%; (ii) said Helios (IKZF2) protein level is decreased by at least 80%; (iii) said Aiolos (IKZF3) protein level is decreased by at least 40%; and (iv) said Eos (IKZF4) protein level is decreased by at least 60%.

In Embodiment 15, the method comprises administering to said patient a therapeutically effective amount of the agent to decrease the Ikaros, Helios, Aiolos, and Eos protein levels, wherein: (i) said Ikaros (IKZF1) protein level is decreased by at least 50%; (ii) said Helios (IKZF2) protein level is decreased by at least 80%; (iii) said Aiolos (IKZF3) protein level is decreased by at least 50%; and (iv) said Eos (IKZF4) protein level is decreased by at least 60%.

In Embodiment 16, the method comprises administering to said patient a therapeutically effective amount of the agent to decrease the Ikaros, Helios, Aiolos, and Eos protein levels, wherein: (i) said Ikaros (IKZF1) protein level is decreased by at least 60%; (ii) said Helios (IKZF2) protein level is decreased by at least 80%; (iii) said Aiolos (IKZF3) protein level is decreased by at least 60%; and (iv) said Eos (IKZF4) protein level is decreased by at least 60%.

In Embodiment 17, the method comprises administering to said patient a therapeutically effective amount of the agent to decrease the Ikaros, Helios, Aiolos, and Eos protein levels, wherein: (i) said Ikaros (IKZF1) protein level is decreased by at least 30%; (ii) said Helios (IKZF2) protein level is decreased by at least 85%; (iii) said Aiolos (IKZF3) protein level is decreased by at least 30%; and (iv) said Eos (IKZF4) protein level is decreased by at least 60%.

In Embodiment 18, the method comprises administering to said patient a therapeutically effective amount of the agent to decrease the Ikaros, Helios, Aiolos, and Eos protein levels, wherein: (i) said Ikaros (IKZF1) protein level is decreased by at least 40%; (ii) said Helios (IKZF2) protein level is decreased by at least 85%; (iii) said Aiolos (IKZF3) protein level is decreased by at least 40%; and (iv) said Eos (IKZF4) protein level is decreased by at least 60%.

In Embodiment 19, the method comprises administering to said patient a therapeutically effective amount of the agent to decrease the Ikaros, Helios, Aiolos, and Eos protein levels, wherein: (i) said Ikaros (IKZF1) protein level is decreased by at least 50%; (ii) said Helios (IKZF2) protein level is decreased by at least 85%; (iii) said Aiolos (IKZF3) protein level is decreased by at least 50%; and (iv) said Eos (IKZF4) protein level is decreased by at least 60%.

In Embodiment 20, the method comprises administering to said patient a therapeutically effective amount of the agent to decrease the Ikaros, Helios, Aiolos, and Eos protein levels, wherein: (i) said Ikaros (IKZF1) protein level is decreased by at least 60%; (ii) said Helios (IKZF2) protein level is decreased by at least 85%; (iii) said Aiolos (IKZF3) protein level is decreased by at least 60%; and (iv) said Eos (IKZF4) protein level is decreased by at 60%.

In Embodiment 21, the method comprises administering to said patient a therapeutically effective amount of the agent to decrease the Ikaros, Helios, Aiolos, and Eos protein levels, wherein: (i) said Ikaros (IKZF1) protein level is decreased by at least 30%; (ii) said Helios (IKZF2) protein level is decreased by at least 90%; (iii) said Aiolos (IKZF3) protein level is decreased by at least 30%; and (iv) said Eos (IKZF4) protein level is decreased by at least 60%.

In Embodiment 22, the method comprises administering to said patient a therapeutically effective amount of the agent to decrease the Ikaros, Helios, Aiolos, and Eos protein levels, wherein: (i) said Ikaros (IKZF1) protein level is decreased by at least 40%; (ii) said Helios (IKZF2) protein level is decreased by at least 90%; (iii) said Aiolos (IKZF3) protein level is decreased by at least 40%; and (iv) said Eos (IKZF4) protein level is decreased by at least 60%.

In Embodiment 23, the method comprises administering to said patient a therapeutically effective amount of the agent to decrease the Ikaros, Helios, Aiolos, and Eos protein levels, wherein: (i) said Ikaros (IKZF1) protein level is decreased by at least 50%; (ii) said Helios (IKZF2) protein level is decreased by at least 90%; (iii) said Aiolos (IKZF3) protein level is decreased by at least 50%; and (iv) said Eos (IKZF4) protein level is decreased by at least 60%.

In Embodiment 24, the method comprises administering to said patient a therapeutically effective amount of the agent to decrease the Ikaros, Helios, Aiolos, and Eos protein levels, wherein: (i) said Ikaros (IKZF1) protein level is decreased by at least 60%; (ii) said Helios (IKZF2) protein level is decreased by at least 90%; (iii) said Aiolos (IKZF3) protein level is decreased by at least 60%; and (iv) said Eos (IKZF4) protein level is decreased by at least 60%.

In Embodiment 25, the method comprises administering to said patient a therapeutically effective amount of the agent to decrease the Ikaros, Helios, Aiolos, and Eos protein levels, wherein: (i) said Ikaros (IKZF1) protein level is decreased by at least 30%; (ii) said Helios (IKZF2) protein level is decreased by at least 90%; (iii) said Aiolos (IKZF3) protein level is decreased by at least 30%; and (iv) said Eos (IKZF4) protein level is decreased by at least 65%.

In Embodiment 26, the method comprises administering to said patient a therapeutically effective amount of the agent to decrease the Ikaros, Helios, Aiolos, and Eos protein levels, wherein: (i) said Ikaros (IKZF1) protein level is decreased by at least 40%; (ii) said Helios (IKZF2) protein level is decreased by at least 90%; (iii) said Aiolos (IKZF3) protein level is decreased by at least 40%; and (iv) said Eos (IKZF4) protein level is decreased by at least 65%.

In Embodiment 27, the method comprises administering to said patient a therapeutically effective amount of the agent to decrease the Ikaros, Helios, Aiolos, and Eos protein levels, wherein: (i) said Ikaros (IKZF1) protein level is decreased by at least 50%; (ii) said Helios (IKZF2) protein level is decreased by at least 90%; (iii) said Aiolos (IKZF3) protein level is decreased by at least 50%; and (iv) said Eos (IKZF4) protein level is decreased by at least 65%.

In Embodiment 28, the method comprises administering to said patient a therapeutically effective amount of the agent to decrease the Ikaros, Helios, Aiolos, and Eos protein levels, wherein: (i) said Ikaros (IKZF1) protein level is decreased by at least 60%; (ii) said Helios (IKZF2) protein level is decreased by at least 90%; (iii) said Aiolos (IKZF3) protein level is decreased by at least 60%; and (iv) said Eos (IKZF4) protein level is decreased by at least 65%.

In Embodiment 29, the method comprises administering to said patient a therapeutically effective amount of the agent to decrease the Ikaros, Helios, Aiolos, and Eos protein levels, wherein: (i) said Ikaros (IKZF1) protein level is decreased in the range of 40 to 70%; (ii) said Helios (IKZF2) protein level is decreased by at least 50%; (iii) said Aiolos (IKZF3) protein level is decreased in the range of 40 to 70%; and (iv) said Eos (IKZF4) protein level is decreased by at least 50%.

In Embodiment 30, the method comprises administering to said patient a therapeutically effective amount of the agent to decrease the Ikaros, Helios, Aiolos, and Eos protein levels, wherein: (i) said Ikaros (IKZF1) protein level is decreased in the range of 40 to 70%; (ii) said Helios (IKZF2) protein level is decreased by at least 60%; (iii) said Aiolos (IKZF3) protein level is decreased in the range of 40 to 70%; and (iv) said Eos (IKZF4) protein level is decreased by at least 60%.

In Embodiment 31, the method comprises administering to said patient a therapeutically effective amount of the agent to decrease the Ikaros, Helios, Aiolos, and Eos protein levels, wherein: (i) said Ikaros (IKZF1) protein level is decreased in the range of 40 to 70%; (ii) said Helios (IKZF2) protein level is decreased by at least 70%; (iii) said Aiolos (IKZF3) protein level is decreased in the range of 40 to 70%; and (iv) said Eos (IKZF4) protein level is decreased by at least 65%.

In Embodiment 32, the method comprises administering to said patient a therapeutically effective amount of the agent to decrease the Ikaros, Helios, Aiolos, and Eos protein levels, wherein: (i) said Ikaros (IKZF1) protein level is decreased in the range of 40 to 70%; (ii) said Helios (IKZF2) protein level is decreased by at least 70%; (iii) said Aiolos (IKZF3) protein level is decreased in the range of 40 to 70%; and (iv) said Eos (IKZF4) protein level is decreased by at least 70%.

In Embodiment 33, the method comprises administering to said patient a therapeutically effective amount of the agent to decrease the Ikaros, Helios, Aiolos, and Eos protein levels, wherein: (i) said Ikaros (IKZF1) protein level is decreased in the range of 40 to 70%; (ii) said Helios (IKZF2) protein level is decreased by at least 80%; (iii) said Aiolos (IKZF3) protein level is decreased in the range of 40 to 70%; and (iv) said Eos (IKZF4) protein level is decreased by at least 65%.

In Embodiment 34, the method comprises administering to said patient a therapeutically effective amount of the agent to decrease the Ikaros, Helios, Aiolos, and Eos protein levels, wherein: (i) said Ikaros (IKZF1) protein level is decreased in the range of 40 to 70%; (ii) said Helios (IKZF2) protein level is decreased by at least 90%; (iii) said Aiolos (IKZF3) protein level is decreased in the range of 40 to 70%; and (iv) said Eos (IKZF4) protein level is decreased by at least 65%.

In Embodiment 35, the method comprises administering to said patient a therapeutically effective amount of the agent to decrease the Ikaros, Helios, Aiolos, and Eos protein levels, wherein: (i) said Ikaros (IKZF1) protein level is decreased in the range of 50 to 70%; (ii) said Helios (IKZF2) protein level is decreased by at least 50%; (iii) said Aiolos (IKZF3) protein level is decreased in the range of 50 to 70%; and (iv) said Eos (IKZF4) protein level is decreased by at least 50%.

In Embodiment 36, the method comprises administering to said patient a therapeutically effective amount of the agent to decrease the Ikaros, Helios, Aiolos, and Eos protein levels, wherein: (i) said Ikaros (IKZF1) protein level is decreased in the range of 50 to 70%; (ii) said Helios (IKZF2) protein level is decreased by at least 60%; (iii) said Aiolos (IKZF3) protein level is decreased in the range of 50 to 70%; and (iv) said Eos (IKZF4) protein level is decreased by at least 60%.

In Embodiment 37, the method comprises administering to said patient a therapeutically effective amount of the agent to decrease the Ikaros, Helios, Aiolos, and Eos protein levels, wherein: (i) said Ikaros (IKZF1) protein level is decreased in the range of 50 to 70%; (ii) said Helios (IKZF2) protein level is decreased by at least 70%; (iii) said Aiolos (IKZF3) protein level is decreased in the range of 50 to 70%; and (iv) said Eos (IKZF4) protein level is decreased by at least 65%.

In Embodiment 38, the method comprises administering to said patient a therapeutically effective amount of the agent to decrease the Ikaros, Helios, Aiolos, and Eos protein levels, wherein: (i) said Ikaros (IKZF1) protein level is decreased in the range of 50 to 70%; (ii) said Helios (IKZF2) protein level is decreased by at least 70%; (iii) said Aiolos (IKZF3) protein level is decreased in the range of 50 to 70%; and (iv) said Eos (IKZF4) protein level is decreased by at least 70%.

In Embodiment 39, the method comprises administering to said patient a therapeutically effective amount of the agent to decrease the Ikaros, Helios, Aiolos, and Eos protein levels, wherein: (i) said Ikaros (IKZF1) protein level is decreased in the range of 50 to 70%; (ii) said Helios (IKZF2) protein level is decreased by at least 80%; (iii) said Aiolos (IKZF3) protein level is decreased in the range of 50 to 70%; and (iv) said Eos (IKZF4) protein level is decreased by at least 65%.

In Embodiment 40, the method comprises administering to said patient a therapeutically effective amount of the agent to decrease the Ikaros, Helios, Aiolos, and Eos protein levels, wherein: (i) said Ikaros (IKZF1) protein level is decreased in the range of 50 to 70%; (ii) said Helios (IKZF2) protein level is decreased by at least 90%; (iii) said Aiolos (IKZF3) protein level is decreased in the range of 50 to 70%; and (iv) said Eos (IKZF4) protein level is decreased by at least 90%.

In Embodiments 1 to 40, the decreases in the protein levels of the IKZF1-4 proteins can be measured using the following assays described hereinbelow: (i) IKZF1: Human CD8⁺ T Cell Reprogramming Assay; (ii) IKZF2: Jurkat Cellular Degradation Assay; (iii) IKZF3: Human CD8⁺ T Cell Reprogramming Assay; and (iv) IKZF4: Human Regulatory T Cell Reprogramming Assay.

Types of cancers that may be treated with the compound of Formula (I) include, but are not limited to, brain cancers, skin cancers, bladder cancers, ovarian cancers, breast cancers, gastric cancers, pancreatic cancers, prostate cancers, colon cancers, blood cancers, lung cancers and bone cancers. Examples of such cancer types include neuroblastoma, intestine carcinoma such as rectum carcinoma, colon carcinoma, anal cancer, familiar adenomatous polyposis carcinoma and hereditary non-polyposis colorectal cancer, esophageal carcinoma, nasopharyngeal carcinoma, labial carcinoma, larynx carcinoma, hypopharynx carcinoma, tongue carcinoma, salivary gland carcinoma, thymic carcinoma, esophagogastric cancer, gastric carcinoma, adenocarcinoma, medullary thyroid carcinoma, papillary thyroid carcinoma, renal carcinoma, kidney parenchymal carcinoma, ovarian carcinoma, cervix carcinoma, uterine corpus carcinoma, endometrium carcinoma, chorion carcinoma, pancreatic carcinoma, prostate carcinoma, testis carcinoma, breast carcinoma, urinary carcinoma, melanoma, brain tumors such as glioblastoma, astrocytoma, meningioma, medulloblastoma and peripheral neuroectodermal tumors, Hodgkin lymphoma, non-Hodgkin lymphoma, Burkitt lymphoma, acute lymphatic leukemia (ALL), chronic lymphatic leukemia (CLL), acute myeloid leukemia (AML), chronic myeloid leukemia (CML), adult T-cell leukemia lymphoma, diffuse large B-cell lymphoma (DLBCL), hepatocellular carcinoma, gall bladder carcinoma, bronchial carcinoma, small cell lung carcinoma, non-small cell lung carcinoma, mesothelioma, multiple myeloma, basalioma, teratoma, retinoblastoma, choroid melanoma, seminoma, rhabdomyosarcoma, craniopharyngioma, osteosarcoma, chondrosarcoma, myosarcoma, liposarcoma, fibrosarcoma, Ewing sarcoma and plasmocytoma.

In one embodiment, the treatment of cancer in a patient comprises administering to said patient a therapeutically effective amount of a compound according to Formula (I), or a stereoisomer, a tautomer, or a pharmaceutically acceptable salt thereof, wherein said cancer is melanoma.

In one embodiment, the treatment of cancer in a patient comprises administering to said patient a therapeutically effective amount of a compound according to Formula (I), or a stereoisomer, a tautomer, or a pharmaceutically acceptable salt thereof, wherein said cancer is lung cancer, including small cell lung cancer (SCLC) and non-small cell lung cancer (NSCLC).

In one embodiment, the treatment of cancer in a patient comprises administering to said patient a therapeutically effective amount of a compound according to Formula (I), or a stereoisomer, a tautomer, or a pharmaceutically acceptable salt thereof, wherein said cancer is mesothelioma.

In one embodiment, the treatment of cancer in a patient comprises administering to said patient a therapeutically effective amount of a compound according to Formula (I), or a stereoisomer, a tautomer, or a pharmaceutically acceptable salt thereof, wherein said cancer is breast cancer, including ductal carcinoma, invasive ductal carcinoma metastatic breast cancer, triple-negative breast cancer, human epidermal growth factor receptor 2 (HER2)-positive breast cancer, estrogen receptor (ER)-positive breast cancer, hormone receptor-positive breast cancer, and hormone receptor-negative breast cancer.

In one embodiment, the treatment of cancer in a patient comprises administering to said patient a therapeutically effective amount of a compound according to Formula (I), or a stereoisomer, a tautomer, or a pharmaceutically acceptable salt thereof, wherein said cancer is prostate cancer, including adenocarcinoma of the prostate and castration-resistant prostate cancer.

In one embodiment, the treatment of cancer in a patient comprises administering to said patient a therapeutically effective amount of a compound according to Formula (I), or a stereoisomer, a tautomer, or a pharmaceutically acceptable salt thereof, wherein said cancer is pancreatic cancer, including pancreatic adenocarcinoma, exocrine pancreatic cancer and neuroendocrine pancreatic cancer.

In one embodiment, the treatment of cancer in a patient comprises administering to said patient a therapeutically effective amount of a compound according to Formula (I), or a stereoisomer, a tautomer, or a pharmaceutically acceptable salt thereof, wherein said cancer is kidney cancer, including renal cell carcinoma, clear cell renal cell carcinoma, and non-clear cell renal cell carcinomas, papillary renal cell carcinoma, Wilms tumor, and renal sarcoma.

In one embodiment, the treatment of cancer in a patient comprises administering to said patient a therapeutically effective amount of a compound according to Formula (I), or a stereoisomer, a tautomer, or a pharmaceutically acceptable salt thereof, wherein said cancer is gastric cancer, including gastric carcinoma.

In one embodiment, the treatment of cancer in a patient comprises administering to said patient a therapeutically effective amount of a compound according to Formula (I), or a stereoisomer, a tautomer, or a pharmaceutically acceptable salt thereof, wherein said cancer is kidney cancer, including renal carcinoma and kidney parenchymal carcinoma.

In one embodiment, the treatment of cancer in a patient comprises administering to said patient a therapeutically effective amount of a compound according to Formula (I), or a stereoisomer, a tautomer, or a pharmaceutically acceptable salt thereof, wherein said cancer is liver cancer, including hepatocellular carcinoma.

In one embodiment, the treatment of cancer in a patient comprises administering to said patient a therapeutically effective amount of a compound according to Formula (I), or a stereoisomer, a tautomer, or a pharmaceutically acceptable salt thereof, wherein said cancer is ovarian cancer, including ovarian carcinoma.

In one embodiment, the treatment of cancer in a patient comprises administering to said patient a therapeutically effective amount of a compound according to Formula (I), or a stereoisomer, a tautomer, or a pharmaceutically acceptable salt thereof, wherein said cancer is lymphoma, including Hodgkin lymphoma, non-Hodgkin lymphoma, Burkitt lymphoma, acute lymphatic leukemia (ALL), chronic lymphatic leukemia (CLL), acute myeloid leukemia (AML), chronic myeloid leukemia (CML), adult T-cell leukemia, and diffuse large B-cell lymphoma (DLBCL).

In one embodiment, the treatment of cancer in a patient comprises administering to said patient a therapeutically effective amount of a compound according to Formula (I), or a stereoisomer, a tautomer, or a pharmaceutically acceptable salt thereof, wherein said cancer is leukemia, including acute lymphatic leukemia (ALL), chronic lymphatic leukemia (CLL), acute myeloid leukemia (AML), chronic myeloid leukemia (CML), adult T-cell leukemia lymphoma, and diffuse large B-cell lymphoma (DLBCL).

In one embodiment, the treatment of cancer in a patient comprises administering to said patient a therapeutically effective amount of a compound according to Formula (I), or a stereoisomer, a tautomer, or a pharmaceutically acceptable salt thereof, wherein said cancer is multiple myeloma.

The compounds for Formula (I) and pharmaceutical compositions comprising at least one compound of Formula (I) are useful in treating or preventing any diseases or conditions that are associated with the activity of IKZF 1-4 proteins. These include viral and other infections (*e.g*., skin infections, GI infection, urinary tract infections, genito-urinary infections, systemic infections), and proliferative diseases (*e.g*., cancer). Any method of administration may be used to deliver the compound or pharmaceutical composition to the patient. In certain embodiments, the compound of Formula (I) or pharmaceutical composition comprising at least compound of Formula (I) is administered orally. In other embodiments, the Formula (I) or pharmaceutical composition comprising at least compound of Formula (I) is administered parenterally.

In one instance (which is not claimed as such), a method is described for the treatment of a viral infection in a patient comprising administering to said patient a therapeutically effective amount of a compound according to Formula (I), or a stereoisomer, a tautomer, or a pharmaceutically acceptable salt thereof, wherein said viral infection is caused by exposure to HIV, hepatitis (A, B, or C), herpes virus (*e.g*., VZV, HSV-1, HAV-6, HSV-II, and CMV, Epstein Barr virus), adenovirus, influenza virus, flaviviruses, echovirus, rhinovirus, coxsackie virus, coronavirus, respiratory syncytial virus, mumps virus, rotavirus, measles virus, rubella virus, parvovirus, vaccinia virus, HTLV virus, dengue virus, papillomavirus, molluscum virus, poliovirus, rabies virus, JC virus and arboviral encephalitis virus.

The compounds of Formula (I) can selectively decrease the protein level of the four IKZF1-4 proteins in cells to control Treg differentiation and/or the immune regulatory state. For example, the compounds of Formula (I) can be used to selectively decrease the protein level, decrease the activity level and/or inhibit the expression level of each of the four IKZF1-4 proteins in the cells to control Treg differentiation and/or the immune regulatory state in a cell or in an individual in need of a decrease in the protein level, decrease in the activity level and/or inhibition of the expression level of each of the four IKZF1-4 proteins by administering an efficacious amount of a compound of Formula (I) or a stereoisomer, a tautomer, or a salt thereof.

In one instance (which is not claimed as such), a combined preparation of a compound of Formula (I), and/or a pharmaceutically acceptable salt thereof; and additional therapeutic agent(s) for simultaneous, separate or sequential use in the treatment and/or prophylaxis of multiple diseases or disorders associated with the activity of IKZF1-4 proteins is described. The combined preparation can be used to decrease the protein level, to decrease the protein activity level, and/or to inhibit the expression level of each of the four IKZF1-4 proteins.

The compound(s) of Formula (I) may be sequentially administered prior to administration of the immuno-oncology agent. The compound(s) of Formula (I) may be administered concurrently with the immuno-oncology agent. The compound(s) of Formula (I) may be sequentially administered after administration of the immuno-oncology agent.

Compounds of Formula (I) may be co-formulated with an immuno-oncology agent.

Immuno-oncology agents include, for example, a small molecule drug, antibody, or other biologic or small molecule. Examples of biologic immuno-oncology agents include, but are not limited to, cancer vaccines, antibodies, and cytokines. The antibody may be a monoclonal antibody. The monoclonal antibody may be humanized or human.

The immuno-oncology agent may be (i) an agonist of a stimulatory (including a co-stimulatory) receptor or (ii) an antagonist of an inhibitory (including a co-inhibitory) signal on T cells, both of which result in amplifying antigen-specific T cell responses (often referred to as immune checkpoint regulators).

Certain of the stimulatory and inhibitory molecules are members of the immunoglobulin super family (IgSF). One important family of membrane-bound ligands that bind to co-stimulatory or co-inhibitory receptors is the B7 family, which includes B7-1, B7-2, B7-H1 (PD-L1), B7-DC (PD-L2), B7-H2 (ICOS-L), B7-H3, B7-H4, B7-H5 (VISTA), and B7-H6. Another family of membrane bound ligands that bind to co-stimulatory or co-inhibitory receptors is the TNF family of molecules that bind to cognate TNF receptor family members, which includes CD40 and CD40L, OX-40, OX-40L, CD70, CD27L, CD30, CD30L, 4-1BBL, CD137 (4-1BB), TRAIL/Apo2-L, TRAILR1/DR4, TRAILR2/DR5, TRAILR3, TRAILR4, OPG, RANK, RANKL, TWEAKR/Fn14, TWEAK, BAFFR, EDAR, XEDAR, TACI, APRIL, BCMA, LTβR, LIGHT, DcR3, HVEM, VEGI/TL1A, TRAMP/DR3, EDAR, EDA1, XEDAR, EDA2, TNFR1, Lymphotoxin α/TNFβ, TNFR2, TNFα, LTβR, Lymphotoxin α 1β2, FAS, FASL, RELT, DR6, TROY, NGFR.

T cell responses can be stimulated by a combination of a compound of Formula (I) and one or more of (i) an antagonist of a protein that inhibits T cell activation (e.g., immune checkpoint inhibitors) such as CTLA-4, PD-1, PD-L1, PD-L2, LAG-3, TIM-3, Galectin 9, CEACAM-1, BTLA, CD69, Galectin-1, TIGIT, CD113, GPR56, VISTA, 2B4, CD48, GARP, PD1H, LAIR1, TIM-1, and TIM-4, and (ii) an agonist of a protein that stimulates T cell activation such as B7-1, B7-2, CD28, 4-1BB (CD137), 4-1BBL, ICOS, ICOS-L, OX40, OX40L, GITR, GITRL, CD70, CD27, CD40, DR3 and CD28H.

Other agents that can be combined with compounds of Formula (I) for the treatment of cancer include antagonists of inhibitory receptors on NK cells or agonists of activating receptors on NK cells. For example, compounds of Formula (I) can be combined with antagonists of KIR, such as lirilumab.

Yet other agents for combination therapies include agents that inhibit or deplete macrophages or monocytes, including but not limited to CSF-1R antagonists such as CSF-1R antagonist antibodies including RG7155 (WO11/70024, WO11/107553, WO11/131407, WO13/87699, WO13/119716, WO13/132044) or FPA-008 (WO11/140249; WO13169264; WO14/036357).

Compounds of Formula (I) can be used with one or more of agonistic agents that ligate positive costimulatory receptors, blocking agents that attenuate signaling through inhibitory receptors, antagonists, and one or more agents that increase systemically the frequency of anti-tumor T cells, agents that overcome distinct immune suppressive pathways within the tumor microenvironment (e.g., block inhibitory receptor engagement (e.g., PD-L1/PD-1 interactions), deplete or inhibit Tregs (e.g., using an anti-CD25 monoclonal antibody (e.g., daclizumab) or by ex vivo anti-CD25 bead depletion), inhibit metabolic enzymes such as IDO, or reverse/prevent T cell anergy or exhaustion) and agents that trigger innate immune activation and/or inflammation at tumor sites.

The immuno-oncology agent may be a CTLA-4 antagonist, such as an antagonistic CTLA-4 antibody. Suitable CTLA-4 antibodies include, for example, YERVOY (ipilimumab) or tremelimumab.

The immuno-oncology agent may be a PD-1 antagonist, such as an antagonistic PD-1 antibody. Suitable PD-1 antibodies include, for example, OPDIVO (nivolumab), KEYTRUDA (pembrolizumab), MEDI-0680 (AMP-514; WO2012/145493), LIBTAYO (cemiplimab), JEMPERLI (dostarlimab), and ZYNYZ (retifanlimab) The immuno-oncology agent may also include pidilizumab (CT-011), though its specificity for PD-1 binding has been questioned. Another approach to target the PD-1 receptor is the recombinant protein composed of the extracellular domain of PD-L2 (B7-DC) fused to the Fc portion of IgG1, called AMP-224.

The immuno-oncology agent may be a PD-L1 antagonist, such as an antagonistic PD-L1 antibody. Suitable PD-L1 antibodies include, for example, MPDL3280A (RG7446; WO2010/077634), durvalumab (MEDI4736), BMS-936559 (WO207/005874), MSB0010718C (WO2013/79174), TECENTRIQ (atezolizumab), and BAVENCIO (avelumab).

The immuno-oncology agent may be a LAG-3 antagonist, such as an antagonistic LAG-3 antibody. Suitable LAG3 antibodies include, for example, BMS-986016 (WO10/19570, WO14/08218), or IMP-731 or IMP-321 (WO08/132601, WO09/44273).

The immuno-oncology agent may be a CD137 (4-1BB) agonist, such as an agonistic CD137 antibody. Suitable CD137 antibodies include, for example, urelumab and PF-05082566 (WO12/32433).

The immuno-oncology agent may be a GITR agonist, such as an agonistic GITR antibody. Suitable GITR antibodies include, for example, BMS-986153, BMS-986156, TRX-518 (WO06/105021, WO09/009116) and MK-4166 (WO11/028683).

The immuno-oncology agent may be an IDO antagonist. Suitable IDO antagonists include, for example, INCB-024360 (WO206/122150, WO07/75598, WO08/36653, WO08/36642), indoximod, or NLG-919 (WO09/73620, WO09/1156652, WO11/56652, WO12/142237).

The immuno-oncology agent may be an OX40 agonist, such as an agonistic OX40 antibody. Suitable OX40 antibodies include, for example, MEDI-6383 or MEDI-6469.

The immuno-oncology agent may be an OX40L antagonist, such as an antagonistic OX40 antibody. Suitable OX40L antagonists include, for example, RG-7888 (WO06/029879).

The immuno-oncology agent may be a CD40 agonist, such as an agonistic CD40 antibody. In yet another embodiment, the immuno-oncology agent is a CD40 antagonist, such as an antagonistic CD40 antibody. Suitable CD40 antibodies include, for example, lucatumumab or dacetuzumab.

The immuno-oncology agent may be a CD27 agonist, such as an agonistic CD27 antibody. Suitable CD27 antibodies include, for example, varlilumab.

The immuno-oncology agent may be MGA271 (to B7H3) (WO11/109400).

The immuno-oncology agent may be an anti-TIGIT agent. Suitable anti-TIGIT agents include antibodies such as an BMS-986207, tiragolumab, or MK-7684.

The immuno-oncology agent may be a KRAS G12C inhibitor. Suitable KRAS G12C inhibitors include LUMAKRAS (sotorasib) or KRAZATI (adagrasib).

The combination therapy is intended to embrace administration of these therapeutic agents in a sequential manner, that is, wherein each therapeutic agent is administered at a different time, as well as administration of these therapeutic agents, or at least two of the therapeutic agents, in a substantially simultaneous manner. Substantially simultaneous administration can be accomplished, for example, by administering to the subject a single dosage form having a fixed ratio of each therapeutic agent or in multiple, single dosage forms for each of the therapeutic agents. Sequential or substantially simultaneous administration of each therapeutic agent can be effected by any appropriate route including, but not limited to, oral routes, intravenous routes, intramuscular routes, and direct absorption through mucous membrane tissues. The therapeutic agents can be administered by the same route or by different routes. For example, a first therapeutic agent of the combination selected may be administered by intravenous injection while the other therapeutic agents of the combination may be administered orally. Altematively, for example, all therapeutic agents may be administered orally or all therapeutic agents may be administered by intravenous injection. Combination therapy also can embrace the administration of the therapeutic agents as described above in further combination with other biologically active ingredients and non-drug therapies (*e.g*., surgery or radiation treatment.) Where the combination therapy further comprises a non-drug treatment, the non-drug treatment may be conducted at any suitable time so long as a beneficial effect from the co-action of the combination of the therapeutic agents and non-drug treatment is achieved. For example, in appropriate cases, the beneficial effect is still achieved when the non-drug treatment is temporally removed from the administration of the therapeutic agents, perhaps by days or even weeks.

One or more additional pharmaceutical agents or treatment methods such as, for example, anti-viral agents, chemotherapeutics or other anti-cancer agents, immune enhancers, immunosuppressants, radiation, anti-tumor and anti-viral vaccines, cytokine therapy (*e.g.*, IL-2 and GM-CSF), and/or tyrosine kinase inhibitors can be optionally used in combination with the compounds of Formula (I) for treatment of IKZF1-4 proteins associated diseases, disorders or conditions. The agents can be combined with the present compounds in a single dosage form, or the agents can be administered simultaneously or sequentially as separate dosage forms.

Suitable chemotherapeutic or other anti-cancer agents include, for example, alkylating agents (including, without limitation, nitrogen mustards, ethylenimine derivatives, alkyl sulfonates, nitrosoureas and triazenes) such as uracil mustard, chlormethine, cyclophosphamide (CYTOXAN^{®}), ifosfamide, melphalan, chlorambucil, pipobroman, triethylene-melamine, triethylenethiophosphoramine, busulfan, carmustine, lomustine, streptozocin, dacarbazine, and temozolomide.

In the treatment of melanoma, suitable agents for use in combination with the compounds of Formula (I) include: dacarbazine (DTIC), optionally, along with other chemotherapy drugs such as carmustine (BCNU) and cisplatin; the "Dartmouth regimen", which consists of DTIC, BCNU, cisplatin and tamoxifen; a combination of cisplatin, vinblastine, and DTIC, temozolomide or YERVOY^{™}. Compounds of Formula (I) may also be combined with immunotherapy drugs, including cytokines such as interferon alpha, interleukin 2, and tumor necrosis factor (TNF) in the treatment of melanoma.

Compounds of Formula (I) may also be used in combination with vaccine therapy in the treatment of melanoma. Antimelanoma vaccines are, in some ways, similar to the anti-virus vaccines which are used to prevent diseases caused by viruses such as polio, measles, and mumps. Weakened melanoma cells or parts of melanoma cells called antigens may be injected into a patient to stimulate the body's immune system to recognize and destroy melanoma cells.

Melanomas that are confined to the arms or legs may also be treated with a combination of agents including one or more compounds of Formula (I), using a hyperthermic isolated limb perfusion technique. This treatment protocol temporarily separates the circulation of the involved limb from the rest of the body and injects high doses of chemotherapy into the artery feeding the limb, thus providing high doses to the area of the tumor without exposing internal organs to these doses that might otherwise cause severe side effects. Usually, the fluid is warmed to 38.9 °C to 40 °C. Melphalan is the drug most often used in this chemotherapy procedure. This can be given with another agent called tumor necrosis factor (TNF).

Suitable chemotherapeutic or other anti-cancer agents include, for example, antimetabolites (including, without limitation, folic acid antagonists, pyrimidine analogs, purine analogs and adenosine deaminase inhibitors) such as methotrexate, 5-fluorouracil, floxuridine, cytarabine, 6-mercaptopurine, 6-thioguanine, fludarabine phosphate, pentostatine, and gemcitabine.

Suitable chemotherapeutic or other anti-cancer agents further include, for example, certain natural products and their derivatives (for example, vinca alkaloids, antitumor antibiotics, enzymes, lymphokines and epipodophyllotoxins) such as vinblastine, vincristine, vindesine, bleomycin, dactinomycin, daunorubicin, doxorubicin, epirubicin, idarubicin, ara-C, paclitaxel (Taxol), mithramycin, deoxyco-formycin, mitomycin-C, L-asparaginase, interferons (especially IFN-alpha), etoposide, and teniposide.

Other cytotoxic agents include navelbene, CPT-11, anastrazole, letrazole, capecitabine, reloxafine, and droloxafine.

Also suitable are cytotoxic agents such as epidophyllotoxin; an antineoplastic enzyme; a topoisomerase inhibitor; procarbazine; mitoxantrone; platinum coordination complexes such as cisplatin and carboplatin; biological response modifiers; growth inhibitors; antihormonal therapeutic agents; leucovorin; tegafur; and haematopoietic growth factors.

Other anti-cancer agent(s) include antibody therapeutics such as trastuzumab (HERCEPTIN^{®}), antibodies to costimulatory molecules such as CTLA-4, 4-1BB and PD-1, or antibodies to cytokines (IL-10 or TGF-β).

Other anti-cancer agents also include those that block immune cell migration such as antagonists to chemokine receptors, including CCR2 and CCR4.

Other anti-cancer agents also include those that augment the immune system such as adjuvants or adoptive T cell transfer.

Anti-cancer vaccines include dendritic cells, synthetic peptides, DNA vaccines and recombinant viruses.

The pharmaceutical composition of the invention may optionally include at least one signal transduction inhibitor (STI). A "signal transduction inhibitor" is an agent that selectively inhibits one or more vital steps in signaling pathways, in the normal function of cancer cells, thereby leading to apoptosis. Suitable STIs include, but are not limited to: (i) bcr/abl kinase inhibitors such as, for example, STI 571 (GLEEVEC^{®}); (ii) epidermal growth factor (EGF) receptor inhibitors such as, for example, kinase inhibitors (IRESSA^{®}, SSI-774) and antibodies (Imclone: C225 [Goldstein et al., Clin. Cancer Res., 1:1311-1318 (1995)], and Abgenix: ABX-EGF); (iii) her-2/neu receptor inhibitors such as farnesyl transferase inhibitors (FTI) such as, for example, L-744,832 (Kohl et al., Nat. Med., 1(8):792-797 (1995)); (iv) inhibitors of Akt family kinases or the Akt pathway, such as, for example, rapamycin (see, for example, Sekulic et al., Cancer Res., 60:3504-3513 (200)); (v) cell cycle kinase inhibitors such as, for example, flavopiridol and UCN-O1 (see, for example, Sausville, Curr. Med. Chem. Anti-Canc. Agents, 3:47-56 (203)); and (vi) phosphatidyl inositol kinase inhibitors such as, for example, LY294002 (see, for example, Vlahos et al., J. Biol. Chem., 269:5241-5248 (1994)). Alternatively, at least one STI and at least one compound of Formula (I) may be in separate pharmaceutical compositions. In a specific embodiment of the present invention, at least one compound of Formula (I) and at least one STI may be administered to the patient concurrently or sequentially. In other words, at least one compound of Formula (I) may be administered first, at least one STI may be administered first, or at least one compound of Formula (I) and at least one STI may be administered at the same time. Additionally, when more than one compound of Formula (I) and/or STI is used, the compounds may be administered in any order.

A pharmaceutical composition for the treatment of a chronic viral infection in a patient may comprise at least one compound of Formula (I), optionally, at least one chemotherapeutic drug, and, optionally, at least one antiviral agent, in a pharmaceutically acceptable carrier.

Also described herein (but not claimed as such) is a method for treating a chronic viral infection in a patient by administering an effective amount of the above pharmaceutical composition.

At least one compound of Formula (I) and at least one chemotherapeutic agent may be administered to the patient concurrently or sequentially. In other words, at least one compound of Formula (I) may be administered first, at least one chemotherapeutic agent may be administered first, or at least one compound of Formula (I) and the at least one STI may be administered at the same time. Additionally, when more than one compound of Formula (I) and/or chemotherapeutic agent is used, the compounds may be administered in any order. Similarly, any antiviral agent or STI may also be administered at any point in comparison to the administration of the compound of Formula (I).

Chronic viral infections that may be treated using the present combinatorial treatment include, but are not limited to, diseases caused by hepatitis C virus (HCV), human papilloma virus (HPV), cytomegalovirus (CMV), herpes simplex virus (HSV), Epstein-Barr virus (EBV), varicella zoster virus, coxsackie virus, and human immunodeficiency virus (HIV).

Suitable antiviral agents contemplated for use in combination with the compound of Formula (I) can comprise nucleoside and nucleotide reverse transcriptase inhibitors (NRTIs), non-nucleoside reverse transcriptase inhibitors (NNRTIs), protease inhibitors and other antiviral drugs.

Examples of suitable NRTIs include zidovudine (AZT); didanosine (ddl); zalcitabine (ddC); stavudine (d4T); lamivudine (3TC); abacavir (1592U89); adefovir dipivoxil [bis(POM)-PMEA]; lobucavir (BMS-180194); BCH-10652; emitricitabine [(-)-FTC]; beta-L-FD4 (also called beta-L-D4C and named beta-L-2',3'-dicleoxy-5-fluoro-cytidene); DAPD, ((-)-beta-D-2,6-diamino-purine dioxolane); and lodenosine (FddA). Typical suitable NNRTIs include nevirapine (BI-RG-587); delaviradine (BHAP, U-90152); efavirenz (DMP-266); PNU-142721; AG-1549; MKC-442 (1-(ethoxy-methyl)-5-(1-methylethyl)-6-(phenylmethyl)-(2,4(1H,3H)-pyrimidinedione); and (+)-calanolide A (NSC-675451) and B. Typical suitable protease inhibitors include saquinavir (Ro 31-8959); ritonavir (ABT-538); indinavir (MK-639); nelfnavir (AG-1343); amprenavir (141W94); lasinavir (BMS-234475); DMP-450; BMS-2322623; ABT-378; and AG-1549. Other antiviral agents include hydroxyurea, ribavirin, IL-2, IL-12, pentafuside and Yissum Project No.11607.

The combination therapy is intended to embrace administration of these therapeutic agents in a sequential manner, that is, wherein each therapeutic agent is administered at a different time, as well as administration of these therapeutic agents, or at least two of the therapeutic agents, in a substantially simultaneous manner. Substantially simultaneous administration can be accomplished, for example, by administering to the subject a single dosage form having a fixed ratio of each therapeutic agent or in multiple, single dosage forms for each of the therapeutic agents. Sequential or substantially simultaneous administration of each therapeutic agent can be effected by any appropriate route including, but not limited to, oral routes, intravenous routes, intramuscular routes, and direct absorption through mucous membrane tissues. The therapeutic agents can be administered by the same route or by different routes. For example, a first therapeutic agent of the combination selected may be administered by intravenous injection while the other therapeutic agents of the combination may be administered orally. Alternatively, for example, all therapeutic agents may be administered orally or all therapeutic agents may be administered by intravenous injection. Combination therapy also can embrace the administration of the therapeutic agents as described above in further combination with other biologically active ingredients and non-drug therapies (*e*.*g*., surgery or radiation treatment). Where the combination therapy further comprises a non-drug treatment, the non-drug treatment may be conducted at any suitable time so long as a beneficial effect from the co-action of the combination of the therapeutic agents and non-drug treatment is achieved. For example, in appropriate cases, the beneficial effect is still achieved when the non-drug treatment is temporally removed from the administration of the therapeutic agents, perhaps by days or even weeks.

### PHARMACEUTICAL COMPOSITIONS

The invention also provides pharmaceutical compositions which comprise a therapeutically effective amount of one or more of the compounds of Formula (I), formulated together with one or more pharmaceutically acceptable carriers (additives) and/or diluents, and optionally, one or more additional therapeutic agents described above.

The compounds of Formula (I) may be administered by any suitable route, preferably in the form of a pharmaceutical composition adapted to such a route, and in a dose effective for the treatment intended. The compounds and compositions of the compound of Formula (I) can be administered for any of the uses described herein by any suitable means, for example, orally, such as tablets, capsules (each of which includes sustained release or timed release formulations), pills, powders, granules, elixirs, tinctures, suspensions (including nanosuspensions, microsuspensions, spray-dried dispersions), syrups, and emulsions; sublingually; bucally; parenterally, such as by subcutaneous, intravenous, intramuscular, or intrasternal injection, or infusion techniques *(e*.*g*., as sterile injectable aqueous or non-aqueous solutions or suspensions); nasally, including administration to the nasal membranes, such as by inhalation spray; topically, such as in the form of a cream or ointment; or rectally such as in the form of suppositories. They can be administered alone, but generally will be administered with a pharmaceutical carrier selected on the basis of the chosen route of administration and standard pharmaceutical practice.

For oral administration, the pharmaceutical composition may be in the form of, for example, a tablet, capsule, liquid capsule, suspension, or liquid. The pharmaceutical composition is preferably made in the form of a dosage unit containing a particular amount of the active ingredient. For example, the pharmaceutical composition may be provided as a tablet or capsule comprising an amount of active ingredient in the range of from about 0.1 to 1000 mg, preferably from about 0.25 to 250 mg, and more preferably from about 0.5 to 100 mg. A suitable daily dose for a human or other mammal may vary widely depending on the condition of the patient and other factors, but can be determined using routine methods.

Any pharmaceutical composition contemplated herein can, for example, be delivered orally via any acceptable and suitable oral preparations. Exemplary oral preparations, include, but are not limited to, for example, tablets, troches, lozenges, aqueous and oily suspensions, dispersible powders or granules, emulsions, hard and soft capsules, liquid capsules, syrups, and elixirs. Pharmaceutical compositions intended for oral administration can be prepared according to any methods known in the art for manufacturing pharmaceutical compositions intended for oral administration. In order to provide pharmaceutically palatable preparations, a pharmaceutical composition in accordance with the invention can contain at least one agent selected from sweetening agents, flavoring agents, coloring agents, demulcents, antioxidants, and preserving agents.

A tablet can, for example, be prepared by admixing at least one compound of Formula (I) and/or at least one pharmaceutically acceptable salt thereof with at least one non-toxic pharmaceutically acceptable excipient suitable for the manufacture of tablets. Exemplary excipients include, but are not limited to, for example, inert diluents, such as, for example, calcium carbonate, sodium carbonate, lactose, calcium phosphate, and sodium phosphate; granulating and disintegrating agents, such as, for example, microcrystalline cellulose, sodium crosscarmellose, corn starch, and alginic acid; binding agents, such as, for example, starch, gelatin, polyvinyl-pyrrolidone, and acacia; and lubricating agents, such as, for example, magnesium stearate, stearic acid, and talc. Additionally, a tablet can either be uncoated, or coated by known techniques to either mask the bad taste of an unpleasant tasting drug, or delay disintegration and absorption of the active ingredient in the gastrointestinal tract thereby sustaining the effects of the active ingredient for a longer period. Exemplary water soluble taste masking materials, include, but are not limited to, hydroxypropyl-methylcellulose and hydroxypropylcellulose. Exemplary time delay materials, include, but are not limited to, ethyl cellulose and cellulose acetate butyrate.

Hard gelatin capsules can, for example, be prepared by mixing at least one compound of Formula (I) and/or at least one salt thereof with at least one inert solid diluent, such as, for example, calcium carbonate; calcium phosphate; and kaolin.

Soft gelatin capsules can, for example, be prepared by mixing at least one compound of Formula (I) and/or at least one pharmaceutically acceptable salt thereof with at least one water soluble carrier, such as, for example, polyethylene glycol; and at least one oil medium, such as, for example, peanut oil, liquid paraffin, and olive oil.

An aqueous suspension can be prepared, for example, by admixing at least one compound of Formula (I) and/or at least one pharmaceutically acceptable salt thereof with at least one excipient suitable for the manufacture of an aqueous suspension. Exemplary excipients suitable for the manufacture of an aqueous suspension, include, but are not limited to, for example, suspending agents, such as, for example, sodium carboxymethylcellulose, methylcellulose, hydroxypropylmethyl-cellulose, sodium alginate, alginic acid, polyvinyl-pyrrolidone, gum tragacanth, and gum acacia; dispersing or wetting agents, such as, for example, a naturally-occurring phosphatide, e.g., lecithin; condensation products of alkylene oxide with fatty acids, such as, for example, polyoxyethylene stearate; condensation products of ethylene oxide with long chain aliphatic alcohols, such as, for example heptadecaethylene-oxycetanol; condensation products of ethylene oxide with partial esters derived from fatty acids and hexitol, such as, for example, polyoxyethylene sorbitol monooleate; and condensation products of ethylene oxide with partial esters derived from fatty acids and hexitol anhydrides, such as, for example, polyethylene sorbitan monooleate. An aqueous suspension can also contain at least one preservative, such as, for example, ethyl and n-propyl p-hydroxybenzoate; at least one coloring agent; at least one flavoring agent; and/or at least one sweetening agent, including but not limited to, for example, sucrose, saccharin, and aspartame.

Oily suspensions can, for example, be prepared by suspending at least one compound of Formula (I) and/or at least one pharmaceutically acceptable salt thereof in either a vegetable oil, such as, for example, arachis oil; olive oil; sesame oil; and coconut oil; or in mineral oil, such as, for example, liquid paraffin. An oily suspension can also contain at least one thickening agent, such as, for example, beeswax; hard paraffin; and cetyl alcohol. In order to provide a palatable oily suspension, at least one of the sweetening agents already described hereinabove, and/or at least one flavoring agent can be added to the oily suspension. An oily suspension can further contain at least one preservative, including, but not limited to, for example, an anti-oxidant, such as, for example, butylated hydroxyanisol, and alpha-tocopherol.

Dispersible powders and granules can, for example, be prepared by admixing at least one compound of Formula (I) and/or at least one pharmaceutically acceptable salt thereof with at least one dispersing and/or wetting agent; at least one suspending agent; and/or at least one preservative. Suitable dispersing agents, wetting agents, and suspending agents are as already described above. Exemplary preservatives include, but are not limited to, for example, anti-oxidants, e.g., ascorbic acid. In addition, dispersible powders and granules can also contain at least one excipient, including, but not limited to, for example, sweetening agents; flavoring agents; and coloring agents.

An emulsion of at least one compound of Formula (I) and/or at least one pharmaceutically acceptable salt thereof can, for example, be prepared as an oil-in-water emulsion. The oily phase of the emulsions comprising compounds of Formula (I) may be constituted from known ingredients in a known manner. The oil phase can be provided by, but is not limited to, for example, a vegetable oil, such as, for example, olive oil and arachis oil; a mineral oil, such as, for example, liquid paraffin; and mixtures thereof. While the phase may comprise merely an emulsifier, it may comprise a mixture of at least one emulsifier with a fat or an oil or with both a fat and an oil. Suitable emulsifying agents include, but are not limited to, for example, naturally-occurring phosphatides, e.g., soybean lecithin; esters or partial esters derived from fatty acids and hexitol anhydrides, such as, for example, sorbitan monooleate; and condensation products of partial esters with ethylene oxide, such as, for example, polyoxyethylene sorbitan monooleate. Preferably, a hydrophilic emulsifier is included together with a lipophilic emulsifier which acts as a stabilizer. It is also preferred to include both an oil and a fat. Together, the emulsifier(s) with or without stabilizer(s) make-up the so-called emulsifying wax, and the wax together with the oil and fat make up the so-called emulsifying ointment base which forms the oily dispersed phase of the cream formulations. An emulsion can also contain a sweetening agent, a flavoring agent, a preservative, and/or an antioxidant. Emulsifiers and emulsion stabilizers suitable for use in the formulation of the present invention include Tween 60, Span 80, cetostearyl alcohol, myristyl alcohol, glyceryl monostearate, sodium lauryl sulfate, glyceryl distearate alone or with a wax, or other materials well known in the art.

The compounds of Formula (I) and/or at least one pharmaceutically acceptable salt thereof can, for example, also be delivered intravenously, subcutaneously, and/or intramuscularly via any pharmaceutically acceptable and suitable injectable form. Exemplary injectable forms include, but are not limited to, for example, sterile aqueous solutions comprising acceptable vehicles and solvents, such as, for example, water, Ringer's solution, and isotonic sodium chloride solution; sterile oil-in-water microemulsions; and aqueous or oleaginous suspensions.

Formulations for parenteral administration may be in the form of aqueous or non-aqueous isotonic sterile injection solutions or suspensions. These solutions and suspensions may be prepared from sterile powders or granules using one or more of the carriers or diluents mentioned for use in the formulations for oral administration or by using other suitable dispersing or wetting agents and suspending agents. The compounds may be dissolved in water, polyethylene glycol, propylene glycol, ethanol, corn oil, cottonseed oil, peanut oil, sesame oil, benzyl alcohol, sodium chloride, tragacanth gum, and/or various buffers. Other adjuvants and modes of administration are well and widely known in the pharmaceutical art. The active ingredient may also be administered by injection as a composition with suitable carriers including saline, dextrose, or water, or with cyclodextrin (i.e., Captisol), cosolvent solubilization (i.e., propylene glycol) or micellar solubilization (i.e., Tween 80).

The sterile injectable preparation may also be a sterile injectable solution or suspension in a non-toxic parenterally acceptable diluent or solvent, for example as a solution in 1,3-butanediol. Among the acceptable vehicles and solvents that may be employed are water, Ringer's solution, and isotonic sodium chloride solution. In addition, sterile, fixed oils are conventionally employed as a solvent or suspending medium. For this purpose, any bland fixed oil may be employed, including synthetic mono- or diglycerides. In addition, fatty acids such as oleic acid find use in the preparation of injectables.

A sterile injectable oil-in-water microemulsion can, for example, be prepared by 1) dissolving at least one compound of Formula (I) in an oily phase, such as, for example, a mixture of soybean oil and lecithin; 2) combining the Formula (I) containing oil phase with a water and glycerol mixture; and 3) processing the combination to form a microemulsion.

A sterile aqueous or oleaginous suspension can be prepared in accordance with methods already known in the art. For example, a sterile aqueous solution or suspension can be prepared with a non-toxic parenterally-acceptable diluent or solvent, such as, for example, 1,3-butane diol; and a sterile oleaginous suspension can be prepared with a sterile non-toxic acceptable solvent or suspending medium, such as, for example, sterile fixed oils, e.g., synthetic mono- or diglycerides; and fatty acids, such as, for example, oleic acid.

Pharmaceutically acceptable carriers are formulated according to a number of factors well within the purview of those of ordinary skill in the art. These include, without limitation: the type and nature of the active agent being formulated; the subject to which the agent-containing composition is to be administered; the intended route of administration of the composition; and the therapeutic indication being targeted. Pharmaceutically acceptable carriers include both aqueous and non-aqueous liquid media, as well as a variety of solid and semi-solid dosage forms. Such carriers can include a number of different ingredients and additives in addition to the active agent, such additional ingredients being included in the formulation for a variety of reasons, e.g., stabilization of the active agent, binders, etc., well known to those of ordinary skill in the art. Descriptions of suitable pharmaceutically acceptable carriers, and factors involved in their selection, are found in a variety of readily available sources such as, for example, Allen, L. V. Jr. et al. Remington: The Science and Practice of Pharmacy (2 Volumes), 22nd Edition (2012), Pharmaceutical Press.

Pharmaceutically acceptable carriers, adjuvants, and vehicles that may be used in the pharmaceutical compositions of this invention include, but are not limited to, ion exchangers, alumina, aluminum stearate, lecithin, self-emulsifying drug delivery systems (SEDDS) such as d-alpha-tocopherol polyethyleneglycol 1000 succinate, surfactants used in pharmaceutical dosage forms such as Tweens, polyethoxylated castor oil such as CREMOPHOR surfactant (BASF), or other similar polymeric delivery matrices, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat. Cyclodextrins such as alpha-, beta-, and gamma-cyclodextrin, or chemically modified derivatives such as hydroxyalkylcyclodextrins, including 2- and 3-hydroxypropyl-cyclodextrins, or other solubilized derivatives may also be advantageously used to enhance delivery of compounds of the formulae described herein.

The pharmaceutically active compounds of this invention can be processed in accordance with conventional methods of pharmacy to produce medicinal agents for administration to patients, including humans and other mammals. The pharmaceutical compositions may be subjected to conventional pharmaceutical operations such as sterilization and/or may contain conventional adjuvants, such as preservatives, stabilizers, wetting agents, emulsifiers, buffers etc. Tablets and pills can additionally be prepared with enteric coatings. Such compositions may also comprise adjuvants, such as wetting, sweetening, flavoring, and perfuming agents.

For therapeutic purposes, the active compounds of this invention are ordinarily combined with one or more adjuvants appropriate to the indicated route of administration. If administered orally, the compounds may be admixed with lactose, sucrose, starch powder, cellulose esters of alkanoic acids, cellulose alkyl esters, talc, stearic acid, magnesium stearate, magnesium oxide, sodium and calcium salts of phosphoric and sulfuric acids, gelatin, acacia gum, sodium alginate, polyvinylpyrrolidone, and/or polyvinyl alcohol, and then tableted or encapsulated for convenient administration. Such capsules or tablets may contain a controlled-release formulation as may be provided in a dispersion of active compound in hydroxypropylmethyl cellulose.

The amounts of compounds that are administered and the dosage regimen for treating a disease condition with the compounds and/or compositions of this invention depends on a variety of factors, including the age, weight, sex, the medical condition of the subject, the type of disease, the severity of the disease, the route and frequency of administration, and the particular compound employed. Thus, the dosage regimen may vary widely, but can be determined routinely using standard methods. A daily dose of about 0.001 to 100 mg/kg body weight, preferably between about 0.0025 and about 50 mg/kg body weight and most preferably between about 0.005 to 10 mg/kg body weight, may be appropriate. The daily dose can be administered in one to four doses per day. Other dosing schedules include one dose per week and one dose per two day cycle.

Pharmaceutical compositions of this disclosure may comprise at least one compound of Formula (I) and/or at least one pharmaceutically acceptable salt thereof, and optionally an additional agent selected from any pharmaceutically acceptable carrier, adjuvant, and vehicle. Alternate compositions may comprise a compound of the Formula (I) described herein, or a prodrug thereof, and a pharmaceutically acceptable carrier, adjuvant, or vehicle.

The present disclosure also includes pharmaceutical kits useful, for example, in the treatment or prevention of IKZF1-4 protein-associated diseases or disorders, and other diseases referred to herein which include one or more containers containing a pharmaceutical composition comprising a therapeutically effective amount of a compound of Formula (I). Such kits (which are not claimed as such) can further include, if desired, one or more of various conventional pharmaceutical kit components, such as, for example, containers with one or more pharmaceutically acceptable carriers, additional containers, as will be readily apparent to those skilled in the art. Instructions, either as inserts or as labels, indicating quantities of the components to be administered, guidelines for administration, and/or guidelines for mixing the components, can also be included in the kit.

The dosage regimen for the compounds of the present invention will, of course, vary depending upon known factors, such as the pharmacodynamic characteristics of the particular agent and its mode and route of administration; the species, age, sex, health, medical condition, and weight of the recipient; the nature and extent of the symptoms; the kind of concurrent treatment; the frequency of treatment; the route of administration, the renal and hepatic function of the patient, and the effect desired.

By way of general guidance, the daily oral dosage of each active ingredient, when used for the indicated effects, will range between about 0.001 to about 5000 mg per day, preferably between about 0.01 to about 1000 mg per day, and most preferably between about 0.1 to about 250 mg per day. Intravenously, the most preferred doses will range from about 0.01 to about 10 mg/kg/minute during a constant rate infusion. Compounds of Formula (I) may be administered in a single daily dose, or the total daily dosage may be administered in divided doses of two, three, or four times daily.

The compounds are typically administered in admixture with suitable pharmaceutical diluents, excipients, or carriers (collectively referred to herein as pharmaceutical carriers) suitably selected with respect to the intended form of administration, e.g., oral tablets, capsules, elixirs, and syrups, and consistent with conventional pharmaceutical practices.

Dosage forms (pharmaceutical compositions) suitable for administration may contain from about 1 milligram to about 200 milligrams of active ingredient per dosage unit. In these pharmaceutical compositions the active ingredient will ordinarily be present in an amount of about 0.1-95% by weight based on the total weight of the composition.

A typical capsule for oral administration contains at least one of the compounds of Formula (I) (250 mg), lactose (75 mg), and magnesium stearate (15 mg). The mixture is passed through a 60 mesh sieve and packed into a No. 1 gelatin capsule.

A typical injectable preparation is produced by aseptically placing at least one of the compounds of Formula (I) (250 mg) into a vial, aseptically freeze-drying and sealing. For use, the contents of the vial are mixed with 2 mL of physiological saline, to produce an injectable preparation.

The present invention includes within its scope pharmaceutical compositions comprising, as an active ingredient, a therapeutically effective amount of at least one of the compounds of Formula (I), alone or in combination with a pharmaceutical carrier. Optionally, compounds of Formula (I) can be used alone, in combination with other compounds of Formula (I), or in combination with one or more other therapeutic agent(s), *e*.*g*., an anticancer agent or other pharmaceutically active material.

Regardless of the route of administration selected, the compounds of Formula (I), which may be used in a suitable hydrated form, and/or the pharmaceutical compositions of the present invention, are formulated into pharmaceutically acceptable dosage forms by conventional methods known to those of skill in the art.

Actual dosage levels of the active ingredients in the pharmaceutical compositions of this invention may be varied so as to obtain an amount of the active ingredient which is effective to achieve the therapeutic response for a particular patient, composition, and mode of administration, without being toxic to the patient.

The selected dosage level will depend upon a variety of factors including the activity of the particular compound of Formula (I) employed, or the ester, salt or amide thereof, the route of administration, the time of administration, the rate of excretion or metabolism of the particular compound being employed, the rate and extent of absorption, the duration of the treatment, other drugs, compounds and/or materials used in combination with the particular compound employed, the age, sex, weight, condition, general health and prior medical history of the patient being treated, and like factors well known in the medical arts.

A physician or veterinarian having ordinary skill in the art can readily determine and prescribe the effective amount of the pharmaceutical composition required. For example, the physician or veterinarian could start doses of the compounds of Formula (I) employed in the pharmaceutical composition at levels lower than that required in order to achieve the therapeutic effect and gradually increase the dosage until the effect is achieved.

In general, a suitable daily dose of a compound of Formula (I) will be that amount of the compound which is the lowest dose effective to produce a therapeutic effect. Such an effective dose will generally depend upon the factors described above. Generally, oral, intravenous, intracerebroventricular and subcutaneous doses of the compounds of Formula (I) for a patient will range from about 0.01 to about 50 mg per kilogram of body weight per day.

If desired, the effective daily dose of the active compound may be administered as two, three, four, five, six or more sub-doses administered separately at appropriate intervals throughout the day, optionally, in unit dosage forms. In certain aspects of the invention, dosing is one administration per day.

While it is possible for a compound of Formula (I) to be administered alone, it is preferable to administer the compound as a pharmaceutical formulation (composition).

The above other therapeutic agents, when employed in combination with the compounds of Formula (I), may be used, for example, in those amounts indicated in the Physicians' Desk Reference (PDR) or as otherwise determined by one of ordinary skill in the art. In the methods of the present invention, such other therapeutic agent(s) may be administered prior to, simultaneously with, or following the administration of the inventive compounds.

### METHODS OF PREPARATION

The compounds of the present invention can be prepared in a number of ways well known to one skilled in the art of organic synthesis. The compounds of the present invention can be synthesized using the methods described below, together with synthetic methods known in the art of synthetic organic chemistry, or variations thereon as appreciated by those skilled in the art. Preferred methods include, but are not limited to, those described below.

The compounds of this invention may be prepared using the reactions and techniques described in this section. The reactions are performed in solvents appropriate to the reagents and materials employed and are suitable for the transformations being effected. Also, in the description of the synthetic methods described below, it is to be understood that all proposed reaction conditions, including choice of solvent, reaction atmosphere, reaction temperature, duration of the experiment and work up procedures, are chosen to be the conditions standard for that reaction, which should be readily recognized by one skilled in the art. It is understood by one skilled in the art of organic synthesis that the functionality present on various portions of the molecule must be compatible with the reagents and reactions proposed. Such restrictions to the substituents that are compatible with the reaction conditions will be readily apparent to one skilled in the art and alternate methods must then be used. This will sometimes require a judgment to modify the order of the synthetic steps or to select one particular process scheme over another in order to obtain a compound of the invention. It will also be recognized that another major consideration in the planning of any synthetic route in this field is the judicious choice of the protecting group used for protection of the reactive functional groups present in the compounds described in this invention. An authoritative account describing the many alternatives to the trained practitioner is Greene and Wuts (Protective Groups In Organic Synthesis, Fourth Edition, Wiley and Sons, 2007).

### EXAMPLES

The following examples illustrate the particular embodiments of the present invention and disclosure and do not limit the scope of the present invention. Chemical abbreviations and symbols as well as scientific abbreviations and symbols have their usual and customary meanings unless otherwise specified. Additional abbreviations employed in the Examples and elsewhere in this application are defined above. Common intermediates are generally useful for the preparation of more than one Example. Compounds of the Examples are identified by the example and step in which they were prepared (e.g., "1-A" denotes the Example 1, step A), or by the example only where the compound is the title compound of the example (for example, "1" denotes the title compound of Example 1). In some instances, alternate preparations of intermediates or examples are described. Frequently chemists skilled in the art of synthesis may devise alternative preparations which may be desirable based on one or more considerations such as shorter reaction time, less expensive starting materials, ease of operation or isolation, improved yield, amenable to catalysis, avoidance of toxic reagents, accessibility of specialized instrumentation, and decreased number of linear steps, etc. The intent of describing alternative preparations is to further enable the preparation of the examples of this invention. In some instances, some functional groups in the outlined examples and claims may be replaced by well-known bioisosteric replacements known in the art, for example, replacement of a carboxylic acid group with a tetrazole or a phosphate moiety.

### ABBREVIATIONS

- ACN: acetonitrile
- BINAP: 2,2'-bis(diphenylphosphino)-1,1'-binaphthyl
- BuLi: n-butyl lithium
- CatacXium PD G3: mesylate[(di(1-adamantyl)-n-butylphosphine)-2-(2'-amino-1,1'-biphenyl)]palladium(II)
- CDI: carbonyldiimidazole
- DBU: 1,8-diazabicyclo[5.4.0]undec-7-ene
- DCM: dichloromethane
- DIAD: diisopropyl azodicarboxylate
- DIPEA: *N,N*-diisopropylethylamine
- DME: dimethyl ether
- DMF: dimethylformamide
- DMSO: dimethyl sulfoxide
- dppf: bis(diphenylphosphino)ferrocene
- EtOAc: ethyl acetate
- HPLC: High Performance Liquid Chromatography
- Me: methyl
- MeOH: methanol
- min: minute(s)
- mL: milliliter(s)
- NCS: *N*-chlorosuccinimide
- NH₄OAc: ammonium acetate
- NMP: *N*-methylpyrrolidinone
- pet ether: petroleum ether
- Pd₂(dba)₃: tris-(dibenzylideneacetone)dipalladium
- SEM-Cl: 2-trimethylsilyl)ethoxymethyl chloride
- TEA: triethylamine
- TEMED: tetramethylethylenediamine
- TFA: trifluoroacetic acid
- THF: tetrahydrofuran
- XPhos Pd G2: chloro(2-dicyclohexylphosphino-2',4',6'-triisopropyl-1,1'-biphenyl)[2-(2'-amino-1,1'-biphenyl)]palladium(II)

### ANALYTICAL LCMS CONDITIONS

Method A: ACQUITY UPLC^{®} BEH C18 (3.0 x 50 mm) 1.7 µm; Mobile Phase A: 95:5 water:acetonitrile with 2.5 mM NH₄OAc; Mobile Phase B: 5:95 water:acetonitrile with 2.5 mM NH₄OAc; Temperature: 40 °C; Gradient: 2.0 %B to 100 %B over 2 min; flow: 0.7 mL/min; Detection: MS and UV (220 nm).

Method B: Column: XBridge BEH XP C18 (50 x 2.1) mm, 2.5 µm; Mobile Phase A: 95:5 water:acetonitrile with 10 mM NH₄OAc; Mobile Phase B: 5:95 water:acetonitrile with 10 mM NH₄OAc; Temperature: 50 °C; Gradient: 0 %B to 100 %B over 3 min; Flow: 1.1 mL/min; Detection: MS and UV (220 nm).

### SYNTHESIS OF OXAZOLONE INTERMEDIATE A 3-(5-(2-Fluoro-4-(4,4,5,5-tetramethyl-1,3,2-dioxaborolan-2-yl)phenyl)-2-oxooxazol-3(2H)-yl)-1-((2-(trimethylsilyl)ethoxy)methyl)piperidine-2,6-dione

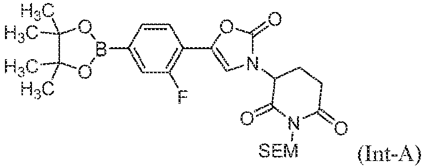

### Preparation of Int-A1 : 2-bromo-1-(4-bromo-2-fluorophenyl)ethan-1-one

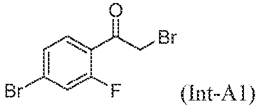

To a stirred solution of 1-(4-bromo-2-fluorophenyl)ethan-1-one (7.5 g, 34.6 mmol) in THF (75 mL) at 0 °C, pyridine hydrobromide perbromide (13.26 g, 41.47 mmol) was slowly added. The reaction mixture was warmed to room temperature and stirred for 3 h. Then, 20% aqueous sodium bisulfite solution was added, and the reaction mixture was stirred for 2 h at room temperature. The reaction mixture was diluted with water and extracted with EtOAc (3 x 100 mL). The combined organic phase was washed with brine solution, dried over anhydrous Na₂SO₄, filtered, and concentrated *in vacuo* to get a crude residue, which was purified by flash column chromatography (SiO₂, 10-50% DCM in pet ether) to afford 2-bromo-1-(4-bromo-2-fluorophenyl)ethan-1-one (6.5 g, 57%). LCMS (Method A) retention time 1.64 min, [M-H]⁺ 294.8; ¹H NMR (400 MHz, CDCl₃) δ ppm 7.84 (t, *J* = 8.0 Hz, 1H), 7.50-7.35 (m, 2H), 4.49 (d, *J* = 2.5 Hz, 2H).

### Preparation of Int-A2: 3-((2-(4-bromo-2-fluorophenyl)-2-oxoethyl)amino) piperidine-2,6-dione

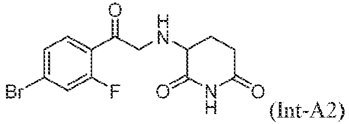

To a stirred solution of 3-aminopiperidine-2,6-dione, HCl (2.2.25 g, 135 mmol) in THF (200 mL) at room temperature under nitrogen atmosphere, was added K₂CO₃ (20 g, 145 mmol) and the reaction mixture was stirred for 15 min. Next, 2-bromo-1-(4-bromo-2-fluorophenyl)ethan-1-one (20 g, 67.6 mmol) was added in portions to the reaction mixture and heated at 70 °C for 2 h. Then, the reaction mixture was cooled to room temperature, and concentrated *in vacuo.* Water (200 mL) was added to yield a solid precipitate which was filtered through a Buchner funnel and dried *in vacuo* to afford 3-((2-(4-bromo-2-fluorophenyl)-2-oxoethyl)amino)piperidine-2,6-dione (19 g, 71%) as an off-white solid. LCMS (Method A): retention time 0.87 min, [M+H]⁺ 343.0.

### Preparation of Int-A3: 3-(5-(4-bromo-2-fluorophenyl)-2-oxooxazol-3(2H)-yl) piperidine-2,6-dione

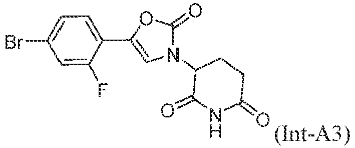

To a stirred solution of 3-((2-(-4-bromo-2-fluorophenyl)-2-oxoethyl)amino) piperidine-2,6-dione (30 g, 87 mmol) in THF (300 mL) at room temperature under nitrogen atmosphere, were added K₂CO₃ (12.08 g, 87 mmol) and CDI (28.4 g, 175 mmol). The reaction mixture was stirred at room temperature for 16 h and then concentrated *in vacuo* to remove excess solvent. Water (400 mL) was added to yield a solid precipitate which was filtered through a Buchner funnel and dried *in vacuo* to afford 3-(5-(4-bromo-2-fluorophenyl)-2-oxooxazol-3(2*H*)-yl)piperidine-2,6-dione (21.1 g, 50%) as a brown solid. LCMS (Method A): retention time 1.79 min, [M+H]⁺ 371.0.

### Preparation of Int-A4: 3-(5-(4-bromo2-fluorophenyl)-2-oxooxazol-3(2H)-yl)-1-((2-(trimethylsilyl)ethoxy)methyl)piperidine-2,6-dione

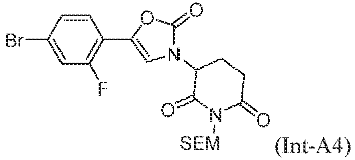

To a stirred solution of 3-(5-(4-bromo-2-fluorophenyl)-2-oxooxazol-3(2*H*)-yl) piperidine-2,6-dione (10 g, 27.1 mmol) in THF (100 mL) at -48 °C under nitrogen atmosphere, were added DBU (6.12 mL, 40.6 mmol) and SEM-CL (5.77 mL, 32.5 mmol). The reaction mixture was stirred at the same temperature for 1h. The reaction was quenched with the addition of water. The reaction mixture was extracted with EtOAc (3 x 60 mL). The combined organic layer was washed with brine, dried over anhydrous Na₂SO₄, filtered, and concentrated *in vacuo.* The resulting residue was purified by flash column chromatography (Biotage, SiO₂, 0-60% EtOAc in pet ether) to afford 3-(5-(4-bromo-2-fluorophenyl)-2-oxooxazol-3(2*H*)-yl)-1-((2-(trimethylsilyl)ethoxy)methyl) piperidine-2,6-dione (6.2 g, 45%) as a light brown solid. LCMS (Method A): retention time 2.04 min, [M-H]⁺ 498.0; ¹H NMR (400 MHz, CDCl₃) δ ppm 7.52 (t, *J* = 8.1 Hz, 1H), 7.37 (d, *J* = 8.5 Hz, 1H), 7.31 (dd, *J* = 10.6, 1.6 Hz, 1H), 6.91 (d, *J* = 2.5 Hz, 1H), 5.29-5.17 (m, 2H), 4.87 (dd, *J* = 12.6, 6.1 Hz, 1H), 3.67-3.58 (m, 2H), 3.10-3.00 (m, 1H), 2.92-2.78 (m, 1H), 2.48-2.30 (m, 2H), 1.02-0.85 (m, 2H), 0.01 (s, 9H).

### Preparation of Int-A: 3-(5-(2-fluoro-4-(4,4,5,5-tetramethyl-1,3,2-dioxaborolan-2-yl) phenyl)-2-oxooxazol-3(2H)-yl)-1-((2-(trimethylsilyl)ethoxy)methyl)piperidine-2,6-dione

To a stirred solution of 3-(5-(4-bromo-2-fluorophenyl)-2-oxooxazol-3(2*H*)-yl)-1-((2-(trimethylsilyl)ethoxy)methyl)piperidine-2,6-dione (7 g, 14 mmol) in 1,4-dioxane (70 mL) at room temperature, were added 4,4,4',4',5,5,5',5'-octamethyl-2,2'-bi(1,3,2-dioxaborolane) (5.34 g, 21 mmol) and potassium acetate (1.65 g, 16.8 mmol). The solution was purged with nitrogen for 10 min and charged with [1,1'-bis (diphenylphosphino)ferrocene]dichloropalladium(II), complex with dichloromethane (1.0 g, 1.4 mmol). The resulting reaction mixture was heated at 80 °C for 1 h, cooled to room temperature, and diluted with EtOAc (70 mL). The suspension was filtered through a celite pad and the filtrate was concentrated *in vacuo.* The resulting residue was purified by flash column chromatography (Biotage, SiO₂, 0-70% EtOAc in pet ether) to afford 3-(5-(2-fluoro-4-(4,4,5,5-tetramethyl-1,3,2-dioxaborolan-2-yl)phenyl)-2-oxooxazol-3(2*H*)-yl)-1-((2-(trimethylsilyl)ethoxy)methyl)piperidine-2,6-dione (7 g, 81%) as a white solid. LCMS (Method A): retention time 2.19 min, [M-H]⁺ 545.2; ¹H NMR (400 MHz, CDCl₃) δ ppm 7.68-7.60 (m, 2H), 7.51 (d, *J* = 11.8 Hz, 1H), 6.96 (d, *J* = 2.5 Hz, 1H), 5.30-5.17 (m, 2H), 4.88 (dd, *J* = 12.6, 6.1 Hz, 1H), 3.67-3.59 (m, 2H), 3.10-2.99 (m, 1H), 2.94-2.78 (m, 1H), 2.48-2.30 (m, 2H), 1.36-1.13 (m, 12H), 1.03-0.85 (m, 2H), 0.07 (s, 9H).

### SYNTHESIS OF OXAZOLONE INTERMEDIATE B

### tert-Butyl 5-amino-4-(5-(2-fluoro-4-(4,4,5,5-tetramethyl-1,3,2-dioxaborolan-2-yl)phenyl)-2-oxooxazol-3(2H)-yl)-5-oxopentanoate

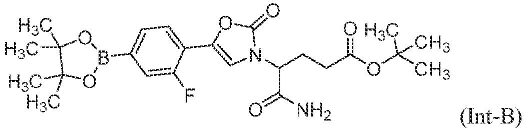

### Preparation of Int-B1: tert-butyl 5-amino-4-((2-(4-bromo-2-fluorophenyl)-2-oxoethyl) amino)-5-oxopentanoate

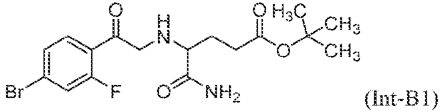

To a stirred solution of 2-bromo-1-(-4-bromo-2-fluorophenyl)ethan-1-one (Int-A1) (2 g, 6.76 mmol) in acetonitrile (15 mL) at room temperature, was added *tert*-butyl 4,5-diamino-5-oxopentanoate, HCl (2.42 g, 11.97 mmol). The reaction mixture was cooled to 0 °C, and sodium iodide (1.22 g, 8.11 mmol) and DIPEA (2.36 mL, 13.52 mmol) were added. The reaction mixture was stirred for 5 h. The reaction was quenched with the addition of 10% sodium bisulphite solution. The reaction mixture was extracted with DCM (3 x 25 mL). The combined organic phase was washed with water, brine solution, dried over anhydrous Na₂SO₄, filtered, and concentrated *in vacuo* to afford *tert*-butyl 5-amino-4-((2-(4-bromo-2-fluorophenyl)-2-oxoethyl)amino)-5-oxopentanoate (2.82 g, crude) as a yellow oil. LCMS (Method A) retention time 1.43 min, [M+H]⁺ 417.2.

### Preparation of Int-B2: tert-butyl 5-amino-4-(5-(4-bromo-2-fluorophenyl)-2-oxooxazol-3(2H)-yl)-5-oxopentanoate

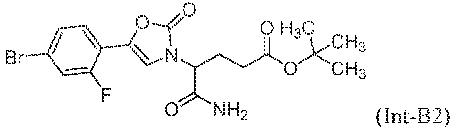

To a stirred solution of *tert*-butyl 5-amino-4-((2-(4-bromo-2-fluorophenyl)-2-oxoethyl)amino)-5-oxopentanoate (2.82 g, 4.53 mmol) in DMF (28 mL) at 0 °C under nitrogen atmosphere, were added CDI (1.84 g, 11.32 mmol) and triethylamine (1.89 mL, 13.58 mmol). The reaction mixture was warmed to room temperature and stirred for 3 h. The reaction mixture was cooled to 0 °C. The reaction was quenched with the addition of ice-cold water. The reaction mixture was filtered. The precipitate was washed with ice cold water, 20% DCM in pet ether, and dried *in vacuo* to afford *tert*-butyl 5-amino-4-(5-(4-bromo-2-fluorophenyl)-2-oxooxazol-3(2*H*)-yl)-5-oxopentanoate (1.57 g, 66%) as a light brown solid. LCMS (Method A) retention time 1.64 min, [M-H]⁺ 440.8,; ¹H NMR (300 MHz, DMSO-*d₆*) δ ppm 7.81-7.68 (m, 2H), 7.58-7.46 (m, 3H), 7.44-7.32 (m, 1H), 4.60-4.48 (m, 1H), 2.33-2.06 (m, 4H), 1.37 (s, 9H).

### Preparation of Int-B: tert-butyl 5-amino-4-(5-(2-fluoro-4-(4,4,5,5-tetramethyl-1,3,2-dioxaborolan-2-yl)phenyl)-2-oxooxazol-3(2H)-yl)-5-oxopentanoate

To a stirred solution of *tert*-butyl 5-amino-4-(5-(4-bromo-2-fluorophenyl)-2-oxooxazol-3(2*H*)-yl)-5-oxopentanoate (1.31 g, 2.75 mmol) in 1,4-dioxane (26 mL) at room temperature, were added bis(pinacolato)diboron (105 g, 412 mmol) and potassium acetate (53.9 g, 550 mmol). The reaction mixture was purged with argon for 10 min, and charged with [1,1'-bis(diphenylphosphino)ferrocene] dichloropalladium(II), DCM complex (0.22 g, 0.27 mmol). The reaction mixture was heated at 90 °C for 3 h, cooled to room temperature, and filtered through a celite pad. The filtrate was concentrated in *vacuo* to get a crude residue, which was purified by flash column chromatography (SiO₂, 0-40% EtOAc in pet ether) to afford *tert*-butyl 5-amino-4-(5-(2-fluoro-4-(4,4,5,5-tetramethyl-1,3,2-dioxaborolan-2-yl)phenyl)-2-oxooxazol-3(2*H*)-yl)-5-oxopentanoate (0.96 g, 68%) as a pale brown solid. LCMS (Method B) retention time 3.23 min, [M+H]⁺ 491.15; ¹H NMR (500 MHz, DMSO-*d₆*) δ ppm 7.79 (br s, 1H), 7.63-7.52 (m, 3H), 7.45 (d, *J* = 11.3 Hz, 1H), 7.40 (br s, 1H), 4.58-4.53 (m, 1H), 2.27-2.11 (m, 4H), 1.36 (s, 9H), 1.31 (s, 12H).

### SYNTHESIS OF INTERMEDIATE C

### cis-3-(Benzyloxy)cyclobutan-1-ol

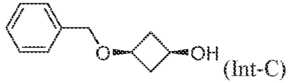

### Preparation of Int-C: cis-3-(benzyloxy)cyclobutan-1-ol

To a stirred solution of 3-(benzyloxy)cyclobutan-1-one (4 g, 22.7 mmol) in methanol (200 mL) at 0 °C, was added sodium borohydride (1.35 g, 35.6 mmol) in small portions. The reaction mixture was warmed to room temperature and stirred for 16 h. The reaction mixture was concentrated *in vacuo* and the resulting residue was diluted with EtOAc. The organic layer was washed with 10% aqueous ammonium chloride solution, water, and brine solution. The organic layer was separated, dried over anhydrous Na₂SO₄, filtered, and concentrated *in vacuo* to yield a crude residue, which was purified by flash column chromatography (SiO₂, 24g column, 0-70% EtOAc in pet ether) to afford a mixture of *cis*/*trans* isomers of 3-(benzyloxy)cyclobutan-1-ol. This isomeric mixture was purified with prep-SFC [(column: Chiral-I Amylose 3 (250 X 50)mm, 5 µm; %CO₂: 90%; %Co solvent: 10% of 0.2% 7 mM methanolic ammonia in methanol; total flow: 250 g/min; back pressure: 100 bar; temp: 40 °C; UV: 220 nm)] to afford *cis*-3-(benzyloxyicyclobutan-1-ol (2.3 g, 57%) as an off-white solid and *trans*-3-(benzyloxy)cyclobutan-1-ol (0.15 g, 4%) as a gummy liquid.

*cis* isomer: LCMS (Method A) retention time 1.02 min, [M+H]⁺ 179.4; ¹H NMR (400 MHz, DMSO-*d₆*) δ ppm 7.37-7.25 (m, 5H), 4.97 (d, *J* = 5.0 Hz, 1H), 4.35-4.24 (m, 3H), 4.18-4.11 (m, 1H), 2.22-2.14 (m, 2H), 2.09-1.95 (m, 2H).

*trans* isomer: LCMS (Method A) retention time 1.06 min, [M+H]⁺ 179.0; ¹H NMR (400 MHz, DMSO-*d₆*) δ ppm 7.37-7.26 (m, 5H), 5.00 (d, *J* = 6.5 Hz, 1H), 4.34 (s, 2H), 3.73-3.64 (m, 1H), 3.58-3.49 (m, 1H), 2.57-2.52 (m, 2H), 1.79-1.69 (m, 2H).

### EXAMPLE 1

### 3-(5-(4-(7-Amino-2,2-dimethyl-2,3-dihydrofuro[2,3-c]pyridin-5-yl)-2-fluorophenyl)-2-oxooxazol-3(2H)-yl)piperidine-2,6-dione

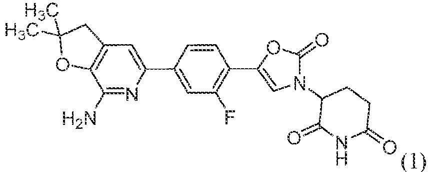

### Preparation 1A: 3-bromo-2,6-dichloro-4-methylpyridine

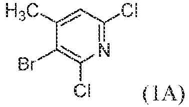

To a stirred solution of 2,6-dichloro-4-methylpyridin-3-amine (2.0 g, 113 mmol) in hydrobromic acid (100 mL, 866 mmol) at 0 °C, a solution of sodium nitrite (21.44 g, 124 mmol) in water (20 mL) was slowly added. The reaction mixture was stirred for an additional 30 min at same temperature. Then, a solution of copper(I) bromide (19.45 g, 136 mmol) in hydrobromic acid (40 mL) was added dropwise and the reaction mixture was warmed to room temperature. After 2 h, the reaction mixture was diluted with water (200 mL) and extracted with EtOAc (3 x 100 mL). The combined organic layer was washed with brine solution, dried over anhydrous Na₂SO₄, filtered, and concentrated in *vacuo* to get a crude residue, which was purified by flash column chromatography (SiO₂, 220 g column, 0-30% EtOAc in pet ether) to afford 3-bromo-2,6-dichloro-4-methylpyridine (22 g, 81%) as an off-white solid. LCMS (Method A) retention time 1.85 min, [M+H]⁺ 241.0; ¹H NMR (400 MHz, CDCl₃) δ ppm 7.29 (s, 1H), 2.48-2.48 (s, 3H).

### Preparation 1B. 1-(3-bromo-2,6-dichloropyridin-4-yl)-2-methylpropan-2-ol

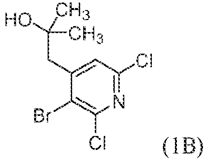

To a stirred solution of diisopropylamine (9.47 mL, 66.4 mmol) in anhydrous THF (150 mL) at -78 ° under nitrogen atmosphere, was added 2.5 M *n*-BuLi in hexanes (23.25 mL, 58.1 mmol) over a period of 20 min. The reaction mixture was stirred at -78 °C for 30 min., warmed to 0 °C, and stirred for additional 30 min. The reaction mixture was again cooled to -78 °C and a solution of 3-bromo-2,6-dichloro-4-methylpyridine (10 g, 41.5 mmol) in anhydrous THF (20 mL) was added. After 1h, acetone (12.19 mL, 166 mmol) in anhydrous THF (10 mL) was added and the reaction mixture was stirred for additional 1h. The reaction was quenched with the addition of 100 mL of ice cold water. The reaction mixture was extracted with EtOAc (2 x 100 mL). The combined organic layer was washed with brine solution, dried over anhydrous Na₂SO₄, filtered, and concentrated *in vacuo* to get a crude residue, which was purified by flash column chromatography (SiO₂, 220g column, 0-70% EtOAc *in* pet ether) to afford 1-(3-bromo-2,6-dichloropyridin-4-yl)-2-methylpropan-2-ol (3 g, 24%) as an off-white solid. LCMS (Method A) retention time 1.54 min, [M+H]⁺ 297.8; ¹H NMR (400 MHz, CDCl₃) δ ppm 7.39 (s, 1H), 3.08 (s, 2H), 1.41 (br s, 1H), 1.29 (s, 6H).

### Preparation 1C: 1-(3-bromo-6-chloro-2-((2,4- dimethoxybenzyl)amino)pyridin-4-yl)-2-methylpropan-2-ol

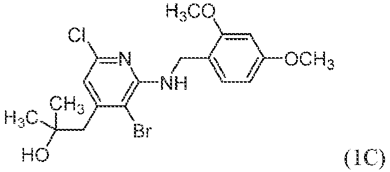

To a stirred solution of 1-(3-bromo-2,6-dichloropyridin-4-yl)-2-methylpropan-2-ol (0.6 g, 2.007 mmol) in NMP (30 mL) at room temperature, were added (2,4-dimethoxyphenyl)methanamine (0.9 mL, 6.02 mmol) and DIPEA (1.402 mL, 8.03 mmol). The resulting reaction mixture was heated at 160 °C for 1.5 h in a microwave reactor. The reaction mixture was cooled to room temperature, diluted with EtOAc, washed with ice cold water and brine solution. The organic layer was dried over anhydrous Na₂SO₄, filtered, and concentrated *in vacuo* to get a crude residue, which was purified by flash column chromatography (SiO₂, 40 g column, 0-70% EtOAc in pet ether) to afford 1-(3-bromo-6-chloro-2-((2,-4-dimethoxybenzyl)amino)pyridin--4-yl)-2-methylpropan-2-ol (0.35 g, 41%) as a pale yellow liquid (LCMS (Method A) retention time 1.89 min, [M+H]⁺ 429.0; ¹H NMR (400 MHz, CDCl₃) δ ppm 7.33-7.30 (m, 1H), 6.59 (s, 1H), 6.51 (d, *J* = 2.5 Hz, 1H), 6.49-6.45 (m, 1H), 5.86-5.76 (m, 1H), 4.58 (d, *J* = 5.5 Hz, 2H), 3.88 (s, 3H), 3.83 (s, 3H), 2.91 (s, 2H), 1.44 (s, 1H), 1.31 (s, 6H)); and 1-(3-bromo-2-chloro-6-((2,4-dimethoxybenzyl)amino)pyridin-4-yl)-2-methylpropan-2-ol (0.15 g, 17%) as a pale yellow liquid (LCMS (Method A) retention time 1.83 min, [M+H]⁺ 429.0; ¹H NMR (400 MHz, CDCl₃) δ ppm 7.17-6.96 (m, 1H), 6.42-6.22 (m, 3H), 4.99 (br t, *J* = 5.7 Hz, 1H), 4.57 (s, 1H), 4.29 (s, 2H), 3.75 (s, 3H), 3.72 (s, 3H), 2.85 (s, 2H), 1.26 (s, 6H)).

### Preparation 1D: 5-chloro-N-(2,4-dimethoxybenzyl)-2,2-dimethyl-2,3-dihydrofuro[2,3-c] pyridin-7-amine

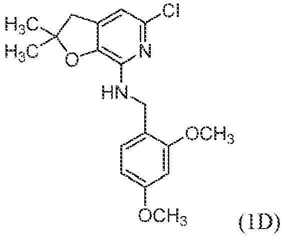

To a stirred solution of 1-(3-bromo-6-chloro-2-((2,4-dimethoxybenzyl)amino) pyridin-4-yl)-2-methylpropan-2-ol (200 mg, 0.465 mmol) in toluene (10 mL) in a pressure tube at room temperature, were added sodium tert-butoxide (90 mg, 0.93 mmol) and 1,2,3,4,5-pentaphenyl-1'-(di-*tert*-butylphosphino)ferrocene (66 mg, 0.093 mmol). The reaction mixture was purged with argon for 15 minutes, and charged with Pd₂(dba)₃ (85 mg, 0.093 mmol) and heated at 90 °C for 2 h. The reaction mixture was cooled to room temperature, filtered through a celite pad and concentrated *in vacuo* to yield a crude residue, which was purified by flash column chromatography (SiO₂, 40 g column, 0-70% EtOAc in pet ether) to afford 5-chloro-*N*-(2,4-dimethoxybenzyl)-2,2-dimethyl-2,3-dihydrofuro[2,3-c]pyridin-7-amine (50 mg, 31(%) as a colourless viscous liquid. LCMS (Method A) retention time 1.96 min, [M+H]⁺ 349.2; ¹H NMR (300 MHz, CDCl₃) δ ppm 7.30 (d, *J* = 7.9 Hz, 1H), 6.47-6.39 (m, 3H), 4.67 (br s, 1H), 4.54 (d, *J* = 5.8 Hz, 2H), 3.83 (s, 3H), 3.80 (s, 3H), 2.90 (s, 2H), 1.44 (s, 6H).

### Preparation 1E: 3-(5-(4-(7-((2,4-dimethoxybenzyl)amino)-2,2-dimethyl-2,3-dihydrofuro[2,3-c]pyridin-5-yl)-2-fluorophenyl)-2-oxooxazol-3(2H)-yl)-1-((2-(trimethylsilyl)ethoxy)methyl)piperidine-2,6-dione

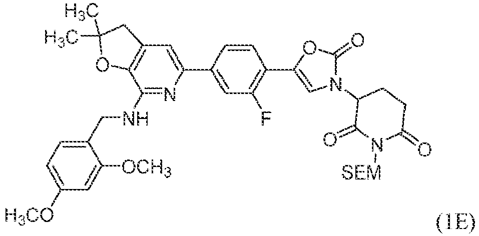

To a stirred solution of 3-(5-(2-fluoro-4-(4,4,5,5-tetramethyl-1,3,2-dioxaborolan-2-yl)phenyl)-2-oxooxazol-3(2*H*)-yl)-1-((2-(trimethylsilyl)ethoxy)methyl)piperidine-2,6-dione (Int-A) (260 mg, 0.48 mmol) in 1,4-dioxane (20 mL)-water (0.5 mL) at room temperature, were added 6-chloro-*N*-(2,4-dimethoxybenzyl)-2-methyl-2*H-*[1,2,3]triazolo[4,5-c]pyridin-4-amine (183 mg, 0.523 mmol) and cesium carbonate (310 mg, 0.952 mmol). The reaction mixture was purged with argon for 10 min and charged with Catacxium Pd G3 (34.6 mg, 0.048 mmol). The reaction mixture was heated at 85 °C for 3 h, cooled to room temperature, diluted with EtOAc, and filtered through a celite pad. The filtrate was concentrated *in vacuo* and the resulting residue was purified by flash column chromatography (SiO₂, 330 g column, 0-80% EtOAc in DCM) to afford 3-(5-(4-(7-((2,4-dimethoxybenzyl)amino)-2,2-dimethyl-2,3-dihydrofuro[2,3-*c*]pyridin-5-yl)-2-fluorophenyl)-2-oxooxazol-3(2*H*)-yl)-1-((2-(trimethylsilyl)ethoxy)methyl)piperidine-2,6-dione (180 mg, 51.6%) as an off-white solid. LCMS (Method A) retention time 2.30 min, [M+H]⁺ 733.4.

### Example 1: 3-(5-(4-(7-amino-2,2-dimethyl-2,3-dihydrofuro[2,3-c]pyridin-5-yl)-2-fluorophenyl)-2-oxooxazol-3(2H)-yl)piperidine-2,6-dione

To a stirred solution of 3-(5-(4-(7-((2,-4-dimethoxybenzyl)amino)-2,2-dimethyl-2,3-dihydrofuro[2,3-c]pyridin-5-yl)-2-fluorophenyl)-2-oxooxazol-3(2*H*)-yl)-1-((2-(trimethylsilyl)ethoxy)methyl)piperidine-2,6-dione (0.1 g, 0.14 mmol) in dichloromethane (5 mL) at 0 °C, was added trifluoroacetic acid (5 mL, 64.9 mmol). The reaction mixture was warmed to room temperature, stirred for 16 h, and then concentrated *in vacuo.* The resulting residue was dissolved in DME (5 mL) and cooled to 0 °C. *N*,*N*'-dimethylethylenediamine (0.07 mL, 0.682 mmol) was added dropwise and the reaction mixture was stirred for 30 minutes at 0 °C. The reaction was quenched with the addition of acetic acid (0.5 mL, 8.73 mmol). The reaction mixture was warmed to room temperature, and stirred for 30 min. The reaction mixture was concentrated *in vacuo* to get a crude residue, which was purified by prep-HPLC (Column: Sun Fire C18 (250* 19*5) mobile phase A: 10 mM ammonium acetate in water (pH: 4.5); mobile phase B: ACN; gradient: A 0-min hold at 10% B, 15-50% B over 10 minutes, then 2-min hold at 50% B; flow rate: 20 mL/min; column temp: 25 °C) to afford 3-(5-(4-(7-amino-2,2-dimethyl-2,3-dihydrofuro[2,3-*c*]pyridin-5-yl)-2-fluorophenyl)-2-oxooxazol-3(2*H*)-yl) piperidine-2,6-dione (40 mg, 65 %) as a white solid. LCMS (Method A) retention time 1.40 min, [M+H]⁺ 453.1; ¹H NMR (400 MHz, DMSO-*d₆*) δ ppm 11.13 (br s, 1H), 7.87 (s, 1H), 7.85 (d, *J* = 5.0 Hz, 1H), 7.62-7.55 (m, 2H), 7.21 (s, 1H), 5.70 (s, 2H), 5.07 (dd, *J* = 13.0, 5.3 Hz, 1H), 3.03 (s, 2H), 2.95-2.82 (m, 1H), 2.65-2.53 (m, 2H), 2.13 (dt, *J =* 10.4, 5.2 Hz, 1H), 1.47 (s, 6H).

### EXAMPLE 2

### trans 3-(5-(4-(6-amino-5-(3-methoxycyclobutoxy)-4-methylpyridin-2-yl)-2-fluorophenyl)-2-oxooxazol-3(2H)-yl)piperidine-2,6-dione

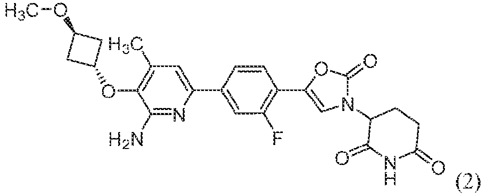

### Preparation 2A: 2,6-dichloro-4-methylpyridin-3-ol

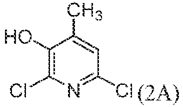

To a stirred solution of 4-methylpyridin-3-ol (12.5 g, 115 mmol) in acetonitrile (250 mL) at 0 °C, was added NCS (33.6 g, 252 mmol) in small portions. The reaction mixture was warmed to room temperature and stirred overnight. The reaction mixture was filtered through a celite pad and concentrated in *vacuo* to get a crude residue, which was purified by flash column chromatography (SiO₂, 220 g column, 0-50% EtOAc in pet ether) to afford 2,6-dichloro-4-methylpyridin-3-ol (10 g, 49%) as a bright yellow solid. LCMS (Method A): retention time 1.07 min, [M+H]⁺ 177.9; ¹H NMR (300 MHz, CDCL₃) δ ppm 7.08 (s, 1H), 5.54 (br s, 1H), 2.31 (s, 3H).

### Preparation 2B: trans 3-(3-(benzyloxy)cyclobutoxy)-2,6-dichloro-4-methylpyridine

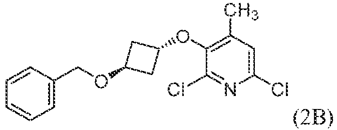

To a stirred solution of 2,6-dichloro-4-methylpyridin-3-ol (1.0 g, 5.62- mmol) in anhydrous THF (2 mL) at room temperature under nitrogen atmosphere, were added *cis-*3-(benzyloxy)cyclobutan-1-ol (Int-C) (1.20 g, 6.74 mmol) and triphenylphosphine (1.77 g, 6.74 mmol). The reaction mixture was cooled 0 °C and DIAD (1.31 mL, 6.74 mmol) was added. The reaction mixture was warmed to room temperature and stirred for 16 h. Next, the reaction mixture was concentrated *in vacuo* to get a crude residue, which was purified by flash column chromatography (SiO₂, 24 g column, 0-40% EtOAc in pet ether) to afford *trans* 3-(3-(benzyloxy)cyclobutoxy)-2,6-dichloro-4-methylpyridine (1.3 g, 68(%) as a colourless liquid. LCMS (Method A) retention time 1.52 min, [M+H]⁺ 338.1; ¹H NMR (300 MHz, CDCl₃) δ ppm 7.39-7.28 (m, 5H), 7.09 (s, 1H), 4.81 (tt, *J* = 7.0, 5.1 Hz, 1H), 4.44 (s, 2H), 4.42-4.32 (m, 1H), 2.60-2.40 (m, 4H), 2.30 (s, 3H).

### Preparation 2C: trans 3-(3-(benzyloxy)cyclobutoxy)-2,6-dichloro--4-methylpyridine 3-((2,6-dichloro-4-methylpyridin-3-yl)oxy)cyclobutan-1-ol

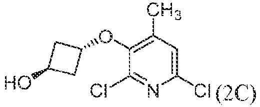

To a stirred solution of *trans* 3-(3-(benzyloxy)cyclobutoxy)-2,6-dichloro-4-methylpyridine (1.3 g, 3.84 mmol) in THF (15 mL) under nitrogen, was added 50% wet Pd/C (10% w/w) (1.08 g, 10.18 mmol). The reaction mixture was flushed with hydrogen and then stirred at room temperature for 6 h under hydrogen atmosphere. The reaction mixture was filtered through a celite pad, and celite pad was washed with THF and MeOH. The combined filtrate was concentrated *in vacuo* and the resulting residue was purified by flash column chromatography (SiO₂, 24 g column, 0-50% EtOAc in pet ether) to afford *trans* 3-((2,6-dichloro-4-methylpyridin-3-yl)oxy)cyclobutan-1-ol (600 mg, 63%) as a colourless gummy mass. LCMS (Method A) retention time 1.26 min, [M+H]⁺ 248.2; ¹H NMR (300 MHz, CDCl₃) δ ppm 7.09 (s, 1H), 4.85 (tt, *J =* 6.9, 5.0 Hz, 1H), 4.72-4.64 (m, 1H), 3.49 (br s, 1H), 2.67-2.54 (m, 2H), 2.39-2.31 (m, 2H), 2.30 (s, 3H).

### Preparation 2D: trans 3-(3-(benzyloxy)cyclobutoxy)-2,6-dichloro-4-methylpyridine 2,6-dichloro-3-(3-methoxycyclobutoxy)-4-methylpyridine

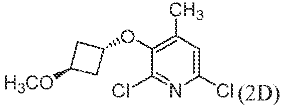

To a stirred solution of *trans* 3-((2,6-dichloro-4-methylpyridin-3-yl)oxy) cyclobutan-1-ol (600 mg, 2.42 mmol) in anhydrous THF (5 mL) at 0 °C under nitrogen atmosphere, was added NaH (60% in mineral oil) (145 mg, 3.63 mmol). The reaction mixture was stirred for 30 min at same temperature and methyl iodide (0.151 mL, 2.42 mmol) was added. The reaction mixture was warmed to room temperature and stirred for 1 h. The reaction was quenched with the addition of ice cold water. The reaction mixture was extracted with EtOAc (3 x 20 mL). The combined organic layer was washed with brine solution, dried over Na₂SO₄, filtered, and concentrated *in vacuo* to get a crude residue, which was purified by flash column chromatography (SiO₂, 40 g column, 30-20% EtOAc in pet ether) to afford *trans* 2,6-dichloro-3-(3-methoxycyclobutoxy)-4-methylpyridine (400 mg, 63%) as an off-white solid. LCMS (Method A) retention time 1.74 min, [M+H]⁺ 262.2; ¹H NMR (300 MHz, CDCl₃) δ ppm 7.09 (s, 1H), 4.77 (tt, *J =* 7.0, 5.3 Hz, 1H), 4.14 (tt, *J =* 6.8, 3.6 Hz, 1H), 3.26 (s, 3H), 2.57-2.48 (m, 2H), 2.45-2.35 (m, 2H), 2.31 (s, 3H).

### Preparation 2E: trans 3-(3-(benzyloxy)cyclobutoxy)-2,6-dichloro-4-methylpyridine 6-chloro-N-(2,4-dimethoxybenzyl)-3-(3-methoxycyclobutoxy)-4-methylpyridin-2-amine

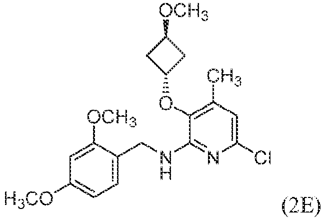

To a stirred solution of *trans* 2,6-dichloro-3-(3-methoxycyclobutoxy)-4-methylpyridine (400 mg, 1.526 mmol) and (2,4-dimethoxyphenyl)methanamine (255 mg, 1.526 mmol) in anhydrous 1,4-dioxane (5 mL) at room temperature, was added cesium carbonate (994 mg, 3.05 mmol). The reaction mixture was purged with argon for 10 min, and charged with palladium (II) acetate (34 mg, 0.153 mmol) and BINAP (14.3 mg, 0.229 mmol). The reaction mixture was heated at 90 °C for 16 h, cooled to room temperature, diluted with EtOAc, and filtered through a celite pad. The filtrate was concentrated in *vacuo* and the resulting residue was purified by flash column chromatography (SiO₂, 24 g column, 0-50% EtOAc in pet ether) to afford *trans* 6-chloro-N-(2,4-dimethoxybenzyl)-3-(3-methoxycyclobutoxy)-4-methylpyridin-2-amine (230 mg, 38%) as an off-white solid. LCMS (Method A) retention time 2.07 min, [M+H]⁺ 393.3; ¹H NMR (300 MHz, CDCl₃) δ ppm 7.26 (d, *J* = 6 Hz, 1H), 6.47-6.41 (m, 2H), 6.33 (s, 1H), 5.23 (br t, *J* = 5.4 Hz, 1H), 4.54-4.45 (m, 3H), 4.06-3.97 (m, 1H), 3.84 (s, 3H), 3.80 (s, 3H), 3.20 (s, 3H), 2.40-2.25 (m, 4H), 2.11 (s, 3H).

### Preparation 2F: trans 3-(3-(benzyloxy)cyclobutoxy)-2,6-dichloro--4-methylpyridine tert-butyl 5-amino-4-(5-(4-(6-((2,4-dimethoxybenzyl)amino)-5-(3-methoxycyclobutoxy)-4-methylpyridin-2-yl)-2-fluorophenyl)-2-oxooxazol-3(2H)-yl)-5-oxopentanoate

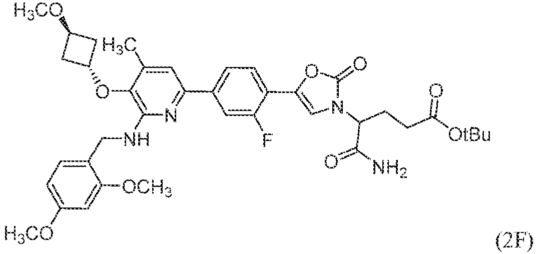

To a stirred solution of *trans* 6-chloro-N-(2,4-dimethoxybenzyl)-3-(3-methoxycyclobutoxy)-4-methylpyridin-2-amine (230 mg, 0.585 mmol) in 1,4-dioxane (8 mL)-water (2.0 mL) at room temperature, were added *tert*-butyl 5-amino-4-(5-(2-fluoro-4-(4,4,5,5-tetramethyl-1,3,2-dioxaborolan-2-yl)phenyl)-2-oxooxazol-3(2*H*)-yl)-5-oxopentanoate (Int-B) (316 mg, 0.644 mmol) and potassium phosphate, tribasic (311 mg, 1.46 mmol). The reaction mixture was purged with argon for 10 min, and charged with XPhos Pd G2 (46 mg, 0.059 mmol). Then, the reaction mixture was heated at 90 °C for 3 h. The reaction mixture was cooled to room temperature, diluted with EtOAc, dried over anhydrous Na₂SO₄ and filtered through a celite pad. The filtrate was concentrated in *vacuo* to get a crude residue, which was purified by flash chromatography (SiO₂, 12 g column, 0-4% of MeOH in DCM) to afford *trans tert*-butyl 5-amino-4-(5-(4-(6-((2,4-dimethoxybenzyl)amino)-5-(3-methoxycyclobutoxy)-4-methylpyridin-2-yl)-2-fluorophenyl)-2-oxooxazol-3(2H)-yl)-5-oxopentanoate (200 mg, 47%) as a light brown solid. LCMS (Method B) retention time 2.04 min, [M+H]⁺ 721.4; ¹H NMR (300 MHz, DMSO-*d₆*) δ ppm 7.95-7.83 (m, 2H), 7.77 (s, 1H), 7.57 (t, *J =* 8.3 Hz, 1H), 7.48 (d, *J* = 2.9 Hz, 1H), 7.40 (s, 1H), 7.19 (d, *J =* 8.3 Hz, 1H), 7.09 (s, 1H), 6.57 (d, *J =* 2.2 Hz, 1H), 6.44. (dd, J= 8.4, 2.3 Hz, 1H), 6.31 (s, 1H), 4.64 (s, 1H), 4.59-4.51 (m, 3H), 4.11 (br dd,*J* = 4.3, 2.9 Hz, 1H), 3.92 (s, 2H), 3.87 (s, 3H), 3.72 (s, 3H), 3.15 (s, 3H), 2.47-2.36 (m, 2H), 2.30-2.20 (m, 7H), 1.38 (s, 9H).

### Example 2: trans 3-(5-(4-(6-amino-5-(3-methoxycyclobutoxy)-4-methylpyridin-2-yl)-2-fluorophenyl)-2-oxooxazol-3(2H)-yl)piperidine-2,6-dione

To a stirred solution of *trans tert*-butyl 5-amino-4-(5-(4-(6-((2,4-dimethoxybenzyl)amino)-5-(3-methoxycyclobutoxy)--4-methylpyridin-2-yl)-2-fluorophenyl)-2-oxooxazol-3(2H)-yl)-5-oxopentanoate (200 mg, 0.277 mmol) in acetonitrile (5 mL) at room temperature, was added methanesulfonic acid (0.054 mL, 0.832 mmol) and heated at 90 °C for 3 h. The reaction mixture was cooled to room temperature, and concentrated *in vacuo* to yield a crude residue, which was purified by reverse phase prep-HPLC (column: YMC EXRS C18 ( 250mm*20mm) 5 µm; mobile phase A: 10 mM ammonium acetate in water pH-4.5; mobile phase B: ACN; flow rate: 20.0 mL/min) to afford *trans* 3-(5-(4-(6-amino-5-(3-methoxycyclobutoxy)-4-methylpyridin-2-yl)-2-fluorophenyl)-2-oxooxazol-3(2H)-yl)piperidine-2,6-dione (50 mg, 36%) as an off-white solid. LCMS (Method B) retention time 1.89 min, [M+H]⁺ 497.15; ¹H NMR (400 MHz, DMSO-*d₆*) δ ppm 11.12 (br s, 1H), 7.91 (s, 1H), 7.89-7.87 (m, 1H), 7.63-7.58 (m, 2H), 7.11 (s, 1H), 5.81 (br s, 2H), 5.07 (dd, *J =* 13.0, 5.3 Hz, 1H), 4.68-4.61 (m, 1H), 4.14-4.08 (m, 1H), 3.15 (s, 3H), 2.95-2.83 (m, 1H), 2.65-2.53 (m, 2H), 2.46-2.37 (m, 2H), 2.26-2.22 (m, 2H), 2.21 (s, 3H), 2.17-2.10 (m, 1H).

### EXAMPLE 3

### 3-(5-(4-(6-Amino--4-methyl-5-(((tetrahydro-2H-pyran-4-yl)methyl)amino)pyridin-2-yl)-2.-fluorophenyl)-2-oxooxazol-3(2H)-yl)piperidine-2,6-dione

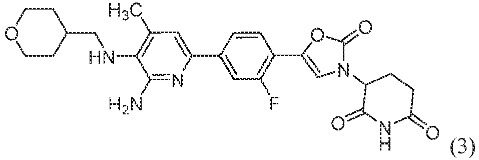

### Preparation 3A: 2,6-dichloro-4-methyl-N-((tetrahydro-2H-pyran-4-yl)methyl)pyridin-3-amine

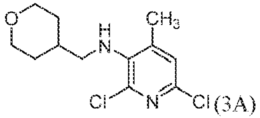

To a stirred solution of 2,6-dichloro-4-methylpyridin-3-amine (1 g, 5.65 mmol) and tetrahydro-2*H*-pyran-4-carbaldehyde (0.96 g, 8.47 mmol) in EtOAc (10 mL) at room temperature, was added TFA (0.87 mL, 11.3 mmol). The reaction mixture was stirred for 30 min. Then, sodium triacetoxyborohydride (2.4 g, 11.3 mmol) was added and the reaction mixture was stirred overnight. The reaction was quenched with the addition of saturate aqueous NaHCO₃ solution. The reaction mixture was extracted with EtOAc (2 x 100 mL). The combined organic phase was washed with water and brine, dried over anhydrous Na₂SO₄, filtered, and concentrated *in vacuo* to get a crude residue, which was purified by flash column chromatography (SiO₂, 40 g column, 0-20% EtOAc in pet ether) to afford 6-dichloro-4-methyl-*N*-((tetrahydro-2*H*-pyran-4-yl)methyl)pyridin-3-amine (0.75 g, 48%)) as a yellow oil. LCMS (Method A): retention time 1.65 min, [M+H]⁺ 275.2; ¹H NMR (400 MHz, CDCl₃) δ ppm 7.04 (s, 1H), 4.02 (br dd, *J =* 11.5, 4.0 Hz, 2H), 3.76 (br s, 1H), 3.46-3.36 (m, 2H), 2.99 (d, *J* = 6.0 Hz, 2H), 2.35 (s, 3H), 1.75-1.71 (m, 2H), 1.64-1.57 (m, 1H), 1.48-1.32 (m, 2H).

### Preparation 3B: 6-chloro-N²-(2,4-dimethoxybenzyl)-4-methyl-N³-((tetrahydro-2H-pyran-4-yl)methyl)pyridine-2,3-diamine

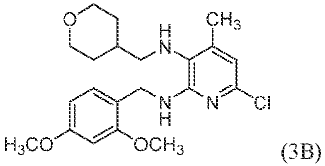

To a stirred solution of 2,6-dichloro-4-methyl-*N*-((tetrahydro-2*H*-pyran-4-yl) methyl)pyridin-3-amine (0.67 g, 2.44 mmol) and 2,4-dimethoxybenzylamine (0.36 mL, 2.44 mmol) in anhydrous 1,4-dioxane (5 mL) at room temperature, was added cesium carbonate (1.6 g, 4.87 mmol). The reaction mixture was purged with argon for 10 min, and charged with BINAP (227 mg, 0.365 mmol) and palladium (II) acetate (55 mg, 0.24 mmol). The reaction mixture was heated at 90 °C for 3 h, cooled to room temperature, diluted with water, and extracted with EtOAc (2 x 50 mL). The combined organic phase was washed with brine, dried over anhydrous Na₂SO₄, filtered, and concentrated *in vacuo* to yield a crude residue, which was purified by flash column chromatography (SiO₂, 24 g column, 0-30% EtOAc in pet ether) to afford 6-chloro-*N*²-(2,4-dimethoxybenzyl)-4-methyl-*N*³-((tetrahydro-2*H*-pyran-4-yl)methyl)pyridine-2,3-diamine (0.42 g, 43%) as a yellow syrup. LCMS (Method A) retention time 2.03 min, [M-H]⁺ 404.1; ¹H NMR (400 MHz, CDCl₃) δ ppm 7.28 (d, *J* = 7.1 Hz, 1H), 6.48 (s, 1H), 6.45 (br d, *J =* 8.5 Hz, 1H), 6.36 (s, 1H), 5.65 (br s, 1H), 4.54 (br d, *J =* 5.5 Hz, 2H), 3.98 (br dd, *J* = 11.0, 3.5 Hz, 2H), 3.85 (s, 3H), 3.81 (s, 3H), 3.39 (br t, *J =* 11.8 Hz, 2H), 2.64 (br d, *J* = 6.0 Hz, 2H), 2.44 (br s, 1H), 2.14 (s, 3H), 1.70 (br d, *J =* 11.0 Hz, 3H), 1.41-1.29 (m, 2H).

### Preparation 3C: tert-butyl 5-amino-4-(5-(4-(6-((2,4-dimethoxybenzyl)amino)-4-methyl-5-(((tetrahydro-2H-pyran-4-yl)methyl)amino)pyridin-2-yl)-2-fluorophenyl)-2-oxooxazol-3(2H)-yl)-5-oxopentanoate

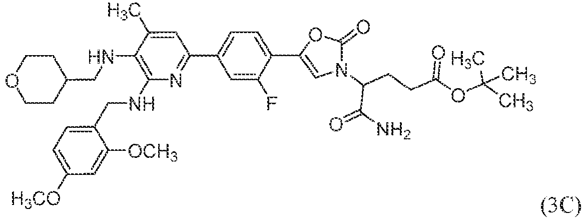

To a stirred solution of 6-chloro-*N*²-(2,4-dimethoxybenzyl)-4-methyl-*N*³-((tetrahydro-2*H*-pyran-4-yl)methyl)pyridine-2,3-diamine (300 mg, 0.739 mmol), 1,4-dioxane (2.9 mL)-water (0.74 mL) at room temperature, were added *tert*-butyl 5-amino-4-(5-(2-fluoro-4-(4,4,5,5-tetramethyl-1,3-dioxolan-2-yl)phenyl)-2-oxooxazol-3(2*H*)-yl)-5-oxopentanoate (Int-B) (364 mg, 0.739 mmol) and potassium phosphate tribasic (392 mg, 1.848 mmol). The reaction mixture was purged with argon for 10 min, and charged with XPhos Pd G2 (58 mg, 0.074 mmol). The reaction mixture was heated at 95 °C for 6 h. The reaction mixture was cooled to room temperature, diluted with EtOAc, dried over anhydrous Na₂SO₄, and filtered through a celite pad. The filtrate was concentrated *in vacuo* to yield a crude residue, which was purified by flash column chromatography (SiO₂, 2.4 g column, 0-5% MeOH in DCM) to afford *tert*-butyl 5-amino-4-(5-(4-(6-((2,4-dimethoxybenzyl)amino)-4-methyl-5-(((tetrahydro-2*H*-pyran-4-yl)methyl)amino)pyridin-2-yl)-2-fluorophenyl)-2-oxooxazol-3(2*H*)-yl)-5-oxopentanoate (0.45 g, 83%). LCMS (Method A) retention time 1.92 min, [M+H]⁺ 734.5.

### Example 3: 3-(5-(4-(6-amino-4-methyl-5-(((tetrahydro-2H-pyran-4-yl)methyl)amino) pyridin-2-yl)-2-fluorophenyl)-2-oxooxazol-3(2H)-yl)piperidine-2,6-dione

To a stirred solution of *tert*-butyl 5-amino-4-(3-(4-(6-((2,4-dimethoxybenzyl) amino)-4-methyl-5-(((tetrahydro-2*H*-pyran-4-yl)methyl)amino)pyridin-2-yl)-2-fluorophenyl)-2-oxooxazol-3(2*H*)-yl)-5-oxopentanoate (300 mg, 0.409 mmol) in acetonitrile (2 mL) at room temperature, was added methanesulfonic acid (66 µL, 1.02 mmol). The reaction mixture was heated at 90 °C for 2 h, cooled to room temperature and concentrated *in vacuo* to afford a crude residue, which was purified by reverse phase prep-HPLC (Column: YMC Triate c18 EXRS1 (250*20*5); mobile phase A: 10 mM ammonium acetate in water; mobile phase B: ACN; Time/%B: 00/20, 15/60, 17/95) to afford 3-(5-(-4-(6-amino-4-methyl-5-(((tetrahydro-2*H*-pyran-4-yl)methyl)amino)pyridin-2-yl)-2-fluorophenyl)-2-oxooxazol-3(2*H*)-yl)piperidine-2,6-dione (19 mg, 9%) as an off-white solid. LCMS (Method B) retention time 1.45 min, [M+H]⁺ 510.20; ¹H NMR (400 MHz, DMSO-*d₆*) δ ppm 11.12 (br s, 1H), 7.89 (s, 1H), 7.86 (d, *J =* 4.4 Hz, 1H), 7.60-7.55 (m, 2H), 7.10 (s, 1H), 5.63 (s, 2H), 5.06 (dd, J= 12.9, 5.3 Hz, IH), 3.92-3.82 (m, 3H), 3.28-3.23 (m, 2H), 2.94-2.83 (m, 1H), 2.76 (br t, *J* = 6.6 Hz, 2H), 2.65-2.54 (m, 2H), 2.23 (s, 3H), 2.17-2.08 (m, 1H), 1.70 (br d, *J =* 10.8 Hz, 3H), 1.26-1.15 (m, 2H).

### EXAMPLE 4

### 3-(5-(4-(6-Amino-5-((4-methoxypiperidin-1-yl)methyl)pyridin-2-yl)-2-fluorophenyl)-2-oxooxazol-3(2H)-yl)piperidine-2,6-dione

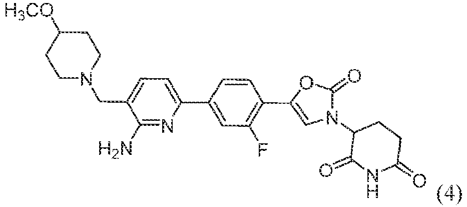

### Preparation 4A: tert-butyl (6-chloro-3-fonnylpyridin-2-yl) carbamate

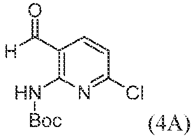

To a stirred solution of *tert*-butyl (6-chloropyridin-2-yl)carbamate (20 g, 87 mmol) and TEMED (33 mL, 219 mmol) in anhydrous THF (300 mL) at -78 °C under nitrogen atmosphere, was added 1.6 M *n*-BuLi in hexane (137 mL, 219 mmol,) over a period of 30 min. The reaction mixture was gradually warmed to -10 °C and stirred for 2 h at the same temperature. The reaction mixture was again cooled to -78 °C, anhydrous DMF (34 mL, 437 mmol) was added and gradually warm to room temperature. The reaction mixture was stirred for additional 2 h, quenched with 1 N hydrochloric acid (0.5 L) and diluted with EtOAc (1 L), and stirred for 15 min. The organic phase was washed with water and saturated NaHCO₃ solution, dried over anhydrous Na₂SO₄, filtered, and concentrated *in vacuo* to get a crude residue, which was triturated with 10% *i*-propanol in pet ether, filtered, and dried *in vacuo* to afford *tert*-butyl (6-chloro-3-formylpyridin-2-yl) carbamate (15 g, 67%) as an off-white solid. LCMS (Method A): retention time 1.45 min, [M-*t*Bu]⁺ 201.1; ¹H NMR (300 MHz, CDCl₃) δ ppm 10.17 (br s, 1H), 9.90 (s, 1H), 7.94 (br d, *J* = 8.3 Hz, 1H), 7.26-7.02 (m, 1H), 1.55 (s, 9H).

### Preparation 4B: tert-butyl 5-amino-4-(5-(4-(6-((tert-butoxycarbonyl)amino)-5-formylpyridin-2-yl)-2-fluorophenyl)-2-oxooxazol-3(2H)-yl)-5-oxopentanoate

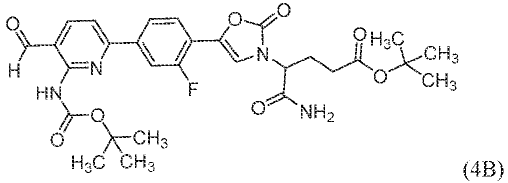

To a stirred solution of 3-(5-(2-fluoro-4-(4,4,5,5-tetramethyl-1,3,2-dioxaborolan-2-yl)phenyl)-2-oxooxazol-3(2*H*)-yl)-1-((2-(trimethylsilyl)ethoxy)methyl)piperidine-2,6-dione (Int-B) (4.78 g, 9.74. mmol) in 1.4-dtoxane (60 mL)-water (4.5 mL) at room temperature, were added 6-chloro-*N*-(2,4-dimethoxybenzyl)-2-methyl-2*H-*[1,2,3]triazolo[4,5-*c*]pyridin-4-amine (2.5 g, 9.74 mmol) and cesium carbonate (4.76 g, 14.6 mmol). The reaction mixture was purged with argon for 10 min and charged with Catacxium Pd G3 (0.36 g, 0.49 mmol). The reaction mixture was heated at 85 °C for 3 h, cooled to room temperature, diluted with EtOAc, and filtered through a celite pad. The filtrate was concentrated *in vacuo* to get a crude residue, which was purified by flash column chromatography (SiO₂, 80 g column, 0-80% EtOAc in pet ether) to afford *tert-*butyl 5-amino-4-(5-(4-(6-((*tert*-butoxycarbonyl)amino)-5-formylpyridin-2-yl)-2-fluorophenyl)-2-oxooxazol-3(2*H*)-yl)-5-oxopentanoate (5.0 g, 88%) as a pale brown solid. LCMS (Method A) retention time 1.81 min, [M-H]⁺ 583.5.

### Preparation 4C: tert-butyl 5-amino-4-(5-(4-(6-((tert-butoxycarbonyl)amino)-5-((4-methoxypiperidin-1-yl)methyl)pyridin-2-yl)-2-fluorophenyl)-2-oxooxazol-3(2H)-yl)-5-oxopentanoate

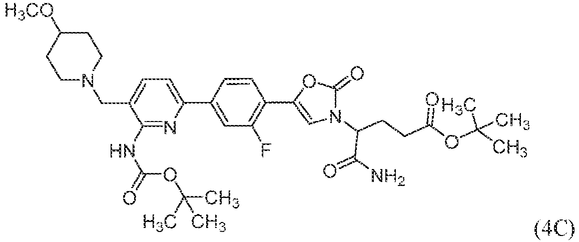

To a stirred solution of 4-methoxypiperidine (47.3 mg, 0.41 mmol) and tert-butyl 5-amino-4-(5-(4-(6-((*tert*-butoxycarbonyl)amino)-5-formylpyridin-2-yl)-2-fluorophenyl)-2-oxooxazol-3(2*H*)-yl)-5-oxopentanoate (120 mg, 0.20 mmol) in DCE (5 mL)-DMF (1 mL) at room temperature, was added triethylamine (0.024 mL, 0.41 mmol). The reaction mixture was stirred overnight. Then, MP-Cyanoborohydride (200 mg) was added and the reaction mixture was again stirred overnight. The reaction mixture was filtered through a celite pad and concentrated *in vacuo* to get a crude residue, which was purified by flash column chromatography (SiO₂, 12 g column, 0-10% MeOH in DCM) to afford *tert*-butyl 5-amino-4-(5-(4-(6-((*tert*-butoxycarbonyl)amino)-5-((4-methoxypiperidin-1-yl)methyl) pyridin-2-yl)-2-fluorophenyl)-2-oxooxazol-3(2*H*)-yl)-5-oxopentanoate (120 mg, 58%) as a yellow solid. LCMS (Method A): retention time 2.01 min, [M+H]⁺ 684.5.

### Example 4: 3-(5-(4-(6-amino-5-((4-methoxypiperidin-1-yl)methyl)pyridin-2-yl)-2-fluorophenyl)-2-oxooxazol-3(2H)-yl)piperidine-2,6-dione

To a stirred solution of *tert*-butyl 5-amino-4-(5-(4-(6-((*tert*-butoxycarbonyl) amino)-5-((4-methoxypiperidin-1-yl)methyl)pyridin-2-yl)-2-fluorophenyl)-2-oxooxazol-3(2*H*)-yl)-5-oxopentanoate (120 mg, 0.18 mmol) in acetonitrile (5 mL) at room temperature, was added benzenesulfonic acid (0.04 mL,0.36 mmol). The reaction mixture was heated at 90 °C for 16 h, cooled to room temperature and concentrated in *vacuo* to get a crude residue, which was purified by reverse phase prep-HPLC (column: Waters XBridge C18, 150 mm x 19 mm, 5-µm particles; mobile phase A: 5:95 acetonitrile: water with 10 mM ammonium acetate; mobile phase B: 95:5 acetonitrile: water with 10 mM ammonium acetate; gradient: 0-min hold at 10% B, 10-48% B over 10 min, then 5-min hold at 48% B; flow rate: 20 mL/min; column temperature: 25 °C) to afford 3-(5-(4-(6-amino-5-((4-methoxypiperidin-1-yl)methyl)pyridin-2-yl)-2-fluorophenyl)-2-oxooxazol-3(2*H*)-yl)piperidine-2,6-dione (50 mg, 55%) as a white solid. LCMS (Method A): retention time 1.62 min, [M+H]⁺ 510.2; ¹H NMR (400 MHz, DMSO-*d₆*) δ ppm 11.13 (s, 1H), 7.98-7.93 (m, 2H), 7.66-7.61 (m, 2H), 7.39 (br d, *J =* 7.5 Hz, 1H), 7.20 (d, *J =* 7.5 Hz, 1H), 6.22 (br s, 2H), 5.08 (dd, *J =* 12.9, 5.4 Hz, 1H), 3.45-3.38 (m, 2H), 3.23 (s, 3H), 3.22-3.16 (m, 1H), 2.94-2.84 (m, 1H), 2.69-2.56 (m, 4H), 2.19-2.07 (m, 3H), 1.83 (br s, 2H), 1.45 (br d, *J =* 9.0 Hz, 2H).

### EXAMPLE 5

### 3-(5-(4-(6-Amino-4-methyl-5-(((tetrahydro-2H-pyran-4-yl)oxy)methyl)pyridin-2-yl)-2-fluorophenyl)-2-oxooxazol-3(2H)-yl)piperidine-2,6-dione

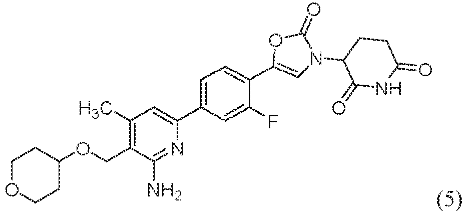

### Preparation 5A: (2,6-dichloro-4-methylpyridin-3-yl)methanol

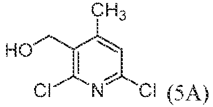

To a stirred solution of 2,6-dichloro-4-methylnicotinic acid (1.0 g, 4.85 mmol) in anhydrous THF (30 ml) at 0 °C under nitrogen, was slowly added 2 M solution of lithium aluminum hydride in THF (6.07 ml, 12.13 mmol). The reaction mixture was warmed to room temperature and stirred for 2 h. The reaction was quenched at 0 °C with the slow addition of water (2.3 mL) and then with 15% NaOH solution (2.3 mL). The reaction mixture was stirred for 5 min and then water (6.9 mL) was added followed by THF (50 mL). The reaction mixture was vigorously stirred for 10 min, dried over anhydrous Na₂SO₄ and filtered through a celite pad. The filtrate was concentrated *in vacuo* and the crude residue obtained was purified by flash column chromatography (SiO₂, 0-80% EtOAc in pet ether) to afford (2,6-dichloro-4-methylpyridin-3-yl)methanol (440 mg, 47%) as a pale amorphous solid. LCMS (Method A): retention time 1.03 min, [M+H]⁺ 192.1; ¹H NMR (300 MHz, CDCl₃) δ ppm 7.15 (s, 1H), 4.83 (s, 2H), 2.49 (s, 3H), 1.95 (br s, 1H).

### Preparation 5B: 2,6-dichloro-3-(chloromethyl)-4-methylpyridine

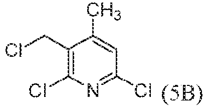

To a stirred solution of (2,6-dichloro-4-methylpyridin-3-yl)methanol (500 mg, 2.6 mmol) in anhydrous DCM (26 ml) at room temperature, was added triethylamine (0.54 ml, 3.91 mmol) followed by DMAP (32 mg, 0.26 mmol). Methanesulfonyl chloride (243 µL, 3.12 mmol) was added at 0 °C under nitrogen. The reaction mixture was warmed to room temperature and stirred for 16 h. Next, the reaction mixture was concentrated *in vacuo* and the crude residue obtained was purified by flash column chromatography (SiO₂, 0-50% EtOAc in pet ether) to afford 2,6-dichloro-3-(chloromethyl)-4-methylpyridine (345 mg, 63%) as a light-yellow oil. LCMS (Method A): retention time 1.70 min, [M+H]⁺ 210.1; ¹H NMR (300 MHz, CDCl₃) δ ppm 7.16 (s, 1H), 4.72 (s, 2H), 2.49 (s, 3H).

### Preparation 5C: 2,6-dichloro-4-methyl-3-(((tetrahydro-2H-pyran-4-yl)oxy)methyl) pyridine

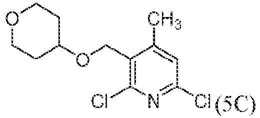

To a stirred solution of tetrahydro-2*H*-pyran-4-ol (580 mg, 5.7 mmol) in anhydrous THF (30 mL) at 0 °C, was added 60% sodium hydride in mineral oil (285 mg, 7.13 mmol). The reaction mixture was stirred for 30 min at 0 °C. Next, 2,6-dichloro-3-(chloromethyl)-4-methylpyridine (1 g, 4.75 mmol) was added and the reaction mixture was stirred at 0 °C for 1 h. The reaction was quenched with the addition of ice-cold water at 0 °C. The reaction mixture was extracted with EtOAc. The combined organic phase was washed with water and brine, dried over anhydrous Na₂SO₄, filtered, and concentrated *in vacuo*. The crude residue obtained was purified by flash column chromatography (SiO₂, 0-80% EtOAc in pet ether) to afford 2,6-dichloro-4-methyl-3-(((tetrahydro-2*H*-pyran-4-yl)oxy)methyl)pyridine (1.1 g, 84%) as a colorless oil. LCMS (Method A): retention time 1.48 min, [M+H]⁺ 276.0; ¹H NMR (300 MHz, CDCl₃) δ ppm 7.14 (s, 1H), 4.68 (s, 2H), 3.96 (dt, *J* = 11.7, 4.6 Hz, 2H), 3.62 (dt, *J =* 8.5, 4.5 Hz, 1H), 3.53-3.41 (m, 2H), 2.46 (s, 3H), 1.96 (br dd, *J* = 13.3, 3.9 Hz, 2H), 1.70-1.61 (m, 2H).

### Preparation 5D: 6-chloro-N-(2,4-dimethoxybenzyl)-4-methyl-3-(((tetrahydro-2H-pyran-4-yl)oxy)methyl)pyridin-2-amine

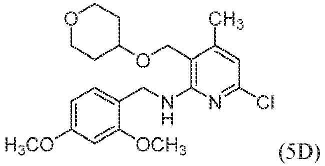

To a stirred solution of 2,6-dichloro-4-methyl-3-(((tetrahydro-2*H*-pyran-4-yl)oxy) methyl)pyridine (1 g, 3.62 mmol) and (2,4-dimethoxyphenyl)methanamine (0.7 g, 4.16 mmol) in anhydrous 1,4-dioxane (12 ml) at room temperature, was added cesium carbonate (2.95 g, 9.05 mmol). The reaction mixture was purged with argon for 10 min at room temperature. Next, palladium (II) acetate (81 mg, 0.36 mmol) and BINAP (338 mg, 0.54 mmol) were added. The reaction mixture was heated at 90 °C for 1.5 h and then cooled to room temperature. The reaction mixture was diluted with EtOAc and filtered through a celite pad. The filtrate was concentrated *in vacuo* and the crude residue obtained was purified by reverse phase flash column chromatography (Column C18 750 g, mobile phase A: ammonium acetate in water, pH 7; mobile phase B: acetonitrile; gradient: time (min)/%B - 0/0%, 40/40%, 90/40%, 95/100%) to afford 6-chloro-*N*-(2,4-dimethoxybenzyl)-4-methyl-3-(((tetrahydro-2*H*-pyran-4-yl)oxy)methyl)pyridin-2-amine (450 mg, 31%) as a pale gummy solid. LCMS (Method A): retention time 1.98 min, [M-H]⁺ 405.4; ¹H NMR (300 MHz, CDCl₃) δ ppm 7.29 (d, *J =* 7.9 Hz, 1H), 6.47-6.38 (m, 3H), 5.88 (br t, *J* = 5.4 Hz, 1H), 4.54 (d, *J =* 5.7 Hz, 2H), 4.47 (s, 2H), 3.87 (dt, *J* = 11.8, 4.5 Hz, 2H), 3.82 (s, 3H), 3.79 (s, 3H), 3.49-3.34 (m, 3H), 2.19 (s, 3H), 1.87-1.74 (m, 2H), 1.54-1.46 (m, 2H).

### Preparation 5E: tert-butyl 5-amino-4-(5-(4-(6-((2,4-dimethoxybenzyl)amino)-4-methyl-5-(((tetrahydro-2H-pyran-4-yl)oxy)methyl)pyridin-2-yl)-2-fluorophenyl)-2-oxooxazol-3(2H)-yl)-5-oxopentanoate

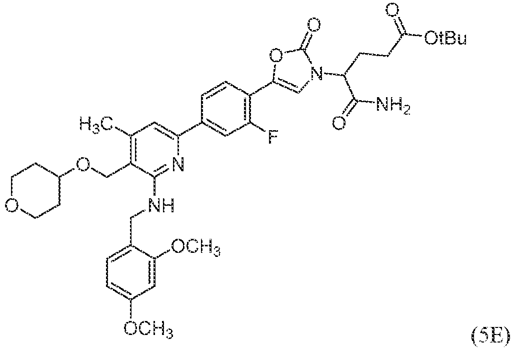

To stirred solution of 6-chloro-*N*-(2,4-dimethoxybenzyl)-4-methyl-3-(((tetrahydro-2*H*-pyran-4-yl)oxy)methyl)pyridin-2-amine (150 mg, 0.37 mmol) and *tert-*butyl 5-amino-4-(5-(2-fluoro-4-(4,4,5,5-tetramethyl-1,3,2-dioxaborolan-2-yl)phenyl)-2-oxooxazol-3(2H)-yl)-5-oxopentanoate (Int-B) (181 mg, 0.37 mmol) in 1,4-dioxane (2 mL)-water (0.5 mL) at room temperature, was added cesium carbonate (300 mg, 0.92 mmol). The reaction mixture was purged with argon for 10 min. Next, Xphos Pd G2 (29 mg, 0.04 mmol) was added and the reaction mixture was heated at 90 °C for 2.5 h. The reaction mixture was cooled to room temperature, diluted with EtOAc, dried over anhydrous Na₂SO₄ and filtered through a celite pad. The filtrate was concentrated *in vacuo* and the crude residue obtained was purified by flash column chromatography (SiO₂, 0-100% EtOAc (contains 15% EtOH) in DCM) to afford tert-butyl 5-amino-4-(5-(4-(6-((2,4-dimethoxybenzyl)amino)-4-methyl-5-(((tetrahydro-2*H*-pyran-4-yl)oxy) methyl)pyridin-2-yl)-2-fluorophenyl)-2-oxooxazol-3(2*H*)-yl)-5-oxopentanoate (250 mg, 92%). LCMS (Method A): retention time 2.02 min, [M-H]⁺ 735.5; ¹H NMR (300 MHz, CDCl₃) δ ppm 7.92-7.81 (m, 2H), 7.68-7.61 (m, 1H), 7.31 (d, *J =* 8.3 Hz, 1H), 7.22 (d, *J* = 2.9 Hz, 1H), 6.87 (s, 1H), 6.48 (d, *J* = 2.5 Hz, 1H), 6.42 (dd, *J* = 8.3, 2.5 Hz, 1H), 6.35 (br s, 1H), 5.89 (t*, J* = 5.4 Hz, 1H), 5.51 (br s, 1H), 4.78-4.72 (m, 1H), 4.70 (d, *J* = 5.4 Hz, 2H), 4.58 (s, 2H), 3.88 (s, 2H), 3.86 (s, 3H), 3.79 (s, 3H), 3.51 (tt, *J* = 8.7, 4.1 Hz, 1H), 3.40 (ddd, *J* = 11.8, 9.5, 2.7 Hz, 2H), 2.37 (br d, *J* = 3.6 Hz, 3H), 2.30 (s, 3H), 2.23-2.12 (m, 1H), 1.91-1.80 (m, 2H), 1.57 (br d, *J* = 9.3 Hz, 2H), 1.46 (s, 9H).

### Example 5: 3-(5-(4-(6-amino-4-methyl-5-(((tetrahydro-2H-pyran-4-yl)oxy)methyl) pyridin-2-yl)-2-fluorophenyl)-2-oxooxazol-3(2H)-yl)piperidine-2,6-dione

To a stirred solution of *tert*-butyl 5-amino-4-(5-(4-(6-((2,4-dimethoxybenzyl) amino)-4-methyl-5-(((tetrahydro-2*H*-pyran-4-yl)oxy)methyl)pyridin-2-yl)-2-fluorophenyl)-2-oxooxazol-3(2*H*)-yl)-5-oxopentanoate (230 mg, 0.31 mmol) in acetonitrile (2 mL) at room temperature, was added methanesulfonic acid (102 µL, 1.56 mmol). The reaction mixture was heated at 90 °C for 2 h. Next, the reaction mixture was cooled to room temperature, concentrated *in vacuo* to yield a crude residue, which was purified by reverse phase prep-HPLC (XSelect CSH C18 (250 mm x 19 mm x 5 µm); mobile phase A: 10 mM ammonium acetate in water (pH 4.5); mobile phase B: acetonitrile; gradient: T/%B - 0/20%, 3/30%, 15/55%, 13/95%, flow: 20 mL/min) to afford 3-(5-(4-(6-amino-4-methyl-5-(((tetrahydro-2*H*-pyran-4-yl)oxy)methyl)pyridin-2-yl)-2-fluorophenyl)-2-oxooxazol-3(2*H*)-yl)piperidine-2,6-dione (37 mg, 27%) as a white amorphous solid. LCMS (Method B) retention time 1.66 min, [M+H]⁺511.15; ¹H NMR (400 MHz, DMSO-*d*₆) δ ppm 11.01 (s, 1H), 7.97 (s, 1H), 7.96-7.93 (m, 1H), 7.66-7.60 (m, 2H), 7.14 (s, 1H), 5.83 (s, 2H), 5.07 (dd, *J =* 12.9, 5.1 Hz, 1H), 4.50 (s, 2H), 3.84-3.78 (m, 2H), 3.59 (dt, *J* = 8.8, 4.5 Hz, 1H), 3.35 (br s, 2H), 2.88 (br d, *J* = 4.5 Hz, 1H), 2.69-2.54 (m, 2H), 2.30 (s, 3H), 2.17-2.09 (m, 1H), 1.94-1.86 (m, 2H), 1.50-1.41 (m, 2H).

### COMPARATIVE COMPOUND A

### 3-(2-oxo-5-phenyloxazol-3(2H)-yl)piperidine-2,6-dione

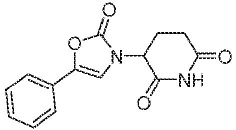

3-(2-oxo-5-phenyloxazol-3(2*H*)-yl)piperidine-2,6-dione is disclosed as compound number I-33 in WO 2019/060693 A1.

### Preparation 1A: tert-butyl 5-amino-5-oxo-4-((2-oxo-2-phenylethyl)amino)pentanoate

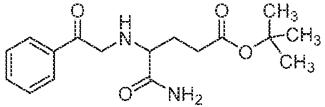

To a stirred suspension of 2-bromo-1-phenylethan-1-one (200 mg, 1.0 mmol) and *tert*-butyl 4,5-diamino-5-oxopentanoate, HCl (360 mg, 1.5 mmol) in anhydrous acetonitrile (4.5 mL) was added sodium iodide (181 mg, 1.21 mmol) at 0 °C under argon. The reaction mixture was stirred for 5 min at the same temperature. DIPEA (351 µL, 2.01 mmol) was added drop wise to the reaction mixture. The reaction mixture was continued to stir at 0 °C for 2 h. The reaction mixture was warmed to room temperature and stirred overnight. The reaction was quenched with the addition of 10% sodium bisulfite solution. The mixture was extracted with DCM (3 x 10 mL). The combined organic phase was washed with water and brine, dried over anhydrous Na₂SO₄, filtered and concentrated under *vacuo* to afford *tert*-butyl 5-amino-5-oxo-4-((2-oxo-2-phenylethyl)amino)pentanoate (322 mg, crude). LCMS (Method A): retention time 1.215 min, [M+H]⁺ 321.1.

### Preparation 1B: tert-butyl 5-amino-5-oxo-4-(2-oxo-5-phenyloxazol-3(2H)-yl)pentanoate

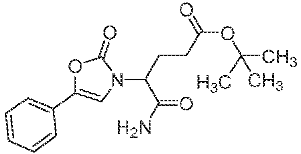

To a stirred solution of *tert*-butyl 5-amino-5-oxo-4-((2-oxo-2-phenylethyl)amino) pentanoate (322 mg, 1.0 mmol) in anhydrous DMF (7 mL) were added CDI (407 mg, 2.5 mmol) and triethylamine (420 µL, 3.0 mmol) at 0 °C under argon. The reaction mixture was slowly warmed to room temperature and stirred overnight. The reaction was quenched by the addition of ice-cold water. The reaction mixture was extracted with EtOAc (3 x 15 mL). The combined organic phase was washed with water and brine solution, dried over anhydrous Na₂SO₄, filtered and concentrated under *vacuo.* The residue was purified by flash chromatography (SiO₂, 24 g column, 0-100% EtOAc/pet ether) to afford *tert*-butyl 5-amino-5-oxo-4-(2-oxo-5-phenyloxazol-3(2*H*)-yl)pentanoate (155 mg, 44%). LCMS (Method A): retention time 1.33 min, [M+Na]⁺ 369.2; ¹H NMR (300 MHz, DMSO-*d*₆) δ ppm 7.75 (s, 2H), 7.5-7.6 (m, 2H), 7.42 (t, 2H, *J =* 7.5 Hz), 7.3- 7.4 (m, 2H), 4.50 (dd, 1H, *J* = 4.3, 10.1 Hz), 2.2-2.3 (m, 3H), 2.0-2.1 (m, 1H), 1.38 (s, 9H).

### Comparative Compound A:

To a stirred solution of *tert*-butyl 5-amino-5-oxo-4-(2-oxo-5-phenyloxazol-3(2*H*)- yl)pentanoate (150 mg, 0.43 mmol) in acetonitrile (3.0 mL) was added methanesulfonic acid (42 µL, 0.65 mmol) at room temperature. The reaction mixture was heated at 90 °C for 1 h, cooled to room temperature, concentrated under *vacuo* and the residue was purified by reverse phase prep-HPLC (X-Bridge Phenyl C18 (250 mm*19 mm) 5 µm; mobile phase A: 10 mM ammonium acetate in water, mobile phase B: ACN; flow rate: 20.0 mL/min; gradient Time/%B: 0/30, 15/43, 15.1/100) to afford 3-(2-oxo-5-phenyloxazol-3(2*H*)-yl)piperidine-2,6-dione (25 mg, 21%) as a white solid. LCMS (Method A): retention time 1.582 min, [M+H]⁺ 273.20; ¹H NMR (400 MHz, DMSO-*d*₆) δ ppm 11.02 (br s, 1H), 7.70 (s, 1H), 7.52-7.47 (m, 2H), 7.47-7.42 (m, 2H), 7.35-7.30 (m, 1H), 5.03 (dd, *J* = 5.3, 13.3 Hz, 1H), 2.95-2.84 (m, 1H), 2.68-2.60 (m, 1H), 2.48-2.35 (m, 1H), 2.21-2.11 (m, 1H).

### COMPARATIVE COMPOUND B

### 3-(5-(4-bromo-2-fluorophenyl)-2-oxooxazol-3(2H)-yl)piperidine-2,6-dione

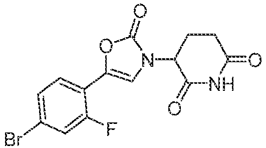

### Preparation 1A: 3-(5-(4-bromo-2-fluorophenyl)-2-oxooxazol-3(2H)-yl)-1-

### (hydroxymethyl)piperidine-2,6-dione

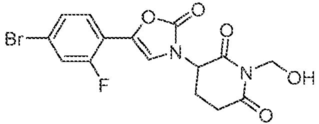

To a stirred solution of 3-(5-(4-bromo-2-fluorophenyl)-2-oxooxazol-3(2*H*)-yl-1-((2-(trimethylsilyl)ethoxy)methyl)piperidine-2,6-dione (200 mg, 0.4 mmol) in anhydrous DCM (2.0 mL) was added TFA (0.154 mL, 2.0 mmol) at room temperature. The reaction mixture was stirred at room temperature for 2 h, concentrated under *vacuo* and the residue was co-evaporated with DME (4 x) to afford 3-(5-(4-bromo-2-fluorophenyl)-2-oxooxazol-3(2*H*)-yl)-1-(hydroxymethyl)piperidine-2,6-dione (150 mg, 94%). LCMS (Method A): retention time 1.37 min, [M+Na]⁺ 423.1.

### Comparative Compound B:

To a stirred solution of 3-(5-(4-bromo-2-fluorophenyl)-2-oxooxazol-3(2*H*)-yl)-1-(hydroxymethyl)piperidine-2,6-dione (180 mg, 0.45 mmol) in anhydrous DME (2255 µL) was added *N,N'*-dimethylethane-1,2-diamine (199 mg, 2.26 mmol) at 0 °C under nitrogen. The reaction mixture was warmed to room temperature and stirred for 30 min, cooled to 0 °C, acidified with acetic acid (258 µL, 4.5 mmol) and then concentrated under *vacuo* (bath temp < 30 °C). The residue was purified with reverse phase prep-HPLC (Method: column: X-Bridge Phenyl (19 mm x 250 mm*5 µm); mobile phase A: 10 mM ammonium acetate in water, mobile phase B: ACN; flow rate: 20 mL/min; gradient condition (Time/%B): 0/30, 14/51, 14.1/100) to afford 3-(5-(4-bromo-2-fluorophenyl)-2-oxooxazol-3(2*H*)-yl)piperidine-2,6-dione (67 mg, 40%). LCMS (Method A): retention time 2.155 min, [M-H]⁺ 366.8; ¹H NMR (400 MHz, DMSO-*d*₆) δ ppm 11.13 (br s, 1H), 7.75-7.70 (m, 1H), 7.66 (d, *J* = 3.0 Hz, 1H), 7.53 (s, 1H), 7.52 (d, *J =* 5.1 Hz, 1H), 5.06 (dd, *J* = 5.3, 13.3 Hz, 1H), 2.94-2.80 (m, 1H), 2.70-2.54 (m, 2H), 2.16-2.08 (m, 1H).

### BIOLOGICAL ASSAYS

The pharmacological properties of the compounds of this invention may be confirmed by a number of biological assays. The exemplified biological assays, which follow, have been carried out with compounds of the invention.

### JURKAT CELLULAR DEGRADATION ASSAY

Jurkat cells were plated at 80,000 cells/well in 40 µL RPMI + 10% FBS in a 384-well cell culture plate prior to using acoustic dispensing technology for adding a compound of interest. Cell cultures were incubated for 24 h at 37 °C and 5% CO₂. In order to facilitate analysis, cell cultures were spun down at 200 rpm for 5 min and the supernatant was discarded. After shaking the plate to dislodge the cell pellet, cells were resuspended in 50 µL of Fixation Buffer (eBioscience FoxP3 buffer set 00-5523-00) for 60 min at room temperature. After centrifuging and discarding the supernatant, cells were permeabilized with 50 µL of Permeabilization buffer (eBioscience FoxP3 buffer set 00-5523-00) for 10 min at room temperature. Following permeabilization, cells were spun down and the supernatant was replaced with 20 µL fluorescently labelled antibodies against Helios, Ikaros and Aiolos or corresponding Isotype controls in 1× Permeabilization buffer (Ikaros-Alexa488 [Biolegend, Cat #368408, 1:50], Helios-PE [CST, Cat #29360, 1:50]. Aiolos-Alexa647 [Biolegend, Cat #371106 Biolegend, 1:25]) and staining reactions were incubated for 1 h at room temperature while protected from light. Subsequently, 30 µL of 1× Permeabilization buffer was added prior to centrifuging the cells and discarding the supernatant. Stained cells were resuspended in 25 µL of flow cytometry staining buffer [PBS + 0.2% bovine serum albumin (BSA)] and analyzed using an Intellicyt Ique Plus flow cytometer.

**TABLE A-1**

| Jurkat Cellular Degradation Assay: Maximum Observed Degradation | | | |
|---|---|---|---|
| Ex. No. | IKZF1 | IKZF2 | IKZF3 |
| | Ikaros max % deg. observed | Helios max % deg. observed | Aiolos max % deg. observed |
| 1 | 63% | 89% | 55% |
| 3 | 64% | 92% | 48% |
| 3 | 57% | 92% | 54% |
| 4 | 69% | 91% | 61% |
| 5 | 51% | 92% | 38% |
| Comparative Compound A | 70% | 27% | 52% |
| Comparative Compound B | 24% | 11% | 25% |

Table A-1 lists the maximum observed degradation of the IKZF1 protein, IKZF2 protein, and IKZF3 protein as measured in the Jurkat Cellular Degradation assay. The results in Table A-1 were rounded to two digits. In the Jurkat Cellular Degradation assay, a value of 100% indicated no detectable protein remaining or complete degradation of the protein; and a value of 0% indicated no detectable degradation of the protein by the test compound. In the study reported in Table A-1, the compounds of the present invention, as exemplified by Examples 1 to 5, were observed to degrade at least 89% of the IKZF2 (Helios) protein. In contrast, the Comparative Compound A and Comparative Compound B were observed to degrade less than 30% of the IKZF2 protein.

**TABLE A-2**

| Jurkat Cellular Degradation Assay: DC₅₀* | | | |
|---|---|---|---|
| Ex. No. | IKZF1 | IKZF2 | IKZF3 |
| | Ikaros DC₅₀ (nM) | Helios DC₅₀ (nM) | Aiolos DC₅₀ (nM) |
| 1 | 1505 | 22 | 600 |
| 2 | 4380 | 37 | 2320 |
| 3 | 5600 | 36 | 9490 |
| 4 | 1200 | 34 | 3005 |
| 5 | 7900 | 50 | >10,000 |
| Comparative Compound A | 1,520 | >10,000 | >10,000 |
| Comparative Compound B | >10,000 | >10,000 | >10,000 |

| | | | |
|---|---|---|---|
| *DC₅₀ is defined as the concentration of compound required to reduce levels of a given protein by 50% compared to treatment with DMSO alone. | | | |

### HUMAN REGULATORY T CELL DEGRADATION ASSAY

Cryopreserved human regulatory T cells were thawed in RPMI + 10%FBS + 20 ng/mL IL-2. After being spun at 1200 rpm for 5 mins, the cells were resuspended in RPMI + 10% FBS+ 20 ng/mL and rested at 37 °C with 5% CO₂ for 3 hours. The cells were then plated at 40,000 cells/well in 40 µL RPMI + 10% FBS + 20 ng/mL human IL-2 in a 384 well cell culture plate prior to using acoustic dispensing technology (ECHO 555) for adding compounds of interest. Cell cultures were incubated for 20 hours at 37 °C and 5% CO₂. In order to facilitate analysis, cell cultures were spun down at 1200 rpm for 5 minutes and the supernatant was discarded using an EL406 plate washer. After washing three times with 70 µL PBS, cell pellets were resuspended in 50 µL of near IR viability staining solution (Life Technologies, Cat# L34975) and incubated for 30 minutes on ice protected from light. Cells were washed three times with 70 µL PBS + 0.5% BSA using an EL406 plate washer. After shaking the plate to dislodge the cell pellet, cells were resuspended in 50 µL of Fixation Buffer (eBioScience FoxP3 buffer set 00-5523-00) for 60 minutes at room temperature. After centrifuging and discarding the supernatant, cells were permeabilized with 50 µL of permeabilization buffer (eBioScience FoxP3 buffer set 00-5523-00) for 10 minutes at room temperature. Following permeabilization, cells were spun down and the supernatant was replaced with 30 µL fluorescently labelled antibodies against the intracellular targets Helios (Helios-APC [BioLegend, Cat# 137222, 1:50]), Aiolos, and Ikaros lx Permeabilization buffer and staining reactions were incubated for 1 hour at room temperature; protected from light. Subsequently, 30 µL of lx Permeabilization buffer was added prior to centrifuging the cells and discarding the supernatant. Stained cells were resuspended in 30 µL of flow cytometry staining buffer (PBS + 0.5%BSA) and analyzed using an Intellicyt Ique Plus flow cytometer.

**TABLE B-1**

| Human Regulatory T Cell Degradation Assay: Maximum Observed Degradation | | | | |
|---|---|---|---|---|
| Ex. No. | IKZF1 | IKZF2 | IKZF3 | IKZF4 |
| | Ikaros max % deg. observed | Helios max % deg. observed | Aiolos max % deg. observed | Eos max % deg. observed |
| 1 | 51% | 98% | 22% | 56% |
| 2 | 56% | 98% | 25% | 62% |
| 3 | 58% | 98% | 25% | 59% |
| 4 | 67% | 98% | 35% | 62% |
| 5 | 52% | 98% | 29% | 67% |

Table B-1 lists the maximum observed degradation of the IKZF1-4 proteins as measured in the Human Regulatory T Cell Degradation assay. The results in Table B-1 were rounded to two digits. In the Human Regulatory T Cell Degradation assay, a value of 100% indicated no detectable protein remaining or complete degradation of the protein; and a value of 0% indicated no detectable degradation of the protein by the test compound. In the study reported in Table B-1, the compounds of the present invention, as exemplified by Examples 1 to 5, were observed to degrade (i) 51%, 56%, 58%, 67%, and 52% of the IKZF1 (Ikaros) protein, respectively; (ii) 98% of IKZF2 (Helios) protein; (iii) 22%, 25%, 25%, 35%, and 29% of the IKZF3 (Aiolos) protein; and (iv) 56%, 62%, 59%, 62%, and 67% of the IKZF4 (Eos) protein.

**TABLE B-2**

| Human Regulatory T Cell Degradation Assay: DC₅₀^{*} | | | | |
|---|---|---|---|---|
| Ex. No. | IKZF1 | IKZF2 | IKZF3 | IKZF4 |
| | Ikaros DC₅₀ (nM) | Helios DC₅₀ (nM) | Aiolos DC₅₀ (nM) | Eos DC₅₀ (nM) |
| 1 | 8227 | 52 | >10,000 | 55 |
| 2 | 950 | 71 | >100060 | 193 |
| 3 | 6466 | 51 | >10,000 | 141 |
| 4 | 3788 | 59 | >10,000 | 203 |
| 5 | 6500 | 46 | >10,000 | 575 |

| | | | | |
|---|---|---|---|---|
| *DC₅₀ is defined as the concentration of compound required to reduce levels of a given protein by 50% compared to treatment with DMSO alone. | | | | |

### HUMAN REGULATORY T CELL REPROGRAMMING ASSAY

Human CD4⁺ T cells were isolated from fresh healthy leukopaks (Stemcell Technologies) using the RosetteSep Human CD4⁺ T cell enrichment cocktail (Stemcell Technologies) and Ficoll density gradient centrifugation. Leukopaks were diluted with an equal volume of phosphate-buffered saline (PBS [Gibco]) supplemented with 2% fetal bovine serum (FBS, VWR Lifescience) and incubated with RosetteSep Human CD4⁺ T cell enrichment cocktail for 20 minutes before layering on Ficoll-Paque Plus solution (GE Health Care). The cell-rich interface layer was harvested and washed twice with PBS supplemented with 2% FBS. Regulatory T cells were then isolated manually using the EasySep Human CD4⁺CD127^{low}CD25⁺ Regulatory T cell isolation kit (Stemcell Technologies) according to the manufacturer's instructions. Cells were rested overnight in Roswell Park Memorial Institute (RPMI) 1640 media (Gibco) supplemented with 10% FBS, Pen/Strep (Gibco), MEM-NEAA (Gibco), and sodium pyruvate (Gibco) in a humidified incubator (37 °C, 5% CO₂). Cells were then stained for CD4 (clone: RPA-T4, Biolegend), CD25 (clone: 2A3, BD Biosciences), and CD127 (clone: hIL-7R-M21, BD Biosciences). CD4⁺CD127^{low}CD25⁺ cells were sorted on a BD FACS Aria Fusion sorter to a purity of 95%) or greater. Sorted cells were immediately used or cryopreserved for downstream assays.

Fresh or cryopreserved FACS-sorted CD4⁺CD127^{low}CD25⁺ Treg cells were cultured at 25,000-50,000 cells/well of 96-well round bottom plates in RPMI 1640 media (Gibco) supplemented with 10% FBS, Pen/Strep (Gibco), MEM-NEAA (Gibco), and sodium pyruvate (Gibco). Cells were stimulated with Treg Xpander beads (Thermo Fisher) at cells-to-beads ratio of 1:4 in the presence of 500 U/mL recombinant human IL-2 (Proleukin). Compounds were added at titrated doses and cells were incubated at 37 °C, 5% CO₂ for 12-13 days. Recombinant human IL-2 and compound were replenished every 2-3 days during the entire culture duration. On day 12 or 13, cells were restimulated with phorbol 12-myristate 13-acetate (PMA,) and ionomycin in the presence of the protein transport inhibitors brefeldin A and monensin (eBioscience Cell Stimulation Cocktail plus protein transport inhibitors, 500x, Catalog number 00-4975-93) before proceeding with flow cytometry staining and analysis.

For flow cytometry staining, cells were washed twice with flow cytometry staining buffer (Thermo Fisher) and incubated in Human Tru-stain Fc block (Biolegend) for 10 minutes before adding eFluor780 viability dye (Thermo Fisher) and surface marker antibody cocktail for 30 minutes at 4 °C. Cells were then fixed and permeabilized by incubation with FoxP3 transcription factor staining buffer (Thermo Fisher) for 30 minutes at 4 °C as per the kit manufacturer's instructions. Cells were washed twice with the Perm/Wash buffer supplied in the kit as per the manufacturer's instructions and incubated overnight at 4 °C with an intracellular antibody cocktail comprised of antibodies specific for the transcription factors shown in Table C. Cells were washed twice with Perm/Wash buffer and resuspended in flow cytometry staining buffer (Thermo Fisher) prior to acquisition. Sample acquisition and analysis was performed using a BD LSRFortessa (BD Biosciences) flow cytometer. Single stain controls for each fluorochrome were prepared using UltraComp eBead Compensation Beads (Thermo Fisher). Data were analyzed using FlowJo version 10 and GraphPad Prism Software.

**TABLE C**

| Antibodies Used for Flow Sorting and Analysis | |
|---|---|
| Marker | Fluorochrome |
| Viability | eFluor780 |
| CD3 | BUV395 |
| CD4 | BUV805 |
| CD8 | FITC |
| CD25 | BV606 |
| FoxP3 | BV421 |
| HELIOS | PE-Cy7 |
| EOS | PE |

**TABLE C- 1**

| Human Regulatory T Cell Reprogramming Assay-Maximum Observed Degradation | | |
|---|---|---|
| Ex. No. | IKZF2 Helios max % deg. observed | IKZF4 Eos max % deg. Observed |
| 1 | 72% | 84% |
| 2 | 74% | 72% |
| 3 | 73% | 84% |
| 4 | 66% | 80% |
| 5 | - | 46% |
| Comparative Compound A | 1% | 0% |
| Comparative Compound B | 6% | 11% |

Table C-1 lists the maximum observed degradation of the IKZF2 protein and IKZF4 protein as measured in the Human Regulatory T Cell Reprogramming assay. The results in Table C-1 were rounded to two digits. In the Human Regulatory T Cell Reprogramming assay, a value of 100% indicated no detectable protein remaining or complete degradation of the protein; and a value of 0% indicated no detectable degradation of the protein by the test compound.

**TABLE C-2**

| Human Regulatory T Cell Reprogramming Assay: DC₅₀^{*} | | |
|---|---|---|
| Ex. No. | IKZF2 Helios DC₅₀ (nM) | IKZF4 Eos DC₅₀ (nM) |
| 1 | 45 | 2 |
| 2 | 205 | 3 |
| 3 | 247 | 4 |
| 4 | 155 | 4 |
| 5 | - | 7 |
| Comparative Compound A | >1000 | >1000 |
| Comparative Compound B | >1000 | >1000 |

| | | |
|---|---|---|
| *DC₅₀ is defined as the concentration of compound required to reduce levels of a given protein by 50% compared to treatment with DMSO alone. | | |

### HUMAN CD8⁺ T CELL DEGRADATION ASSAY

Cryopreserved healthy donor human peripheral blood mononuclear cells (PBMC) from healthy donors were thawed and seeded at 500,000 cells/well of 96-well round bottom plates in RPMI 1640 media (Gibco) supplemented with 10% FBS, Pen/Strep (Gibco), MEM-NEAA (Gibco), and sodium pyruvate (Gibco). Cells were treated with titrated doses of compound for 24 hours at 37 °C, 5% CO₂, before analyzing by flow cytometry.

For flow cytometry staining, cells were washed twice with flow cytometry staining buffer (Thermo Fisher) and incubated in Human Tru-stain Fc block (Biolegend) for 10 minutes before adding eFluor780 viability dye (Thermo Fisher) and surface marker antibody cocktail containing LD-eFluor780, CD3-BUV-395, CD4-BUV805, CD8-FITC, and CD25-BV605 for 30 minutes at 4 °C, Cells were then fixed and permeabilized by incubation with permeabilization buffer (eBioscience FoxP3 buffer set 00-5523-00) for 30 minutes at 4 °C as per the kit manufacturer's instructions. Cells were washed twice with the Perm/Wash buffer supplied in the kit as per the manufacturer's instructions and incubated overnight at 4 °C with an intracellular antibody cocktail comprised of antibodies specific for the transcription factors (i.e. , Foxp3-BV421, HELIOS-PE-Cy7, EOS-PE, IKAROS-PECF594, AIOLOS-AF647). Cells were washed twice with Perm/Wash buffer and resuspended in flow cytometry staining buffer (Thermo Fisher) prior to acquisition. Sample acquisition and analysis was performed using a BD LSRFortessa (BD Biosciences) flow cytometer. Single-stain controls for each fluorochrome were prepared using UltraComp eBead Compensation Beads (Thermo Fisher). Data were analyzed using FlowJo version 10 and GraphPad Prism Software.

**TABLE D-1**

| Human CD8⁺ T Cell Reprogramming Assay-Maximum Observed Degradation | | |
|---|---|---|
| Ex. No. | IKZF1 Ikaros | IKZF3 Aiolos |
| 1 | 76% | 76% |
| 2 | 52% | 58% |
| 3 | 67% | 67% |
| 4 | 80% | 77% |
| 5 | 56% | 72% |
| Comparative Compound A | 74% | 80% |
| Comparative Compound B | 17% | 0% |

**TABLE D-2**

| Human CD8⁺ T Cell Reprogramming Assay: DC₅₀^{*} | | |
|---|---|---|
| Ex. No. | IKZF1 Ikaros DC₅₀ (nM) | IKZF3 Aiolos DC₅₀ (nM) |
| 1 | 53 | 18 |
| 2 | 132 | 86 |
| 3 | 181 | 193 |
| 4 | 15 | 18 |
| 5 | 366 | 131 |
| Comparative Compound A | 113 | 21 |
| Comparative Compound B | >1000 | >1000 |

| | | |
|---|---|---|
| *DC₅₀ is defined as the concentration of compound required to reduce levels of a given protein by 50% compared to treatment with DMSO alone. | | |

Table D-1 lists the maximum observed degradation of the IKZF1 protein and IKZF3 protein as measured in the Human CD8⁺ T Cell Reprogramming assay. The results in Table D-1 were rounded to two digits. In the Human CD8⁺ T Cell Reprogramming assay, a value of 100% indicated no detectable protein remaining or complete degradation of the protein; and a value of 0% indicated no detectable degradation of the protein by the test compound.

**TABLE E**

| Maximum Observed Degradation of IKZF1-4: Comparison of Examples 1-5 and Comparative Compounds A and B | | | | |
|---|---|---|---|---|
| Ex. No. | IKZF1^{*} Ikaros max % deg. observed | IKZF2^{**} Helios max % deg. observed | IKZF3^{*} Aiolos max % deg. observed | IKZF4^{***} Eos max % or deg. observed |
| 1 | 76% | 89% | 76% | 84% |
| 2 | 53% | 92% | 58% | 72% |
| 3 | 67% | 92% | 67% | 84% |
| 4 | 80% | 91% | 77% | 80% |
| 5 | 56% | 92% | 72% | 46% |
| Comparative Compound A | 74% | 27% | 80% | 0% |
| Comparative Compound B | 17% | 11% | 0% | 11% |

| | | | | |
|---|---|---|---|---|
| * IKZF1 and IKZF3: Human CD8⁺ T Cell Reprogramming Assay (D-1) ** IKZF2: Jurkat Cellular Degradation Assay (A-1) *** IKZF4: Human Regulatory T Cell Reprogramming Assay {(C-1) | | | | |

Examples 1-5 have been compared to Comparative Compound A disclosed in WO 2019/060693 A1 and Comparative Compound B, and have been found to be especially advantageous. Examples 1-5 have the surprising advantage of decreasing the levels of the four IKZF1-4 proteins Ikaros, Helios, Aiolos, and Eos. As shown in Tables A-1, C-1, and D-1 in the reported tests: (i) Examples 1-5 decreased the level of IKZF1 (Ikaros) by at least 53% (Table D-1); (ii) Examples 1-4 decreased the level of the IKZF2 (Helios) protein by at least 89% (Table A-1); (iii) Examples 1-4 decreased the level of IKZF3 (Aiolos) by at least 58% (Table D-1); and (iv) Examples 1-4 decreased the level of IKZF4 (Eos) by at least 46% (Table C-1). In contrast, in similar tests, the Comparative Compound A and Comparative Compound B were observed to degrade the IKZF2 (Helios) protein (Table A-1) by 27% or less, and degrade IKZF4 (Eos) protein (Table C-1) by 11% or less.

The present invention fills the foregoing need by providing compounds that are useful to decrease the levels of the four IKZF1-4 proteins Ikaros, Helios, Aiolos, and Eos.

## Claims

1. A compound of Formula (I): 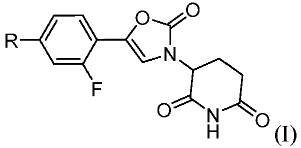 or stereoisomers, tautomers, or salts thereof, wherein:
R is:

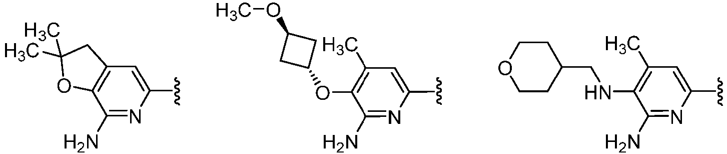

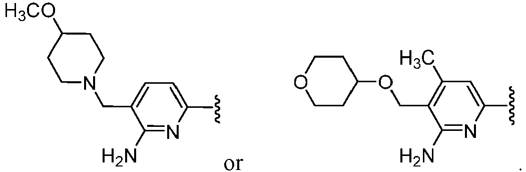

2. The compound according to claim 1, or stereoisomers, tautomers, or salts thereof, wherein said compound is:

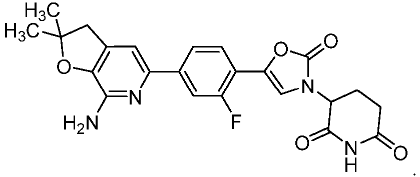

3. The compound according to claim 1, or stereoisomers, tautomers, or salts thereof, wherein said compound is:

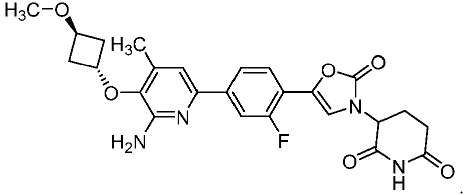

4. The compound according to claim 1, or stereoisomers, tautomers, or salts thereof, wherein said compound is:

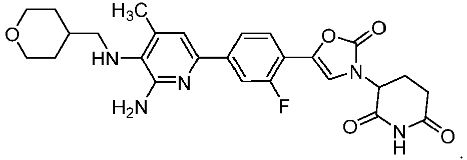

5. The compound according to claim 1, or stereoisomers, tautomers, or salts thereof, wherein said compound is:

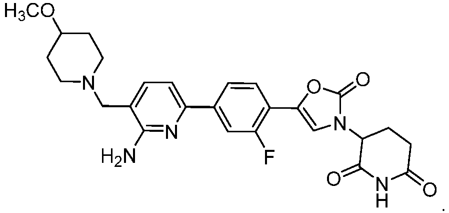

6. The compound according to claim 1, or stereoisomers, tautomers, or salts thereof, wherein said compound is:

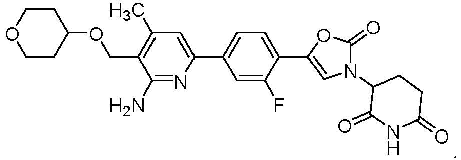

7. A pharmaceutical composition comprising a compound according to any one of claims 1 to 6, or stereoisomers, tautomers, or pharmaceutically acceptable salts thereof; and a pharmaceutically acceptable carrier.

8. A compound, or stereoisomers, tautomers, or pharmaceutically acceptable salts thereof, according to any one of claims 1 to 6 for use in the treatment of cancer.

9. The compound, or stereoisomers, tautomers, or pharmaceutically acceptable salts thereof, for use according to claim 8, wherein said cancer is selected from cancer of the colon, gastric, pancreatic cancer, breast cancer, prostate cancer, lung cancer, ovarian cancer, cervical cancer, renal cancer, cancer of the head and neck, lymphoma, leukemia, and melanoma.

10. The compound, or stereoisomers, tautomers, or pharmaceutically acceptable salts thereof, for use according to claim 9 in combination with a second agent, wherein said second agent is selected from an antagonist of PD1/PD-L1 axis, an antagonist of CTLA4, a chemotherapeutic agent, radiation, or an anti-tumor vaccine.

11. A compound, or a stereoisomer, a tautomer, or a pharmaceutically acceptable salt thereof according to any one of claims 1 to 6 for use in a method of treating cancer, wherein the method comprises decreasing Ikaros, Helios, Aiolos, and Eos protein levels in cells by contacting said cells with said compound, or a stereoisomer, a tautomer, or a pharmaceutically acceptable salt thereof, wherein
a) said Ikaros protein level is decreased by at least 30 %;
b) said Helios protein level is decreased by at least 50 %;
c) said Aiolos protein level is decreased by at least 30 %; and
d) said Eos protein level is decreased by at least 50 %.

## Patentansprüche

1. Verbindung von Formel (I): 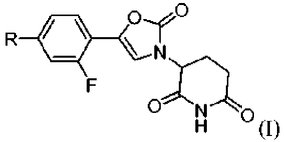 oder Stereoisomere, Tautomere oder Salze davon, wobei:
Rist:

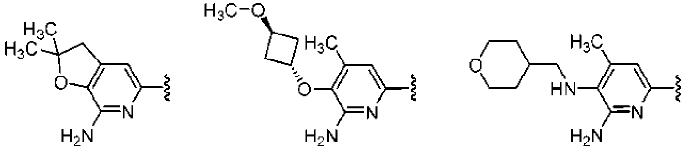

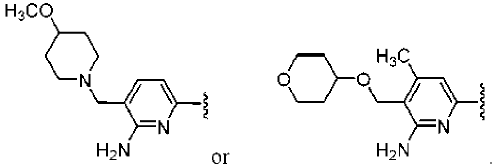

2. Verbindung nach Anspruch 1 oder Stereoisomere, Tautomere oder Salze davon, wobei die Verbindung Folgendes ist:

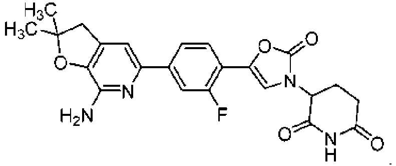

3. Verbindung nach Anspruch 1 oder Stereoisomere, Tautomere oder Salze davon, wobei die Verbindung Folgendes ist:

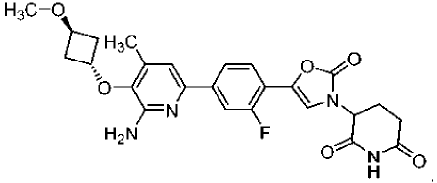

4. Verbindung nach Anspruch 1 oder Stereoisomere, Tautomere oder Salze davon, wobei die Verbindung Folgendes ist:

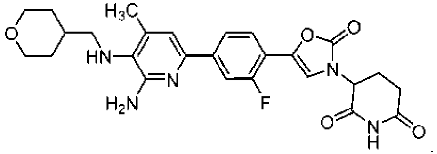

5. Verbindung nach Anspruch 1 oder Stereoisomere, Tautomere oder Salze davon, wobei die Verbindung Folgendes ist:

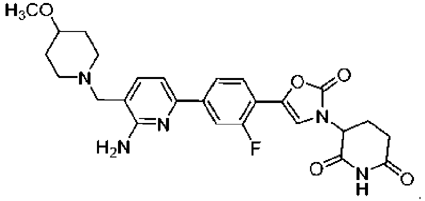

6. Verbindung nach Anspruch 1 oder Stereoisomere, Tautomere oder Salze davon, wobei die Verbindung Folgendes ist:

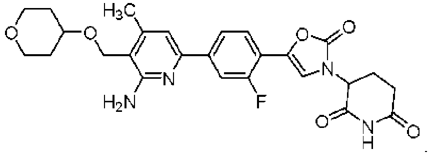

7. Pharmazeutische Zusammensetzung, umfassend eine Verbindung nach einem der Ansprüche 1 bis 6 oder Stereoisomere, Tautomere oder pharmazeutisch annehmbare Salze davon; und einen pharmazeutisch annehmbaren Träger.

8. Verbindung oder Stereoisomere, Tautomere oder pharmazeutisch annehmbare Salze davon nach einem der Ansprüche 1 bis 6 zur Verwendung bei der Behandlung von Krebs.

9. Verbindung oder Stereoisomere, Tautomere oder pharmazeutisch annehmbare Salze davon, zur Verwendung nach Anspruch 8, wobei der Krebs ausgewählt ist aus Dickdarmkrebs, Magen-, Bauchspeicheldrüsenkrebs, Brustkrebs, Prostatakrebs, Lungenkrebs, Eierstockkrebs, Gebärmutterhalskrebs, Nierenkrebs, Kopf- und Halskrebs, Lymphomen, Leukämie und Melanomen.

10. Verbindung oder Stereoisomere, Tautomere oder pharmazeutisch annehmbare Salze davon, zur Verwendung nach Anspruch 9 in Kombination mit einem zweiten Mittel, wobei das zweite Mittel ausgewählt ist aus einem Antagonisten der PD1/PD-L1-Achse, einem Antagonisten von CTLA4, einem chemotherapeutischen Mittel, Bestrahlung oder einem Antitumor-Impfstoff.

11. Verbindung oder ein Stereoisomer, ein Tautomer oder ein pharmazeutisch annehmbares Salz davon nach einem der Ansprüche 1 bis 6 zur Verwendung in einem Verfahren zum Behandeln von Krebs, wobei das Verfahren die Verringerung der Ikaros-, Helios-, Aiolos- und Eos-Proteinspiegel in Zellen durch Inkontaktbringen der Zellen mit der Verbindung oder einem Stereoisomer, einem Tautomer oder einem pharmazeutisch annehmbaren Salz davon umfasst, wobei
a) der Ikaros-Proteinspiegel um mindestens 30 % verringert wird;
b) der Helios-Proteinspiegel um mindestens 50 % verringert wird;
c) der Aiolos-Proteinspiegel um mindestens 30 % verringert wird; und
d) der Eos-Proteinspiegel um mindestens 50 % verringert wird.

## Revendications

1. Composé de Formule (I) : 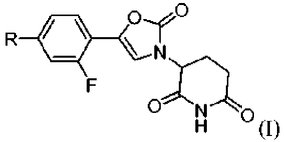 ou stéréoisomères, tautomères, ou sels de celui-ci, dans lequel :
R est :

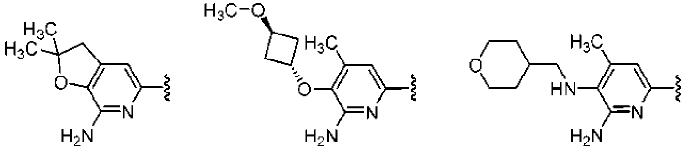

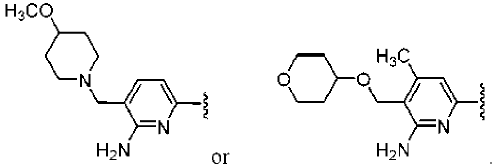

2. Composé selon la revendication 1 ou stéréoisomères, tautomères ou sels de celui-ci, dans lequel ledit composé est :

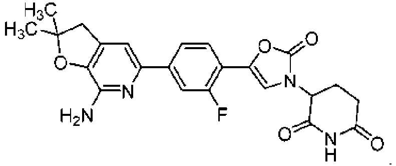

3. Composé selon la revendication 1 ou stéréoisomères, tautomères ou sels de celui-ci, dans lequel ledit composé est :

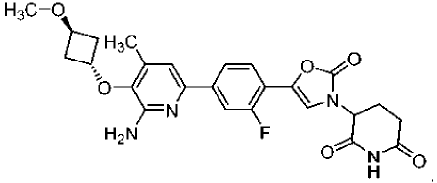

4. Composé selon la revendication 1 ou stéréoisomères, tautomères ou sels de celui-ci, dans lequel ledit composé est :

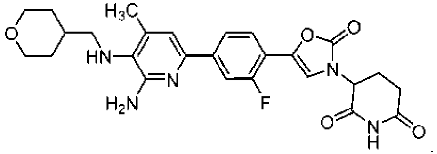

5. Composé selon la revendication 1 ou stéréoisomères, tautomères ou sels de celui-ci, dans lequel ledit composé est :

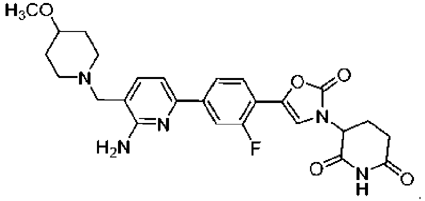

6. Composé selon la revendication 1 ou stéréoisomères, tautomères ou sels de celui-ci, dans lequel ledit composé est :

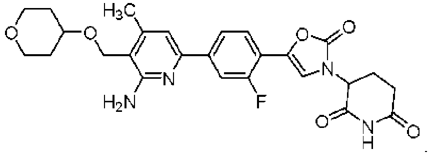

7. Composition pharmaceutique comprenant un composé selon l'une quelconque des revendications 1 à 6 ou stéréoisomères, tautomères ou sels pharmaceutiquement acceptable de celui-ci, et un support pharmaceutiquement acceptable.

8. Composé, ou stéréoisomères, tautomères ou sels pharmaceutiquement acceptables de celui-ci, selon l'une quelconque des revendications 1 à 6, destiné à être utilisé dans le traitement du cancer.

9. Composé, ou stéréoisomères, tautomères ou sels pharmaceutiquement acceptables de celui-ci, destiné à être utilisé selon la revendication 8, dans lequel ledit cancer est sélectionné parmi le cancer du côlon, gastrique, du pancréas, du sein, de la prostate, du poumon, de l'ovaire, du col de l'utérus, du rein, de la tête et du cou, le lymphome, la leucémie et le mélanome.

10. Composé, ou stéréoisomères, tautomères ou sels pharmaceutiquement acceptables de celui-ci, destiné à être utilisé selon la revendication 9 en combinaison avec un second agent, dans lequel ledit second agent est sélectionné parmi un antagoniste de l'axe PD1/PD-L1, un antagoniste de CTLA4, un agent chimiothérapeutique, un rayonnement ou un vaccin antitumoral.

11. Composé, ou un stéréoisomère, un tautomère ou un sel pharmaceutiquement acceptable de celui-ci selon l'une quelconque des revendications 1 à 6, destiné à être utilisé dans un procédé de traitement du cancer, dans lequel le procédé comprend la diminution des taux de protéines Ikaros, Helios, Aiolos et Eos dans des cellules en mettant en contact lesdites cellules avec ledit composé, ou un stéréoisomère, un tautomère ou un sel pharmaceutiquement acceptable de celui-ci, dans lequel
a) ledit taux de protéine Ikaros est réduit d'au moins 30 % ;
b- ledit taux de protéine Helios est réduit d'au moins 50 % ;
c) ledit taux de protéine Aiolos est réduit d'au moins 30 % ; et
d- ledit taux de protéine Eos est réduit d'au moins 50 %.
